# EUROPEAN PATENT SPECIFICATION

(11) **EP 2 500 010 B1**
(45) Date of publication and mention of the grant of the patent: **22.01.2020**
(21) Application number: 10829986.8
(22) Date of filing: 11.11.2010
(51) Int. Cl.: A61K 8/04, A61K 8/41, A61K 8/22, A61Q 5/08, A61Q 5/10, A45D 34/00

(54) **METHOD OF DYEING OR BLEACHING HAIR BY USING A COSMETIC COMPOSITION IN FORM OF A FOAM**
VERFAHREN ZUM FÄRBEN ODER BLEICHEN DER HAARE UNTER VERWENDUNG EINER SCHAUMFÖRMIGEN KOSMETISCHEN ZUSAMMENSETZUNG
PROCÉDÉ DE COLORATION OU D'ÉCLAIRCISSEMENT DES CHEVEUX UTILISANT UNE COMPOSITION COSMÉTIQUE EN FORME D'UNE MOUSSE

(30) Priority: 13.11.2009 JP 2009260490
(43) Date of publication of application: 19.09.2012
(73) Proprietor: Henkel AG & Co. KGaA, 40589 Düsseldorf (DE)
(72) Inventor: KONNO, Yoshihiro, Aichi-gun Aichi 480-1131 (JP); KOBAYASHI, Yosuke, Aichi-gun Aichi 480-1131 (JP)
(86) International application number: PCT/JP2010/070112
(87) International publication number: WO 2011/059027

(56) References cited:
- EP-A1- 1 658 234
- WO-A1-99/60993
- JP-A- 3 287 521
- JP-A- 63 174 917
- JP-A- 2006 342 125
- JP-A- 2007 503 409
- JP-A- 2009 154 884
- US-A- 4 921 170
- US-A- 5 769 901
- US-A1- 2004 103 488
- DATABASE GNPD [Online] MINTEL; 30 June 2008 (2008-06-30), anonymous: "soft color foam colorant", XP002748635, Database accession no. 922867
- DATABASE GNPD [Online] MINTEL; 28 February 2010 (2010-02-28), anonymous: "Shaking whipped hair colour", XP002748636, Database accession no. 1294574

## Description

### Technical field

The present invention relates to a method of dyeing hair or bleaching hair or removing dye from hair by applying to the hair a cosmetic preparation for hair in the form of a foam. More specifically, it relates to preparing a cosmetic preparation for hair that can be made to foam simply by mixing and can hold well to the hair in the form of a foam.

### Background art

Compositions that are a foam at the time of use, for example, composed of a first agent containing an alkali agent and a second agent containing an oxidising agent, are known as cosmetic preparations for hair to be used to dye, bleach, or remove dye from hair. Such cosmetic preparations for hair used in the form of a foam are characterised in prompting fewer concerns about dripping, being easier to apply to the hair, and feeling better when used than cosmetic preparations for hair used in the form of a liquid. Conventional cosmetic preparations for hair are disclosed, for example, in Patent References 1 to 3.

Patent Reference 1 discloses an aerosol-type foamy oxidation hair dyeing agent preparation prepared in the form of a foam at the time of use using a propellant, for example, LPG. Patent Reference 2 discloses a two-component-type composition for dyeing hair or removing colour prepared in the form of a foam at the time of use by using a flexible synthetic resin container, dip tube for suction, and a squeeze-foamer comprising foam-discharge means. The formation of a cosmetic preparation for hair in the form of a foam by causing the content solution to foam by mixing the content solution with air by shaking a squeeze-type foam discharge container containing a content solution comprising a first agent and a second agent and squeezing the foam through foam homogenisation means composed of a net or porous material is described in Patent Reference 3.

### Prior Art References - Patent References

Patent Reference 1: JP Kokai 09-136818
Patent Reference 2: JP Kokai 2008-291020
Patent Reference 3: JP Kokai 2009-154884

WO99/60993 discloses compositions for removing dye from hair comprising a powdered alkali agent and a liquid oxidising agent.

### Summary of the Invention

### Problems to Be Solved by the Invention

However, disposal is inconvenient in the case of the aerosol-type foamy oxidation hair dyeing agent preparation of Patent Reference 1 because it is mainly packed in a can. Since the mixture of the two agents is not simply discharged after foaming in the case of the two-component-type composition for dyeing hair or removing colour of Patent Reference 2, poor mixing tends to occur and the discharge procedure must be performed carefully. This is especially true when mixing a powdered agent and a liquid agent, and becomes a cause of decreased usability.
The container must be squeezed with a relatively large amount of force to push out the large foam that has just been shaken by causing it to pass through the foam homogenisation means in the case of the foamy cosmetic composition for hair of Patent Reference 3, and usability is a problem. Lowering the viscosity of the foam might be considered as means for lessening the force necessary to push out the foam, but the foamy cosmetic composition for hair applied to the hair then tends to drip immediately and does not hold well to the hair.
The present invention is based on the discovery made by the present inventors as a result of in-depth research that a foamed cosmetic preparation for hair obtained by mixing a powdered agent and a liquid agent and producing a foam by shaking has small foam. Since no foam homogenisation means need be used if the foam is small, the cosmetic preparation for hair can be mixed and foamed easily. Such a foamy cosmetic preparation for hair was also understood to hold well to the hair.
The object of the present invention is to provide a method for the application of a cosmetic preparation for hair that can be made to foam simply by mixing and can hold well to the hair in the form of a foam.

### Means Used to Solve the Above-mentioned Problems

To achieve the aforestated object, according to a first aspect of the present invention, there is provided a method of dyeing hair or bleaching hair or removing dye from hair,
the method being characterised in comprising:
a step of forming a cosmetic preparation for hair in the form of a foam by
introducing a powdered first agent 11 including an alkali agent and liquid second agent 12 of a cosmetic preparation for hair including hydrogen peroxide as an oxidising agent into the container body 21, bringing the first agent 11 and second agent 12 into contact in this way and obtaining a mixture 13;
attaching a lid 22 to the container body 21, wherein a flange-shaped fitting part is formed on the rim of the lid 22, and the fitting part of the lid 22 is fitted to the opening of the container body 21 so that the lid 22 is attached in a fluid-tight manner to the container body 21 by rotating the lid 22,
thereby obtaining a fluid-tight, sealable container 20 and
creating a cosmetic preparation for hair in the form of a foam 14 through shaking the container 20;
removing the lid 22 from the container body 21; and
a step of manually removing the foam 14 directly from the container body 21 by hand and applying it to the hair.

The cosmetic preparation for hair used in the method mentioned above may comprise an amphoteric surfactant or cationic surfactant. When the cosmetic preparation for hair used in the method mentioned above contains an anionic surfactant in addition to a cationic surfactant, the cosmetic preparation for hair is preferably constituted so that the anionic surfactant and cationic surfactant come into contact in the presence of a solvent at the time of use.

The above cationic surfactant preferably comprises an ammonium-type cationic surfactant comprising an alkyl group having from 16 to 22 carbon atoms and an ammonium-type cationic surfactant comprising an alkyl group having from 10 to fewer than 16 carbon atoms.

When the cosmetic preparation for hair used in the method mentioned above comprises a nonionic polymer, the nonionic polymer is preferably combined with the above powdered agent. When the cosmetic preparation for hair of the above embodiment comprises a thickener, the thickener is preferably combined with the above powdered agent.

The cosmetic preparation for hair used in the method mentioned above may be comprise a powdered first agent including an alkali agent, and a liquid second agent including hydrogen peroxide as an oxidising agent. The second agent may also include an amphoteric surfactant and cationic polymer.
The cosmetic preparation for hair used in the method mentioned above may also include an alkali metal inorganic salt. The above second agent may also contain phenoxyethanol and at least one component selected from benzoic acid and benzoates.
The above alkali agent is preferably a carbonate. When the cosmetic preparation for hair used in the method mentioned above also includes 1 to 5 wt% of a chelating agent, the weight ratio of the carbonate content of the cosmetic preparation for hair to the chelating agent content of the cosmetic preparation for hair is preferably 0.02 to 6.5.

### Effect of the Invention

The present invention provides a method for the application of a cosmetic preparation for hair that can be made to foam simply by mixing and can hold well to the hair in the form of a foam.

### Brief Description of the Drawings

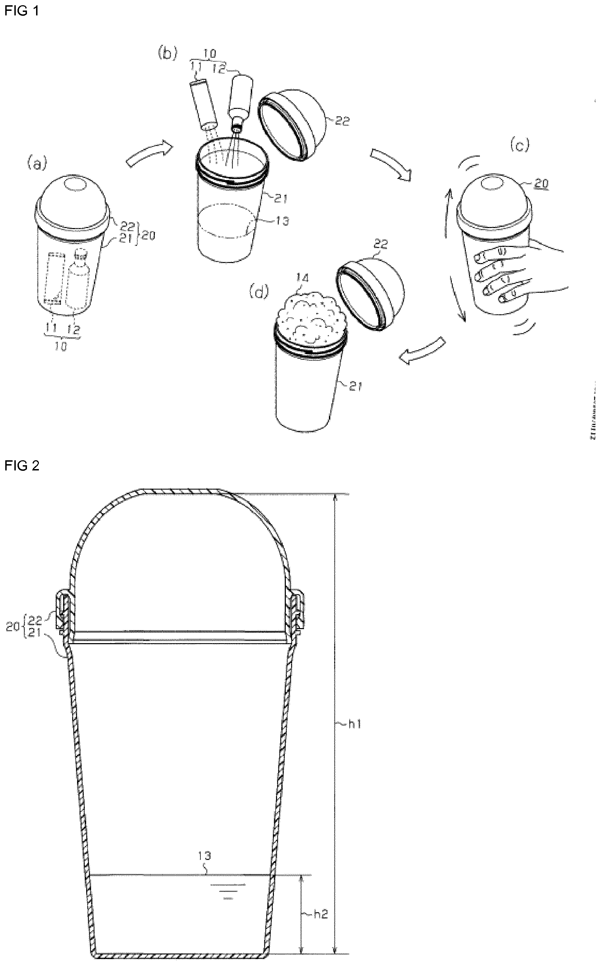

[FIG 1] FIG 1(a) through FIG 1(d) are schematic drawings that illustrate a method of use of a cosmetic preparation for hair containing a two-component-type agent for bleaching or removing dye from hair according to one embodiment of the present invention. More specifically, FIG 1(a) is a drawing illustrating a container for housing the first agent and second agent of the agent for bleaching or removing dye from hair before use. FIG 1(b) is a drawing illustrating how to introduce the first agent and second agent into the body of the container. FIG 1(c) is a drawing illustrating how to attach the lid to the container and shake the container up and down. FIG 1(d) is a drawing illustrating the lid removed from the container so that the foamy agent for bleaching or removing dye from hair inside the container can be removed directly and applied to the hair. [FIG 2] FIG 2 is a cross-sectional drawing of the container of FIG 1.

### Best Mode for Carrying Out the Invention

### (First embodiment)

A first embodiment, in which the cosmetic preparation for hair which is applied in the method of dyeing hair or bleaching hair or removing dye from hair of the present invention is embodied by a two-component agent for bleaching or removing dye from hair and a three-component agent for bleaching or removing dye from hair, is described below.

### <Two-component agent for bleaching or removing dye from hair>

The two-component agent for bleaching or removing dye from hair is constituted from a powdered first agent containing at least an alkali agent (also called component F hereinafter) and a liquid second agent containing hydrogen peroxide as an oxidising agent. This agent for bleaching or removing dye from hair is applied to the hair to bleach or remove dye from the hair after being made into the form of a foam by mixing the first agent and second agent and creating a foam through shaking.

### <First agent of the two-component agent for bleaching or removing dye from hair>

In addition to the alkali agent of component F, the first agent also contains, for example, an anionic surfactant (also called component C hereinafter), nonionic polymer (also called component D hereinafter), thickener (also called component E hereinafter), and chelating agent (also called component L hereinafter).
The alkali agent acts to improve the hair colour- or dye-removing effect by promoting the action of the oxidising agent contained in the second agent. The alkali agent used is preferably a solid at 25°C (normal temperature). Examples of such alkali agents include silicates, carbonates (also called component f-1 hereinafter), metasilicates, sulphates, chlorides, and phosphates.

Examples of silicates include sodium silicate, potassium silicate, and magnesium silicate. Bicarbonates are also included in the carbonates of component f-1. Therefore, examples of carbonates include sodium carbonate, magnesium carbonate, ammonium carbonate, sodium bicarbonate, and ammonium bicarbonate. Examples of metasilicates include sodium metasilicate and potassium metasilicate. Examples of sulphates include ammonium sulphate. Examples of chlorides include ammonium chloride. Examples of phosphates include primary ammonium phosphate and secondary ammonium phosphate.

Carbonates are especially preferred among these concrete examples of alkali agents. When a carbonate is used as the alkali agent, the ease of mixing and foaming the agent for bleaching or removing dye from hair improves and the ability of the foamy agent for bleaching or removing dye from hair to hold to the hair improves. Furthermore, the development of an ammonia odour at the time of use of the agent for bleaching or removing dye from hair can be suppressed. In addition, since carbonates do not generate heat of fusion when dissolved in water or another such solvent, the development of uneven brightness in the hair after it has been treated by the agent for bleaching or removing dye from hair can also be suppressed.

The alkali agent is preferably combined in a quantity such that the pH of the mixture of the first agent and a second agent, that is, the agent for bleaching or removing dye from hair, falls in the range of 7 to 12 at the time of use. If the pH of the mixture of the first agent and second agent is 7 or higher, the action of hydrogen peroxide can be strongly promoted because the oxidising agent contained in the second agent is hydrogen peroxide (also called component G hereinafter). If the pH of the mixture of the first agent and second agent is 12 or lower, damage to the hair when the agent for removing colour or dye from the hair is applied to the hair can be suppressed.

The anionic surfactant of component C acts to improve the ease of mixing and foaming of the agent for bleaching or removing dye from hair and the ability of the foamy agent for bleaching or removing dye from hair to hold to the hair. Therefore, the first agent preferably contains an anionic surfactant. An anionic surfactant may be combined with either one or the other of the first agent and second agent or may be combined with both the first agent and second agent. Examples of anionic surfactants include alkyl ether sulphates, alkyl sulphates, alkenyl ether sulphates, alkenyl sulphates, olefin sulphonates, alkane sulphonates, saturated or unsaturated fatty acid salts, alkyl or alkenyl ether carboxylates, α-sulphone fatty acid salts, N-acylamino acid surfactants, phosphoric acid mono- or di-ester surfactants, and sulphosuccinates. The counter ion of the anionic group of these surfactants may be any of sodium ion, potassium ion, and triethanolamine. More specifically, examples of alkyl sulphates include sodium lauryl sulphate and sodium cetyl sulphate. Examples of sulphosuccinates include disodium lauryl sulphosuccinate. The anionic surfactant used may be only one type, or two or more types of anionic surfactant may be used in combination.

The anionic surfactant content of the mixture of the first agent and second agent is preferably 0.1 to 10 wt%, more preferably 0.5 to 5 wt%. When the anionic surfactant content is within either of the above ranges, it is possible to satisfactorily obtain an effect of improvement in the ease of mixing and foaming of the agent for bleaching or removing dye from hair and in the ability of the foamy agent for bleaching or removing dye from hair to hold to the hair. The feeling of dryness of the hair after being treated with the agent for bleaching or removing dye from hair can also be suppressed.

The anionic surfactant is preferably brought into contact with the cationic surfactant of component B in the presence of a solvent at the time of use of the agent for bleaching or removing dye from hair. Therefore, for example, the anionic surfactant may be combined with either the first agent or second agent and the cationic surfactant may be combined with the other, or both the anionic surfactant and cationic surfactant may be combined with the first agent having a powdered form. This improves the foamability of the agent for bleaching or removing dye from hair and improves the hold of the foam obtained by foaming. This further improves the ease of mixing and foaming of the agent for bleaching or removing dye from hair and the ability of the foamy agent for bleaching or removing dye from hair to the hold to the hair. Other advantages are faster onset of the thickening effect when mixing the first agent and second agent, and improvement of the viscosity stability of the agent for bleaching or removing dye from hair.

To further improve the hold of the foam obtained by foaming, the anionic surfactant content of the mixture of the first agent and second agent is preferably 0.1 to 3.0 wt%, more preferably 0.5 to 1.5 wt%. The cationic surfactant content of the mixture of the first agent and second agent is preferably 0.1 to 5.0 wt%, more preferably 0.5 to 4.5 wt%, even more preferably 0.5 to 2.0 wt%. Furthermore, the weight ratio of the anionic surfactant content of the mixture of the first agent and second agent to the cationic surfactant content of the mixture is preferably in the range of 0.25 to 3, more preferably the range of 0.4 to 3, and even more preferably the range of 0.8 to 2.

To further accelerate the rate of onset of the thickening effect when mixing the first agent and second agent and further improve the viscosity stability of the agent for bleaching or removing dye from hair, the anionic surfactant content of the mixture of the first agent and second agent is preferably 0.01 to 10 wt%, more preferably 0.5 to 5 wt%. The weight ratio of the anionic surfactant content of the mixture to the cationic surfactant content of the mixture of the first agent and second agent is preferably 0.1 to 20, more preferably 0.5 to 10, and even more preferably 1 to 7.

The nonionic polymer of component D acts to improve the ease of mixing and foaming of the agent for bleaching or removing dye from hair and the ability of the foamy agent for bleaching or removing dye from hair to hold to the hair. It also acts to improve the foam quality of the foamy agent for bleaching or removing dye from hair, for example, the homogeneity and elasticity of the foam. Therefore, the first agent preferably contains a nonionic polymer. A nonionic polymer may be combined with either one or the other of the first agent and second agent or may be combined with both the first agent and second agent.

The nonionic polymer used may be a natural polymer or a semisynthetic or synthetic polymer. Natural or semisynthetic nonionic saccharides are preferably used as this further improves the ease of mixing and foaming of the agent for bleaching or removing dye from hair and the ability of the foamy agent for bleaching or removing dye from hair to hold to the hair as well as the homogeneity and elasticity of the foam of the foamy agent for bleaching or removing dye from hair. It is especially preferable to use α-glucose and oligosaccharides and polysaccharides having an α-glucose compound as a structural unit such as α-glucose derivatives. Examples of oligosaccharides and polysaccharides having an α-glucose compound as a structural unit include starch, hydrolysed starch, starch derivatives, dextran, dextrin, and cyclodextrin. Examples of other nonionic saccharides include cellulose and agar. It is more preferable to use a dispersive nonionic polymer having low water solubility (for example, starch and cyclodextrin) than a highly water-soluble nonionic polymer for the same reasons as the natural or semisynthetic nonionic saccharides. Examples of nonionic synthetic polymers include polyvinyl caprolactam, PVP, (vinyl pyrrolidone/VA) copolymers, polyvinyl alcohol, polyvinyl butyral, and high molecular-weight polyethylene glycol.

When two or more types of nonionic polymer are used in combination, the ease of mixing and foaming of the agent for bleaching or removing dye from hair and the ability of the foamy agent for bleaching or removing dye from hair to hold to the hair are further improved, and the homogeneity and elasticity of the foam of the foamy agent for bleaching or removing dye from hair are sometimes further improved. It is especially preferable to use two or more types of dispersive nonionic polymer in combination. It is even more preferable if at least one of the dispersive nonionic polymers used in combination is starch.

The nonionic polymer content of the mixture of the first agent and second agent is preferably 0.3 to 15 wt%, more preferably 1 to 10 wt%. When the nonionic surfactant content is within either of the above ranges, it is possible to satisfactorily obtain an effect of improvement in the ease of mixing and foaming of the agent for bleaching or removing dye from hair and in the ability of the foamy agent for bleaching or removing dye from hair to hold to the hair. The homogeneity and elasticity of the foam of the foamy agent for bleaching or removing dye from hair also improve further.

When starch is combined as a nonionic polymer with the first agent having the form of a powder, the onset of the thickening effect when mixing the first agent and second agent is hastened and the viscosity stability of the agent for bleaching or removing dye from hair is improved. Examples of starches include starch from grains, starch from potatoes, starch from beans, starch from wild grasses, starch from trunks and stems, and modified forms of these starches (processed starch). More concretely speaking, corn starch, tapioca starch, potato starch, sweet potato starch, sago starch, and modified forms of these starches can be used. Modified starch means starch in which properties inherent to the starch have been artificially changed by subjecting starch to etherification, esterification, grafting, or other such treatments to create derivatives; firing, enzymatic denaturation, oxidation, acid treatment, or other such degradation treatments; or alphatisation, milling, porosification, or other such process. The starch used may be only one type, or two or more types of starch may be used in combination.

To further accelerate the rate of onset of the thickening effect when mixing the first agent and second agent and further improve the viscosity stability of the agent for bleaching or removing dye from hair, the starch content of the mixture of the first agent and second agent is preferably 0.1 to 20 wt%, more preferably 2 to 10 wt%.

The thickener of component E acts to improve the ease of mixing and foaming of the agent for bleaching or removing dye from hair and the ability of the foamy agent for bleaching or removing dye from hair to hold to the hair. It also acts to suppress dripping from the hair of the agent for bleaching or removing dye from hair when the agent for bleaching or removing dye from hair is applied to the hair, by imparting proper viscosity to the agent for bleaching or removing dye from hair. Therefore, the first agent preferably contains a thickener. A thickener may be combined with either one or the other of the first agent and second agent or may be combined with both the first agent and second agent. Concrete examples of thickeners include natural polymers, semisynthetic polymers, synthetic polymers, and inorganic polymers.

Examples of natural polymers include guar gum, locust bean gum, quince seed, carrageenan, galactan, gum Arabic, tragacanth gum, pectin, mannan, xanthan gum, succinoglycan, curdlan, hyaluronic acid, gelatine, casein, albumin, and collagen. Examples of semisynthetic polymers include methyl cellulose, ethyl cellulose, hydroxyethyl cellulose, hydroxypropyl cellulose, hydroxypropyl methyl cellulose, carboxymethyl cellulose, carboxymethyl cellulose sodium, methyl hydroxypropyl cellulose, alginic acid propylene glycol ester, and alginates. Examples of synthetic polymers include polyvinyl pyrrolidone, polyvinyl methyl ether, carboxyvinyl polymers, sodium polyacrylate, polyacrylamide, polyethylene oxide, ethylene oxide-propylene oxide block copolymers, and acrylic acid/alkyl acrylate copolymers. Copolymers comprising a half ester of itaconic acid and polyoxyethylene alkyl ether or an ester of methacrylic acid and polyoxyethylene alkyl ether and at least one monomer selected from acrylic acid, methacrylic acid, and alkyl esters of these can also be used. Examples of inorganic polymers include bentonite, magnesium aluminium silicate, Laponite, hectorite, and silicic anhydride. The thickener used may be only one type, or two or more types of thickener may be used in combination.

The thickener content of the mixture of the first agent and second agent is preferably 0.1 to 20 wt%, more preferably 1 to 5 wt%. When the thickener content is within either of the above ranges, it is possible to satisfactorily obtain an effect of improvement in the ease of mixing and foaming of the agent for bleaching or removing dye from hair and in the ability of the foamy agent for bleaching or removing dye from hair to hold to the hair. It is also possible to suppress a decline in the applicability of the agent for bleaching or removing dye from hair.

For accelerating the rate of onset of the thickening effect when mixing the first agent and second agent and further improve the viscosity stability of the agent for bleaching or removing dye from hair, the thickener content of the mixture of the first agent and second agent is preferably 0.05 to 20 wt%, more preferably 0.5 to 4 wt%. The weight ratio of the starch content of the mixture of the first agent and second agent to the thickener content of the mixture is preferably 0.1 to 20, more preferably 1 to 7, and even more preferably 1 to 3. When this weight ratio is within any of the above ranges, the rate of onset of the thickening effect when mixing the first agent and second agent is further hastened and the viscosity stability of the agent for bleaching or removing dye from hair improves further.

The chelating agent of component L acts to improve the ease of mixing and foaming of the agent for bleaching or removing dye from hair and the ability of the foamy agent for bleaching or removing dye from hair to hold to the hair. It also acts to suppress the rough feel of the hair after treatment by the agent for bleaching or removing dye from hair that can occur due to carbonates when a carbonate is used as the alkali agent of component F and to improve the feel of the hair, that is, the feeling of sleekness when passing a hand through the hair, after treatment by the agent for bleaching or removing dye from hair. Therefore, the first agent preferably contains a chelating agent. A chelating agent may be combined with either one or the other of the first agent and second agent or may be combined with both the first agent and second agent.

Examples of chelating agents include edetic acid (ethylenediaminetetraacetic acid (EDTA)), ethylenediaminehydroxyethyltriacetic acid, dihydroxyethylethylenediaminediacetic acid, 1,3-propanediaminetetraacetic acid, diethylenetriaminepentaacetic acid, triethylenetetraminehexaacetic acid, nitrilotriacetic acid, hydroxyethyliminodiacetic acid, L-aspartic acid-N,N-diacetic acid, aminotrimethylene phosphonic acid, hydroxyethane diphosphonic acid, salts of these, derivatives of these, and salts of derivatives of these. The chelating agent used may be only one type, or two or more types of chelating agent may be used in combination.

The chelating agent content of the mixture of the first agent and second agent is preferably 1 to 5 wt%, more preferably 1 to 3 wt%. When the chelating agent content is within either of the above ranges, it is possible to satisfactorily obtain an effect of improvement in the ease of mixing and foaming of the agent for bleaching or removing dye from hair and in the ability of the foamy agent for bleaching or removing dye from hair to hold to the hair. The feel of the hair after treatment by the agent for bleaching or removing dye from hair can also be further improved.

When a carbonate is used as the alkali agent of component F, the weight ratio ((f-1)/L) of the carbonate content of the mixture of the first agent and second agent to the chelating agent content of the mixture is preferably 0.02 to 6.5, more preferably 0.5 to 4.5, and even more preferably 0.9 to 4.5. When this weight ratio is within any of the above ranges, a substantially greater improvement is obtained in regard to the ease of mixing and foaming of the agent for bleaching or removing dye from hair and the ability of the foamy agent for bleaching or removing dye from hair to hold to the hair. A decline in the feel of the hair after treatment by the agent for bleaching or removing dye from hair that can be caused by the carbonate can also be more strongly suppressed.

The first agent may also contain any components other than the components discussed above, for example, oily components, polyhydric alcohols, additional surfactants other than the above surfactants, sugars, preservatives, stabilisers, pH regulators, plant extracts, raw drug extracts, vitamins, perfumes, antioxidants, ultraviolet absorbers, oxidation auxiliaries, excipients, and additives, as needed.

Oily components play a role in imparting moisture to the hair. Examples of oily components include oils, waxes, higher alcohols, hydrocarbons, higher fatty acids, alkyl glyceryl ethers, esters, and silicones.

Examples of oils include lanolin, olive oil, camellia oil, shea butter, almond oil, safflower oil, sunflower oil, soybean oil, cottonseed oil, sesame oil, corn oil, canola oil, rice bran oil, rice germ oil, grape seed oil, avocado oil, macadamia nut oil, castor oil, coconut oil, and evening primrose oil. Examples of waxes include beeswax, candelilla wax, carnauba wax, jojoba oil, and lanolin. Examples of higher alcohols include cetyl alcohol (cetanol), 2-hexyl decanol, stearyl alcohol, isostearyl alcohol, cetostearyl alcohol, oleyl alcohol, aralkyl alcohol, behenyl alcohol, 2-octyl dodecanol, lauryl alcohol, myristyl alcohol, decyl tetradecanol, and lanolin alcohol. Examples of hydrocarbons include paraffin, olefin oligomers, polyisobutene, hydrogenated polyisobutene, mineral oil, squalane, polybutene, polyethylene, microcrystalline wax, and petrolatum. Examples of higher fatty acids include lauric acid, myristic acid, palmitic acid, stearic acid, behenic acid, isostearic acid, 12-hydroxystearic acid, oleic acid, and lanolin fatty acid. Examples of alkyl glyceryl ethers include batyl alcohol, chimyl alcohol, selachyl alcohol, and isostearyl glyceryl ether. Examples of esters include diisopropyl adipate, isopropyl myristate, cetyl octanoate, isononyl isononanoate, octyl dodecyl myristate, isopropyl palmitate, stearyl stearate, myristyl myristate, isotridecyl myristate, 2-ethylhexyl palmitate, octyl dodecyl ricinoleate, cholesteryl/lanosteryl fatty acids having 10 to 30 carbon atoms, cetyl lactate, lanolin acetate, di-2-ethylhexanoic acid ethylene glycol, pentaerythritol fatty acid esters, dipentaerythritol fatty acid esters, cetyl caprate, glyceryl tricaprylate, diisostearyl malate, dioctyl succinate, and cetyl 2-ethylhexanoate. Examples of silicones include dimethyl polysiloxane (dimethicone), methyl phenyl polysiloxane, decamethyl cyclopentasiloxane, dodecamethyl cyclohexasiloxane, end hydroxy group-modified dimethyl polysiloxane, a silicone high polymer having an average degree of polymerisation of 650 to 10,000, polyethermodified silicone (for example, (PEG/PPG/butylene/dimethicone) copolymer), amino-modified silicone, betaine-modified silicone, alkyl-modified silicone, alkoxy-modified silicone, mercapto-modified silicone, carboxy-modified silicone, and fluorine-modified silicone. The silicone used may be only one type, or two or more types of silicone may be used in combination. Examples of polyhydric alcohols include glycol compounds and glycerine compounds. Examples of glycol compounds include ethylene glycol, diethylene glycol, triethylene glycol, polyethylene glycol, propylene glycol, dipropylene glycol, isoprene glycol, and 1,3-butylene glycol. Examples of glycerine compounds include glycerine, diglycerine, and polyglycerine.

An additional surfactant plays the role of an emulsifier or a solubiliser and is used to adjust the viscosity or improve the viscosity stability of the agent for bleaching or removing dye from hair. It also acts to improve the foamability of the agent for bleaching or removing dye from hair and acts to improve the foam quality, for example, the elasticity of the foam, of the foamy agent for bleaching or removing dye from hair. The surfactant used may be any of a cationic surfactant, amphoteric surfactant, and nonionic surfactant. A cationic surfactant may be combined with the first agent within the range that does not interfere with the effects of the present invention.

Examples of cationic surfactants include lauryl trimethylammonium chloride, cetyl trimethylammonium chloride, stearyl trimethylammonium chloride, alkyl trimethylammonium chloride, distearyl dimethylammonium chloride, cetyl trimethylammonium bromide, stearyl trimethylammonium bromide, ethyl sulphate lanolin fatty acid aminopropylethyldimethylammonium, stearyl trimethylammonium saccharine, cetyl trimethylammonium saccharine, methacryloyloxyethyl trimethylammonium chloride, and behenyl trimethylammonium methyl sulphate.

Examples of amphoteric surfactants include fatty acid amidopropyl dimethylaminoacetic acid betaines, alkyl dimethylaminoacetic acid betaines, N-acylaminoethyl-N-2-hydroxyethyl aminocarboxylates, N-acylaminoethyl-N-carboxymethoxyethyl amino-carboxylates, and hydroxyalkyl (C12 to 14) hydroxyethylsarcosines.

Examples of fatty acid amidopropyl dimethylaminoacetic acid betaines include coconut oil fatty acid amidopropyl dimethylaminoacetic acid betaine (also called cocamidopropyl betaine or coconut oil fatty acid amidopropyl betaine), palm oil fatty acid amidopropyl dimethylaminoacetic acid betaine, lauric acid amidopropyl dimethylaminoacetic acid betaine (also called lauramidopropyl betaine or lauric acid amidopropyl betaine), and ricinoleic acid amidopropyl dimethylaminoacetic acid betaine. Fatty acid amidopropyl dimethylaminoacetic acid betaines may be combined with the first agent in the form of a sodium salt, potassium salt, triethanolamine salt, and other such salts.

Examples of alkyl dimethylaminoacetic acid betaines include decyl dimethylaminoacetic acid betaine, lauryl dimethylaminoacetic acid betaine, myristyl dimethylaminoacetic acid betaine, cetyl dimethylaminoacetic acid betaine, stearyl dimethylaminoacetic acid betaine, oleyl dimethylaminoacetic acid betaine, behenyl dimethylaminoacetic acid betaine, and coconut oil alkyl dimethylaminoacetic acid betaine. Alkyl dimethylaminoacetic acid betaines may be combined with the first agent in the form of a sodium salt, potassium salt, triethanolamine salt, and other such salts.

Examples of N-acylaminoethyl-N-2-hydroxyethyl aminocarboxylates include sodium cocoamphoacetate (this is N-coconut oil fatty acid acyl-N'-carboxymethyl-N'-hydroxyethylethylenediamine; also called 2-alkyl-N-carboxymethyl-N-hydroxyethylimidazolinium betaine), sodium cocoamphopropionate (N-coconut oil fatty acid acyl-N'-carboxyethyl-N'-hydroxyethylethylenediamine), sodium lauroamphoacetate (N-lauroyl-N'-carboxymethyl-N'-hydroxyethylethylenediamine), sodium olive amphoacetate, sodium cocoa butter amphoacetate, sodium sesame amphoacetate, sodium sweet almond amphoacetate, stearamphoacetate, sodium palm amphoacetate, sodium peanut amphoacetate, sodium sunflower seed amphoacetate, and sodium cotton seed amphoacetate.

Examples of N-acylaminoethyl-N-carboxymethoxyethyl aminocarboxylates include sodium cocoamphodiacetate, sodium cocoamphodipropionate, and sodium lauroamphodiacetate.

Specific examples of nonionic surfactants include ether-type nonionic surfactants and ester-type nonionic surfactants. Concrete examples of ether-type nonionic surfactants include polyoxyethylene (referred to hereinafter as POE) cetyl ether (ceteth), POE stearyl ether (steareth), POE behenyl ether, POE oleyl ether (oleth), POE lauryl ether (laureth), POE octyldodecyl ether, POE hexyldecyl ether, POE isostearyl ether, POE nonylphenyl ether, and POE octylphenyl ether.

Specific examples of ester-type nonionic surfactants include POE sorbitan monooleate, POE sorbitan monostearate, POE sorbitan monopalmitate, POE sorbitan monolaurate, POE sorbitan trioleate, POE glycerine monostearate, POE glycerine monomyristate, POE sorbitol tetraoleate, POE sorbitol hexastearate, POE sorbitol monolaurate, POE sorbitol beeswax, polyethylene glycol monooleate, polyethylene glycol monostearate, polyethylene glycol monolaurate, lipophilic glycerine monooleate, lipophilic glycerine monostearate, self-emulsifying glycerine monostearate, sorbitan monooleate, sorbitan sesquioleate, sorbitan trioleate, sorbitan monostearate, sorbitan monopalmitate, sorbitan monolaurate, sucrose fatty acid esters, decaglyceryl monolaurate, decaglyceryl monostearate, decaglyceryl monooleate, and decaglyceryl monomyristate.

The additional surfactant used may be only one type, or two or more types of additional surfactant may be used in combination. To improve the foam quality of the foamy agent for bleaching or removing dye from hair, the surfactant content (at the time of use) of the mixture of the first agent and second agent is preferably 1.5 to 10 wt%, more preferably 3 to 8 wt%, and even more preferably 3 to 5 wt%.

Examples of sugars include sorbitol and maltose. Examples of preservatives include paraben. Examples of stabilisers include phenacetin, 8-hydroxyquinoline, acetanilide, sodium pyrophosphate, barbituric acid, uric acid, and tannic acid.
Examples of pH regulators include citric acid, tartaric acid, lactic acid, malic acid, succinic acid, fumaric acid, maleic acid, pyrophosphoric acid, gluconic acid, glucuronic acid, benzoic acid, 2-amino-2-methyl-1,3-propanediol, and basic amino acids.

Examples of antioxidants include ascorbic acid and sulphites. Examples of oxidation auxiliaries include ammonium persulphate, potassium persulphate, sodium persulphate, and other such persulphates. Oxidation auxiliaries are used to further improve the colour- and dye-removing effects of the agent for removing colour and dye from hair. Examples of excipients include sodium sulphate. Examples of additives include dispersants. Examples of dispersants include calcium stearate, magnesium stearate, and other such stearic acid metal salts, talc, crystalline cellulose, and low degree of substitution hydroxypropyl cellulose.

The form of the first agent is a powder. Furthermore, the term 'powder' in this specification is a concept that includes particles.

### <Second agent of a two-component agent for bleaching or removing dye from hair>

The second agent contains, in addition to hydrogen peroxide as an oxidising agent, for example, an amphoteric surfactant (also called component A hereinafter), a cationic surfactant (also called component
B hereinafter), a cationic polymer (also called component H hereinafter), an alkali metal inorganic salt (also called component I hereinafter), phenoxyethanol (also called component J hereinafter), and at least one component selected from benzoic acid and benzoates (also called component K hereinafter).

The amphoteric surfactant of component A acts to improve the ease of mixing and foaming of the agent for bleaching or removing dye from hair and the ability of the foamy agent for bleaching or removing dye from hair to hold to the hair. It also acts to emulsify the second agent and adjust it to the proper viscosity and improve the viscosity stability. It also acts to increase the amount and the fineness of the foam obtained by foaming the mixture of the first agent and second agent, to make non-aerosol foaming start quickly, and to improve the feel of the hair after treatment by the agent for bleaching or removing dye from hair in cooperation with the cationic polymer of component H. The second agent therefore preferably contains an amphoteric surfactant. An amphoteric surfactant may be combined with either one of the first agent or second agent or may be combined with both the first agent and second agent. Examples of the amphoteric surfactant contained in the second agent are the same as those explained previously as concrete examples of the amphoteric surfactant contained in the first agent. A betaine-based amphoteric surfactant is preferably used because it stabilises the pH of the second agent. The amphoteric surfactant used may be only one type, or two or more types of amphoteric surfactant may be used in combination. Commercial amphoteric surfactants that contain alkali metal inorganic salts may also be used.

Examples of commercial amphoteric surfactants that contain alkali metal inorganic salts include Obazolin CBA-30 (coconut oil fatty acid amidopropyl betaine, made by Toho Chemical Industry Co, Ltd), Softazolin CPB (coconut oil fatty acid amidopropyl betaine, made by Kawaken Fine Chemicals Co, Ltd), Rikabion B-200 (coconut oil fatty acid amidopropyl betaine, made by New Japan Chemical Co, Ltd), Obazolin LB (lauryl dimethylaminoacetic acid betaine, made by Toho Chemical Industry Co, Ltd), and Softazolin LPB (lauric acid amidopropyl betaine, made by Kawaken Fine Chemicals Co, Ltd). Examples of commercial amphoteric surfactants that do not contain alkali metal inorganic salts include Taipol Soft AM-100N (coconut oil fatty acid amidopropyl betaine, made by Taiko Oil Chemicals Co, Ltd), Amogen CB-H (coconut oil fatty acid amidopropyl betaine, made by Daiichi Kogyo Co, Ltd), Obazolin LB-SF (lauryl dimethylaminoacetic acid betaine, made by Toho Chemical Industry Co, Ltd), Softazolin CPB-R (coconut oil fatty acid amidopropyl betaine, made by Kawaken Fine Chemicals Co, Ltd), and Softazolin LPB-R (lauric acid amidopropyl betaine, made by Kawaken Fine Chemicals Co, Ltd).

The amphoteric surfactant content of the mixture of the first and second agent is preferably 0.1 to 10 wt%, more preferably 0.5 to 5 wt%. When the amphoteric surfactant content is within either of the above ranges, it is possible to satisfactorily obtain an effect of improvement in the ease of mixing and foaming of the agent for bleaching or removing dye from hair and in the ability of the foamy agent for bleaching or removing dye from hair to hold to the hair. The feeling of dryness in the hair after treatment by the agent for bleaching or removing dye from hair can also be suppressed.

To further improve the foamability of the agent for bleaching or removing dye from hair, the amphoteric surfactant content of the mixture of the first agent and second agent is preferably 0.5 to 10.0 wt%, more preferably 1.5 to 4.0 wt%.

To further improve the feel of the hair after treatment by the agent for bleaching or removing dye from hair, the amphoteric surfactant content of the second agent is preferably 0.5 to 12 wt%, more preferably 1.5 to 10 wt%, and even more preferably 1.5 to 3.5 wt%.

The cationic surfactant of component B acts to improve the ease of mixing and foaming of the agent for bleaching or removing dye from hair and the ability of the foamy agent for bleaching or removing dye from hair to hold to the hair. The second agent therefore preferably contains a cationic surfactant. An anionic surfactant may be combined with the second agent within the range that does not interfere with the effects of the present invention. Examples of cationic surfactants are the same as those explained previously as concrete examples of cationic surfactants contained in the first agent. The cationic surfactant used may be only one type, or two or more types of cationic surfactant may be used in combination.

To further improve the ease of mixing and foaming of the agent for bleaching or removing dye from hair and the ability of the foamy agent for bleaching or removing dye from hair to hold to the hair, the cationic surfactant preferably contains an ammonium-type cationic surfactant comprising an alkyl group having from 16 to 22 carbon atoms (also called component b-1 hereinafter) and an ammonium-type cationic surfactant comprising an alkyl group having from 10 to fewer than 16 carbon atoms (also called component b-2 hereinafter). This is also advantageous for improving the foam quality of the foamy agent for bleaching or removing dye from hair.
Examples of the ammonium-type cationic surfactant of component b-1 include dimethylammonium-type cationic surfactants and trimethylammonium-type cationic surfactants. More specifically, for example, cetyl trimethylammonium chloride (cetrimonium chloride, C16), stearyl trimethylammonium chloride (steartrimonium chloride, C18), distearyl dimethylammonium chloride (C18), behenyl trimethylammonium chloride (behentrimonium chloride, C22), distearyl dimethylammonium chloride (distearyldimonium chloride, C18), cetyl trimethylammonium bromide (C16), stearyl trimethylammonium bromide (C18), stearyl trimethylammonium saccharine (C18), cetyl trimethylammonium saccharine (C16), and behenyl trimethylammonium methyl sulphate (C22) can be used. Preferred among them are trimethylammonium-type surfactants comprising an alkyl group having 16 or more carbon atoms; more preferred are cetyl trimethylammonium and salts thereof.

Examples of the ammonium-type cationic surfactant of component b-2 include dimethylammonium-type cationic surfactants and trimethylammonium-type cationic surfactants. More specifically, examples include compounds explained previously as concrete examples of ammonium-type surfactants of component b-1 in which the number of carbon atoms of the alkyl group has been changed to from 10 to fewer than 16. Preferred among them are trimethylammonium-type cationic surfactants comprising an alkyl group having from 10 to fewer than 16 carbon atoms; more preferred are trimethylammonium-type cationic surfactants comprising an alkyl group having from 10 to 15 carbon atoms. Even more preferred are trimethylammonium-type cationic surfactants comprising an alkyl group having from 10 to 14 carbon atoms. Concrete examples of trimethylammonium-type cationic surfactants comprising an alkyl group having from 10 to 14 carbon atoms include dodecyl trimethylammonium (C10), lauryl trimethylammonium (C12), myristyl trimethylammonium (C14), and salts thereof. Preferred are lauryl trimethylammonium (C12) and salts thereof. Furthermore, the number of carbon atoms of the ammonium-type cationic surfactant means the number of carbon atoms in the main chain of the alkyl group. For example, it means the number of carbon atoms of the alkyl chain having the greatest number of carbon atoms in the case of a double-chain cationic surfactant.

The cationic surfactant content of the mixture of the first agent and second agent is preferably 0.1 to 10 wt%, more preferably 0.5 to 5 wt%. When the cationic surfactant content is within either of the above ranges, it is possible to satisfactorily obtain an effect of improvement in the ease of mixing and foaming of the agent for bleaching or removing dye from hair and in the ability of the foamy agent for bleaching or removing dye from hair to hold to the hair. The feeling of dryness of the hair after being treated with the agent for bleaching or removing dye from hair can also be suppressed.

To further improve the foam quality of the foamy agent for bleaching or removing dye from hair and the feel of the hair after being treated with the agent for bleaching or removing dye from hair, the amount of ammonium-type cationic surfactant of component b-1 in the mixture of the first agent and second agent is preferably 0.1 to 10 wt%, more preferably 0.2 to 2 wt%. The amount of ammonium-type cationic surfactant of component b-2 in the mixture of the first agent and second agent is also preferably 0.1 to 10 wt%, more preferably 0.4 to 4 wt%. The weight ratio of the amount of ammonium-type cationic surfactant of component b-2 in the mixture to the amount of ammonium-type cationic surfactant of component b-1 in the mixture of the first agent and second agent is also preferably in the range of 0.4 to 5.

The cationic polymer of component H acts to improve the ease of mixing and foaming of the agent for bleaching or removing dye from hair and the ability of the foamy agent for bleaching or removing dye from hair to hold to the hair. It also acts in cooperation with the amphoteric surfactant of component A to improve the feel of the hair after treatment by the agent for bleaching or removing dye from hair. The second agent therefore preferably contains a cationic polymer. A cationic polymer may be combined with either one of the first agent or second agent or may be combined with both the first agent and second agent.

The cationic polymer may be one that contains, for example, an amino group or ammonium group in the polymer chain or one that contains a diallyl quaternary ammonium salt as a structural unit. Examples of cationic polymers include cationised cellulose derivatives, cationic starch, cationised guar gum derivatives, diallyl quaternary ammonium salt/acrylamide copolymers, and quaternised polyvinyl pyrrolidone derivatives. More specifically, examples include dimethyl diallyl ammonium chloride-acrylamide copolymers, dimethyl diallyl ammonium chloride-acrylic acid copolymers, polydimethylmethylenepiperidium chloride, and hydroxyethylcellulose dimethyl diallyl ammonium.

Among these, cationic polymers that are liquid at 25°C are preferably used because they further improve the feel of the hair after treatment by the agent for bleaching or removing dye from hair. Examples of cationic polymers that are liquid at 25°C include Merquat 550 (dimethyl diallyl ammonium chloride-acrylamide copolymer), Merquat 280 and 295 (dimethyl diallyl ammonium chloride-acrylic acid copolymer), and Merquat 100 (polydimethylmethylenepiperidium chloride). These commercial cationic polymers mix well with other components because they dissolve in solvents.

The feel the hair after treatment by the agent for bleaching or removing dye from hair is further improved when dimethyl diallyl ammonium chloride polymers, dimethyl diallyl ammonium chloride-acrylamide copolymers, and dimethyl diallyl ammonium chloride-acrylic acid copolymers are used as cationic polymers as well. In particular, the rough feel of the hair after treatment by the agent for bleaching or removing dye from hair that can occur due to carbonates when a carbonate is used as the alkali agent of component F can be suppressed, as with the chelating agent of component L.

The cationic polymer content of the mixture of the first agent and second agent is preferably 0.05 to 5 wt%, more preferably 0.1 to 2 wt%. When the cationic polymer content is within either of the above ranges, it is possible to satisfactorily obtain an effect of improvement in the ease of mixing and foaming of the agent for bleaching or removing dye from hair and in the ability of the foamy agent for bleaching or removing dye from hair to hold to the hair. It is also possible to minimise any stickiness of the hair after treatment by the agent for bleaching or removing dye from hair, and a deterioration of feel.

To further improve the feel of the hair after treatment by the agent for bleaching or removing dye from hair, the cationic polymer content of the second agent is preferably 0.01 to 5 wt%, more preferably 0.1 to 4 wt%.

The alkali metal inorganic salt of component I acts to improve the ease of mixing and foaming of the agent for bleaching or removing dye from hair and the ability of the foamy agent for bleaching or removing dye from hair to hold to the hair. In particular, when hydrogen peroxide is used as an oxidising agent and an amphoteric surfactant is combined in the second agent, the alkali metal inorganic salt of component I also acts to stabilise the pH of the second agent and to improve the stability of the hydrogen peroxide. The second agent therefore preferably contains an alkali metal inorganic salt. An alkali metal inorganic salt may be combined with either one of the first agent or second agent or may be combined with both the first agent and second agent. Examples of alkali metal inorganic salts include alkali metal chlorides, sulphates, nitrates, and phosphates. The alkali metal may be, for example, sodium, potassium, or lithium. The alkali metal inorganic salt may therefore be, for example, sodium chloride, potassium chloride, sodium sulphate, or potassium sulphate. Alkali metal inorganic salts that are neutral salts are preferred from the viewpoint of pH stability.

The alkali metal inorganic salt content of the mixture of the first agent and second agent is preferably 0.05 to 5 wt%, more preferably 0.1 to 2 wt%. When the alkali metal inorganic salt content is within either of the above ranges, it is possible to satisfactorily obtain an effect of improvement in the ease of mixing and foaming of the agent for bleaching or removing dye from hair and in the ability of the foamy agent for bleaching or removing dye from hair to hold to the hair.

To improve the pH stability of the second agent, the alkali metal inorganic salt content of the second agent is preferably 0.01 to 5 wt%, more preferably 0.05 to 1.5 wt%, and even more preferably 0.25 to 1.25 wt%.

Similarly, to improve the pH stability of the second agent, the weight ratio (A/I) of the amphoteric surfactant content of the second agent to the alkali metal inorganic salt content of the second agent is preferably 1.5 to 50, more preferably 2 to 25, even more preferably 2 to 10, and ideally 2.5 to 7.5.

To further improve the stability of the hydrogen peroxide, the pH of the second agent is preferably 2 to 6, more preferably 3 to 5. The phenoxyethanol of component J and at least one component selected from benzoic acid and benzoates of component K act to improve the ease of mixing and foaming of the agent for bleaching or removing dye from hair and the ability of the foamy agent for bleaching or removing dye from hair to hold to the hair. They also act to improve the stability of the hydrogen peroxide when hydrogen peroxide is used as an oxidising agent. The second agent therefore preferably contains phenoxyethanol and at least one component selected from benzoic acid and benzoates. Examples of benzoates include sodium benzoate and potassium benzoate.

The phenoxyethanol content of the mixture of the first agent and second agent is preferably 0.05 to 1 wt%, more preferably 0.15 to 1 wt%, and even more preferably 0.15 to 0.8 wt%. When the phenoxyethanol content is within either of the above ranges, it is possible to satisfactorily obtain an effect of improvement in the ease of mixing and foaming of the agent for bleaching or removing dye from hair and in the ability of the foamy agent for bleaching or removing dye from hair to hold to the hair. Moreover, the stability of the hydrogen peroxide is further improved when hydrogen peroxide is used as an oxidising agent. In addition, the generation of odour from the phenoxyethanol can be suppressed.

The amount of at least one component selected from benzoic acid and benzoates, that is, component K, in the mixture of the first agent and second agent is preferably 0.01 to 1 wt%, more preferably 0.1 to 1 wt%, and even more preferably 0.15 to 1 wt%. When the component K content is within either of the above ranges, it is possible to satisfactorily obtain an effect of improvement in the ease of mixing and foaming of the agent for bleaching or removing dye from hair and in the ability of the foamy agent for bleaching or removing dye from hair to hold to the hair. The stability of the hydrogen peroxide is also further improved when hydrogen peroxide is used as an oxidising agent. Moreover, elevation of the alkalinity of the agent for bleaching or removing dye from hair that causes irritation of the scalp when the agent for bleaching or removing dye from hair is applied to the hair and decreases in the solubility of the benzoic acid and benzoates can also be suppressed.

To suppress decomposition of the hydrogen peroxide and improve the stability of the hydrogen peroxide when the second agent contains hydrogen peroxide, the weight ratio (J/K) of the phenoxyethanol content of the second agent to the content of at least one component selected from benzoic acid and benzoates in the second agent is preferably 0.2 to 2.5, more preferably 0.2 to 1.0.

The oxidising agent acts to decolourise the melanin contained in hair. Examples of oxidising agents include hydrogen peroxide, urea peroxide, melamine peroxide, sodium percarbonate, potassium percarbonate, sodium perborate, potassium perborate, ammonium persulphate, sodium peroxide, potassium peroxide, magnesium peroxide, barium peroxide, calcium peroxide, strontium peroxide, hydrogen peroxide adducts of sulphates, hydrogen peroxide adducts of phosphates, and hydrogen peroxide adducts of pyrophosphates.

The oxidising agent content of the second agent is preferably 0.1 to 15.0 wt%, more preferably 2.0 to 9.0 wt%, and ideally 3.0 to 6.0 wt%. When the oxidising agent content is within any of the above ranges, the melanin in the hair can be decolourised well without causing any significant damage to the hair when the agent for bleaching or removing dye from hair is applied to the hair.

To improve the stability of the hydrogen peroxide when hydrogen peroxide is contained as an oxidising agent in the second agent, the second agent preferably contains a stabiliser such as hydroxyethane diphosphonic acid or a salt thereof. Examples of hydroxyethane diphosphonates include tetrasodium hydroxyethane diphosphonate and disodium hydroxyethane diphosphonate.

The second agent may also contain components commonly contained in conventional agents to bleach or remove dye from hair as long as they do not interfere with the effects of the components of the second agent discussed above. For example, components other than alkali agents that can be contained in the first agent discussed above may be combined as is appropriate within the range that does not interfere with the effects of the present invention.

The form of the second agent is a liquid. The term 'liquid' in this specification is a concept that includes a gel, cream, and emulsion. For example, water, ethanol, n-propanol, isopropanol, methyl Cellosolve, ethyl Cellosolve, methyl carbitol, ethyl carbitol, benzyl alcohol, phenethyl alcohol, γ-phenylpropyl alcohol, cinnamic alcohol, anise alcohol, p-methylbenzyl alcohol, α-dimethylphenethyl alcohol, α-phenylethanol, phenoxyethanol, phenoxyisopropanol, 2-benzyloxyethanol, N-alkyl pyrrolidones, alkylene carbonates, and alkyl ethers can all be used as solvents to dissolve the components of the second agent. Water among them is preferably used because it has superior ability to dissolve the components of the second agent. When water is used as the solvent, the water content (at the time of use) of the mixture of the first agent and second agent is preferably 50 wt% or higher, more preferably 60 wt% or higher.

The second agent is prepared by mixing the components of the second agent. The order of mixture of the components of the second agent is not particularly restricted. To improve the stability of the hydrogen peroxide by improving the pH stability of the second agent through the action of the alkali metal inorganic salt, it is preferable to first mix the components of the second agent other than hydrogen peroxide to prepare an aqueous solution containing amphoteric surfactant, before mixing the hydrogen peroxide and amphoteric surfactant. The hydrogen peroxide and amphoteric surfactant are thus preferably mixed by mixing an aqueous solution containing hydrogen peroxide with this aqueous solution containing amphoteric surfactant.

With, for example, an amphoteric surfactant and water, the aqueous solution containing the amphoteric surfactant is prepared by mixing with the alkali metal inorganic salt after mixing all of the other components except for hydrogen peroxide and alkali metal inorganic salt. All of the above other components may be mixed into a mixture obtained by mixing the amphoteric surfactant and water with the alkali metal inorganic salt. Alternatively, the alkali metal inorganic salt and the remaining above other components may be mixed in the stated order after mixing the amphoteric surfactant and water with some of the above other components.

Alternatively, with alkali metal inorganic salt, the aqueous solution containing amphoteric surfactant can also be prepared by further mixing with the amphoteric surfactant after mixing all of the other components other than hydrogen peroxide and the amphoteric surfactant. All of the above other components may be mixed into a mixture obtained by mixing the alkali metal inorganic salt and amphoteric surfactant. Alternatively, the amphoteric surfactant and the remaining above other components may be further mixed in the stated order after mixing the alkali metal inorganic salt with some of the above other components.

The amphoteric surfactant content of the aqueous solution containing amphoteric surfactant is preferably 10 to 50 wt%, more preferably 20 to 40 wt%, and even more preferably 30 to 40 wt%. Good improvement of the stability of the hydrogen peroxide can be obtained if the amphoteric surfactant content is 10 wt% or higher. Precipitation of the amphoteric surfactant can be suppressed as long as the amphoteric surfactant content is 50 wt% or less.

The alkali metal inorganic salt content of the aqueous solution containing amphoteric surfactant is preferably 0.1 to 20 wt%, more preferably 0.5 to 15 wt%, even more preferably 2.5 to 12.5 wt%, and ideally 3 to 10 wt%. Good improvement of the stability of the hydrogen peroxide can be obtained when the alkali metal inorganic salt content is within any of the above ranges.

The weight ratio (A/I) of the amphoteric surfactant content of the aqueous solution containing amphoteric surfactant to the alkali metal inorganic salt content of the aqueous solution containing amphoteric surfactant is preferably 1.5 to 50, more preferably 2 to 25, even more preferably 2 to 10, and ideally 2.5 to 7.5. The stability of the hydrogen peroxide is further improved when this ratio is within any of the above ranges.

The amphoteric surfactant and alkali metal inorganic salt are preferably mixed with each other in concentrations within the respective prescribed ranges at the time of preparation of the aqueous solution containing amphoteric surfactant. For example, if the alkali metal inorganic salt is added after mixing water with the amphoteric surfactant, the amount of water added per part by weight of amphoteric surfactant is preferably 0.5 to 4 parts by weight, more preferably 1 to 3 parts by weight. When the amount of water added is less than 0.5 part by weight, there is a risk that the solubility of the amphoteric surfactant will decline. If the amount of water added exceeds 4 parts by weight, the concentration of amphoteric surfactant in the aqueous solution containing amphoteric surfactant decreases, as a result of which a risk is presented that the pH stability of the second agent decreases and the stability of the hydrogen peroxide decreases.

If, for example, the amphoteric surfactant is added after mixing water with the alkali metal inorganic salt, the amount of water added per part by weight of the alkali metal inorganic salt is preferably 5 to 30 parts by weight, more preferably 10 to 20 parts by weight. When the amount of water added is less than 5 parts by weight, there is a risk of decreased solubility of the alkali metal inorganic salt. When the amount of water added exceeds 30 parts by weight, the concentration of alkali metal inorganic salt in the aqueous solution containing amphoteric surfactant decreases, as a result of which a risk is presented that the pH stability of the second agent decreases and the stability of the hydrogen peroxide decreases.

The hydrogen peroxide content of the aqueous solution containing hydrogen peroxide mixed with the aqueous solution containing amphoteric surfactant at the time of preparation of the second agent is preferably 25 to 40 wt%, more preferably 30 to 35 wt%. When the hydrogen peroxide content is less than 25 wt%, the stability of the hydrogen peroxide in the second agent sometimes declines. When the hydrogen peroxide content exceeds 40 wt%, it becomes difficult to acquire such an aqueous solution containing hydrogen peroxide as a commercial product and there is a risk of an elevated rate of decomposition of the hydrogen peroxide during storage.

After a foam has been produced by mixing the first agent and the second agent and creating a foam through shaking, only the necessary amount of agent for bleaching or removing dye from hair is applied to the hair using a hand covered in a thin glove, comb, or brush.

### <Three-component agent for bleaching or removing dye from hair>

A three-component agent for bleaching or removing dye from hair is constituted, for example, of a first agent containing an alkali agent, a second agent having the same composition as the second agent of the two-component agent for bleaching or removing dye from hair, and a third agent having the composition of the first agent of the two-component agent for bleaching or removing dye from hair with the alkali agent removed. After a foam has been produced by mixing all of the first agent, second agent, and third agent and creating a foam through shaking, the agent for bleaching or removing dye from hair is applied to the hair to bleach or remove dye from the hair. A three-component agent for bleaching or removing dye from hair constituted in this way has good storage stability.

Next, a hair cosmetic product used to prepare an agent for bleaching or removing dye from hair having the form of a foam will be explained with reference to FIG 1(a) through FIG 2. As illustrated in FIG 1(a), the hair cosmetic product includes the agent for bleaching or removing dye from hair 10 and a fluid-tight, sealable container 20 as a foaming tool to shake the agent for bleaching or removing dye from hair 10. The first agent 11 and second agent 12 of the agent for bleaching or removing dye from hair 10 are stored housed in the container 20 in a separately packaged state until the time of use. The form of packaging of the first agent 11 and second agent 12 is not particularly restricted and may be, for example, any of bottle packaging, pillow packaging, and tube packaging.

The container 20 is provided with a bottomed cylindrical container body 21 and a hemispherical lid 22 that seals the opening of the container body 21. The container body 21 is shaped so that the opening is wider than the bottom. The inner surface of the container body 21 is also curved.

A flange-shaped fitting part is formed on the rim of the lid 22, and this fitting part fits the opening on the container body 21. In the container 20 illustrated in FIG 1(a), the fitting part of the lid 22 is fitted to the opening of the container body 21 so that the lid 22 is attached in a fluid-tight manner to the container body 21 by rotating the lid 22.

The container 20 is formed to be able to house the first agent 11 and second agent 12 in the forms in which they are individually packaged. In other words, as illustrated in FIG 1(a), container 20 is used as an outer container to store both the first agent 11 and second agent 12 until the time of use of the agent for bleaching or removing dye from hair 10. Not only the first agent 11 and second agent 12, but also gloves to be used during treatment to bleach or remove dye from the hair, instructions, and other such accessories may be housed in the container 20. The container 20 is preferably formed from resin or paper that has been made water resistant, from the viewpoint of obtaining a light weight. The outer surface of the container body 21 may be printed, for example, using shrink film.

At the time of use, the lid 22 is first removed from the container body 21, and the individually packaged first agent 11 and second agent 12 are taken out from inside the container body 21. Next, the packaging of the first agent 11 and second agent 12 is opened and, as illustrated in FIG 1(b), the first agent 11 and second agent 12 are introduced into the container body 21. The first agent 11 and second agent 12 are brought into contact in this way in the container body 21, and a mixture 13 is obtained. Next, as illustrated in FIG 1(c), the lid 22 is attached to the container body 21, and the container 20 is shaken up and down. This both promotes mixing of the first agent 11 and second agent 12 inside the container 20 and also mixes air with the mixture 13 by shaking. This shaking and mixture of air with the mixture 13 makes the mixture 13 foam. After shaking the container 20 the prescribed number of times, foaming is completed by stopping shaking. A foamy agent for bleaching or removing dye from hair 14 obtained in this way is removed directly from the container body 21 by hand and applied to the hair after removing the lid 22 from the container body 21, as illustrated in FIG 1(d). Since the agent for bleaching or removing dye from hair is a foam, it can hold to the hair easily without dripping. Colour or dye removal from the hair advances during the standing time after the agent for bleaching or removing dye from hair has been applied to the hair. After the prescribed length of time has passed, the agent for bleaching or removing dye from hair is removed by washing the hair with water or warm water, and treatment to remove colour or dye from hair is completed.

The container body 21 has the shape of a bottomed cylinder that is wider at the opening than at the bottom, as illustrated in FIG 2, whereby the foamy agent for bleaching or removing dye from hair 14 inside the container body 21 is removed easily, for example, by hand. The foamy agent for bleaching or removing dye from hair 14 inside the container body 21 can be removed easily, even by hand, with little left behind because the inner surface of the container body 21 is curved.

The ratio (mL/g) of the inner capacity (mL) of the container 20 to the weight (g) of the mixture 13 of the first agent 11 and second agent 12 of the agent for bleaching or removing dye from hair is preferably in the 2 to 15 range. When this ratio is 2 or higher, it is easy to ensure empty space inside the container 20 into which the mixture 13 is placed, thereby promoting the admixture of air into the mixture 13 by shaking. As a result, the number of times the container must be shaken until the mixture 13 is adequately foamed can be reduced. However, when the above ratio exceeds 15, there is a risk that the increase in excess space inside the container 20 after foaming has been completed will make it difficult to remove the foamy agent for bleaching or removing dye from hair 14 inside the container 20.

The container 20 has a larger dimension in the direction of height than the dimension in the radial direction. This makes it easy to hold the container 20. The height of the housing part inside the container 20 indicated by h1 in FIG 2 is preferably 10 to 25 cm. When this height h1 is 10 cm or more, it is easy to ensure adequate capacity of the container 20. However, when the height h1 exceeds 25 cm, there is a risk that the container 20 will be too large and become difficult to hold.

The ratio (h1/h2) of the height h1 (cm) of the housing part inside the container 20 to the height (cm) of the mixture 13 of agent for bleaching or removing dye from hair inside the container 20 before foaming shown by h2 in FIG 2 is preferably in the 2 to 15 range. When this ratio is 2 or higher, it is easy to ensure empty space inside the container 20 into which the mixture 13 is placed, thereby promoting the admixture of air into the mixture 13 by shaking. As a result, the number of times the container must be shaken until the mixture 13 is adequately foamed can be reduced. However, when the ratio h1/h2 exceeds 15, there is a risk that the increase in excess space inside the container 20 after foaming has been completed will make it difficult to remove the foamy agent for bleaching or removing dye from hair 14 inside the container 20.

This embodiment has the following advantages. The two-component agent for bleaching or removing dye from hair of this embodiment is constituted of a powdered first agent and a liquid second agent. The bubbles are small in the foamy agent for bleaching or removing dye from hair obtained by mixing this first agent and second agent and creating a foam through shaking. The agent for bleaching or removing dye from hair can be foamed simply by mixing since the use of foam homogenisation means as described in Patent Reference 3 is unnecessary in this case. The small bubbles also allow the foamy agent for bleaching or removing dye from hair to hold well to the hair.

The agent for bleaching or removing dye from hair can be made to foam to a high degree easily by using a container 20 of the simple construction illustrated in FIG 1(a) through FIG 2 without using a foamer container of a complex construction or any propellant as in an aerosol. A relatively large amount of foamy agent for bleaching or removing dye from hair can also be obtained easily in one operation when this container 20 is used. Moreover, the foaming operation using this container 20 can be conducted enjoyably because it is easy and does not require any training.

When the agent for bleaching or removing dye from hair contains an amphoteric surfactant, the ease of mixing and foaming of the agent for bleaching or removing dye from hair improves, and the ability of the foamy agent for bleaching or removing dye from hair to hold to the hair also improves. An amphoteric surfactant can also adjust the second agent to the proper viscosity by emulsification and improve the viscosity stability.

When the agent for bleaching or removing dye from hair contains a cationic surfactant, the ease of mixing and foaming of the agent for bleaching or removing dye from hair improves, and the ability of the foamy agent for bleaching or removing dye from hair to hold to the hair also improves.

When the agent for bleaching or removing dye from hair contains an anionic surfactant in addition to a cationic surfactant, the agent for bleaching or removing dye from hair is preferably constituted so that the anionic surfactant and cationic surfactant come into contact in the presence of a solvent at the time of use of the agent for bleaching or removing dye from hair. This further improves the ease of mixing and foaming of the agent for bleaching or removing dye from hair, and further improves the ability of the foamy agent for bleaching or removing dye from hair to hold to the hair. Together with improving the foamability of the agent for bleaching or removing dye from hair, the durability of the foam obtained by foaming also improves.

When the cationic surfactant contains an ammonium-type cationic surfactant comprising an alkyl group having from 16 to 22 carbon atoms and an ammonium-type cationic surfactant comprising an alkyl group having from 10 to fewer than 16 carbon atoms, the ease of mixing and foaming of the agent for bleaching or removing dye from hair improves further, and the ability of the foamy agent for bleaching or removing dye from hair to hold to the hair also improves further. The foam quality of the foamy agent for bleaching or removing dye from hair is also improved.

When the agent for bleaching or removing dye from hair contains an anionic surfactant or amphoteric surfactant, it becomes easier to increase the foamability of the agent for bleaching or removing dye from hair. When the first agent of the agent for bleaching or removing dye from hair contains a nonionic polymer, the ease of mixing and foaming of the agent for bleaching or removing dye from hair improves, and the ability of the foamy agent for bleaching or removing dye from hair to hold to the hair also improves. The foam quality of the foamy agent for bleaching or removing dye from hair, for example, the foam homogeneity and elasticity, also improves.

When the first agent of the agent for bleaching or removing dye from hair contains a thickener, the ease of mixing and foaming of the agent for bleaching or removing dye from hair improves, and the ability of the foamy agent for bleaching or removing dye from hair to hold to the hair also improves. The thickener also imparts proper viscosity to the agent for bleaching or removing dye from hair and thereby makes it more difficult for the agent for bleaching or removing dye from hair to drip from the hair when the agent for bleaching or removing dye from hair has been applied to the hair.

The agent for bleaching or removing dye from hair may contain an anionic surfactant, cationic surfactant, thickener other than starch, and starch. In this case, the starch is combined with the first agent having the form of a powder, and the anionic surfactant and cationic surfactant may be brought into contact in the presence of a solvent at the time of use of the agent for bleaching or removing dye from hair. The thickening effect can be rapidly achieved when the first agent and second agent are mixed in this case.

When the starch is combined with the powdered first agent, the viscosity stability of the agent for bleaching or removing dye from hair can be improved in addition to hastening the rate of onset of the thickening effect when the first agent and second agent are mixed. When the second agent of the agent for bleaching or removing dye from hair contains a cationic polymer as well, the ease of mixing and foaming of the agent for bleaching or removing dye from hair improves, and the ability of the foamy agent for bleaching or removing dye from hair to hold to the hair also improves. The feel of the hair after treatment by the agent for bleaching or removing dye from hair can also be improved. The improvement of the feel of the hair by the cationic polymer can be increased in particular when the cationic polymer and hydrogen peroxide are combined together with either one of the first agent or second agent.

As also occurs when the second agent of the agent for bleaching or removing dye from hair contains an alkali metal inorganic salt, the ease of mixing and foaming of the agent for bleaching or removing dye from hair improves, and the ability of the foamy agent for bleaching or removing dye from hair to hold to the hair also improves. The alkali metal inorganic salt can also improve the stability of the hydrogen peroxide when hydrogen peroxide is used as an oxidising agent and an amphoteric surfactant is combined in the second agent.

When the second agent of the agent for bleaching or removing dye from hair contains phenoxyethanol and at least one component selected from benzoic acid and benzoates as well, the ease of mixing and foaming of the agent for bleaching or removing dye from hair improves, and the ability of the foamy agent for bleaching or removing dye from hair to hold to the hair also improves. Phenoxyethanol, benzoic acid, and benzoates can also improve the stability of the hydrogen peroxide when hydrogen peroxide is used as an oxidising agent.

The stability of the hydrogen peroxide can be further improved by using in combination a surfactant, phenoxyethanol, and at least one component selected from benzoic acid and benzoates, when hydrogen peroxide is used as an oxidising agent.

The alkali agent is preferably a carbonate. When the chelating agent content of the mixture of the first agent and second agent is 1 to 5 wt%, the weight ratio of the carbonate content of the mixture to the chelating agent content of the mixture of the first agent and second agent is preferably 0.02 to 6.5. In this case as well, the ease of mixing and foaming of the agent for bleaching or removing dye from hair improves, and the ability of the foamy agent for bleaching or removing dye from hair to hold to the hair also improves. Deterioration of the feel of the hair after treatment by the agent for bleaching or removing dye from hair caused by the use of carbonate can also be suppressed.

### (Second embodiment)

A second embodiment in which the cosmetic preparation for hair which is applied in the method of dyeing hair or bleaching hair or removing dye from hair of the present invention is embodied by a two-component hair dye is explained below. The hair dye of this embodiment is constituted of a powdered first agent 11 including an alkali agent and a liquid second agent 12 containing hydrogen peroxide as an oxidising agent. This hair dye is applied to the hair to dye the hair after having been made into the form of a foam by mixing the first agent and second agent and creating a foam through shaking.
The first agent also contains, in addition to the alkali agent of component F, for example, an anionic surfactant of component C, nonionic polymer of component D, thickener of component E, chelating agent of component L, and oxidation dye.

The oxidation dye makes colouring possible due to oxidative polymerisation by the oxidising agent contained in the second agent of the hair dye, and contains at least a dye intermediate. The oxidation dye may also contain a coupler in addition to the dye intermediate. Examples of dye intermediates include p-phenylenediamine, toluene-2,5-diamine (p-toluylenediamine), N-phenyl-p-phenylenediamine, 4,4'-diaminodiphenylamine, p-aminophenol, o-aminophenol, p-methylaminophenol, N,N-bis(2-hydroxyethyl)-p-phenylenediamine, 2-hydroxyethyl-p-phenylenediamine, o-chloro-p-phenylenediamine, 4-amino-m-cresol, 2-amino-4-hydroxyethylaminoanisole, 2,4-diaminophenol, and salts of these. The dye intermediate used may be only one type, or two or more types of dye intermediates may be used in combination. The coupler produces colour by bonding with the dye intermediate. Examples of couplers include resorcinol, 5-amino-o-cresol, m-aminophenol, α(alpha)-naphthol, 5-(2-hydroxyethylamino)-2-methylphenol, m-phenylenediamine, 2,4-diaminophenoxyethanol, toluene-3,4-diamine, 2,6-diaminopyridine, diphenylamine, N,N-diethyl-m-aminophenol, phenylmethyl pyrazolone, and salts of these. The coupler used may be only one type, or two or more types of couplers may be used in combination. An oxidation dye that contains both a dye intermediate and a coupler is preferably used because it makes it easy to change the colour of the hair as desired.

The first agent of the hair dye may also appropriately contain at least one component selected from direct dyes and oxidation dyes listed, for example, in the Japanese Standards of Quasi-drug Ingredients (JSQI) (published June 2006, Yakuji Nippo, Ltd).

The second agent of the hair dye has, for example, the same composition as the second agent of the agent for bleaching or removing dye from hair of the first embodiment. The form of the first agent of the hair dye is a powder. The form of the second agent is a liquid. After a foam has been produced by mixing the first agent and the second agent and creating a foam through shaking, only the necessary amount of hair dye is applied to the hair using a hand covered in a thin glove, a comb, or a brush. The hair dye having the form of a foam may be prepared using the container 20 illustrated in FIG 1(a) to FIG 2, in the same manner as the agent for bleaching or removing dye from hair of the first embodiment.

The second embodiment has the following advantages in addition to the advantages of the first embodiment. The hair dye of the second embodiment is constructed from a powdered first agent and a liquid second agent. The foamy hair dye obtained by mixing the first agent and second agent and creating a foam through shaking is a small foam. Therefore, the hair dye can be made to foam simply by mixing, as in the case of the agent for bleaching or removing dye from hair of the first embodiment.

Modifications such as the following may be made to the first and second embodiments. The first agent is a powder and the second agent is a liquid in the cosmetic preparation for hair of the first and second embodiments.

The two-component hair dye of the second embodiment may be modified and constituted of three components or multiple components comprising four or more components including at least a powdered agent and a liquid agent. The foaming procedure carried out to obtain a foamy cosmetic preparation for hair in the first and second embodiments may be modified as follows. Specifically, the cosmetic preparation for hair may be made into a foam by admixing air by applying vibration to the un-foamed cosmetic preparation for hair. Alternatively, the cosmetic preparation for hair may be made into a foam by admixing air by rotating the un-foamed cosmetic preparation for hair. In sum, foaming by shaking the cosmetic preparation for hair implies making the cosmetic preparation for hair into a foam by mixing air into the cosmetic preparation for hair by shaking, applying vibration to the cosmetic preparation for hair, or applying rotation to the cosmetic preparation for hair.

The foaming tool used to foam the cosmetic preparation for hair can be modified in accordance with the type of foaming procedure. For example, when the cosmetic preparation for hair is made to foam mainly by applying vibration, a shaker is suitable as the foaming tool. The cosmetic preparation for hair that employs such foaming tools is foamed inside a container, for example, by placing the cosmetic preparation for hair in a container having an opening at the upper end. The cosmetic preparation for hair can be made to foam easily and well in all of these instances as well.

The container body 21 of the container 20 illustrated in FIG 1(a) to FIG 2 is not limited to having the configuration of a bottomed round cylinder; it may, for example, be a bottomed cylinder of polygonal cross-section. The shape of the lid 22 may also be modified appropriately in accordance with the shape of the container body 21.

The first agent and second agent packaged individually before use are not limited to being housed and stored in the container 20 as illustrated in FIG 1(a). Specifically, at least one of the first agent and second agent may be stored outside the container 20.

When the cosmetic preparation for hair is made to foam by shaking and mixing air into it, the container 20 may be shaken by twisting the wrist of the hand holding the container 20, for example, rather than by shaking the container 20 up and down as illustrated in FIG 1(c). Working Examples
The present invention is explained more concretely below through working and comparative examples.

### Test 1

The agents for removing colour from hair of Working Examples 1-1 to 1-12 and Comparative Examples 1-1 to 1-8 were prepared. The agents for removing colour from hair of Working Examples 1-1 to 1-12 are two-component preparations comprising a powdered first agent and a milky-liquid-form second agent. The first and second agents of the agents for removing colour from hair of these working examples have the compositions shown in Table 1. The agents for removing colour from hair of Comparative Examples 1-1 to 1-8 are two-component preparations comprising liquid first and second agents. The first and second agents of the agents for removing colour from hair of these comparative examples have the compositions shown in Table 2. In Tables 1 and 2, The numerical values representing the contents of each component of the agents for removing colour from hair are expressed in units of percentage by weight. The first agent and second agent of the agents for removing colour from hair of the working examples were mixed in a weight ratio of 1:5. The first agent and second agent of the agents for removing colour from hair of the comparative examples were mixed in a weight ratio of 1:1.5.

In Working Examples 1-1 to 1-11 and Comparative Examples 1-3 and 1-7, the first agent and second agent were mixed using a shaker comprising the same container as the container 20 illustrated in FIG 1(a) to FIG 2. More specifically, a foamy hair bleaching agent was obtained by shaking the container 20 times after placing the first agent and second agent in the container. In Working Example 1-12, a foamy hair bleaching agent was obtained using a whisk (muddler for hair colour). In Comparative Examples 1-1 and 1-5, a foamy hair bleaching agent was obtained by mixing the first agent and second agent without foaming and then discharging from the container using a squeeze foamer container 1 (Puritia (made by Kao Corp) accessory container). In Comparative Examples 1-2 and 1-6, a foamy hair bleaching agent was obtained by mixing the first agent and second agent without foaming and then discharging from the container using a pumping container (F5L type foamer container, made by Daiwa Can Company). In Comparative Examples 1-4 and 1-8, a foamy hair bleaching agent was obtained by discharging after mixing the first agent and second agent by shaking using a squeeze foamer container 2 (one having the suction dip tube removed from the Puritia (made by Kao Corp.) accessory container). The ease of mixing and foaming and the ability to hold to the hair were evaluated for the hair bleaching agent of each of the examples. The results are shown in Tables 1 and 2.

The column 'Carbonate weight ratio (f-1)/L' in Table 1 shows the weight ratio of the content of carbonate in the hair bleaching agent to the chelating agent content of the hair bleaching agent at the time of use.

### <Ease of mixing and foaming>

Whether or not the agents for removing colour from hair of each of the examples foamed easily by mixing the first agent and second agent was evaluated on the following scale of five. 5: Very easy 4: Easy 3: Fairly easy 2: Not very easy 1: Not easy at all

### <Ability to hold to hair>

A 30 cm long strand of hair was hung, and the foamy hair bleaching agent of each of the examples was applied to the top of the strand. The movement of the applied foam was observed upon the foam being allowed to stand for 30 minutes thereafter, and was evaluated on the following scale of five.

| | |
|---|---|
| 5: The foam did not move at all | 4: The foam virtually did not move |
| 3: The foam did not move much | 2: The foam moved considerably |
| 1: The foam moved a lot | |

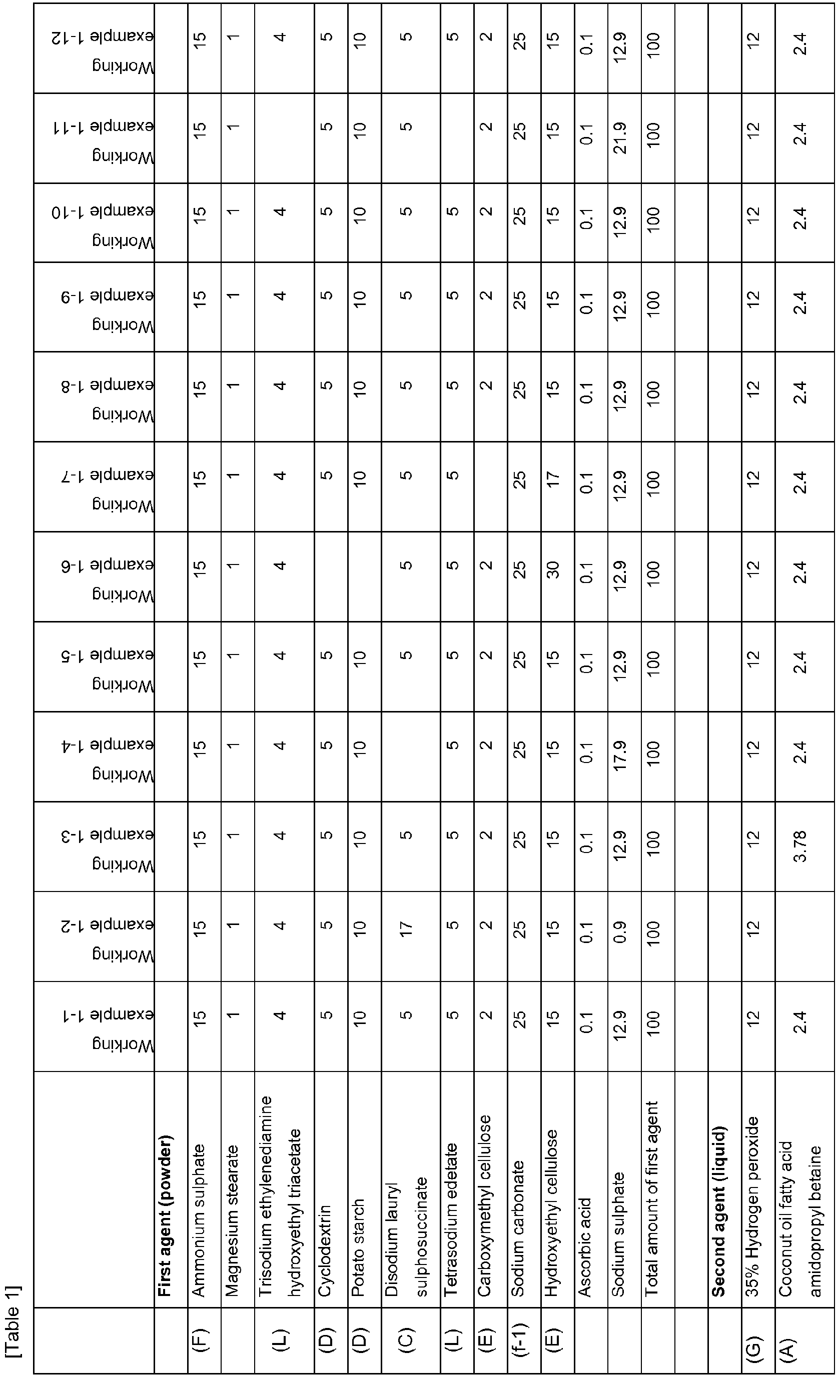

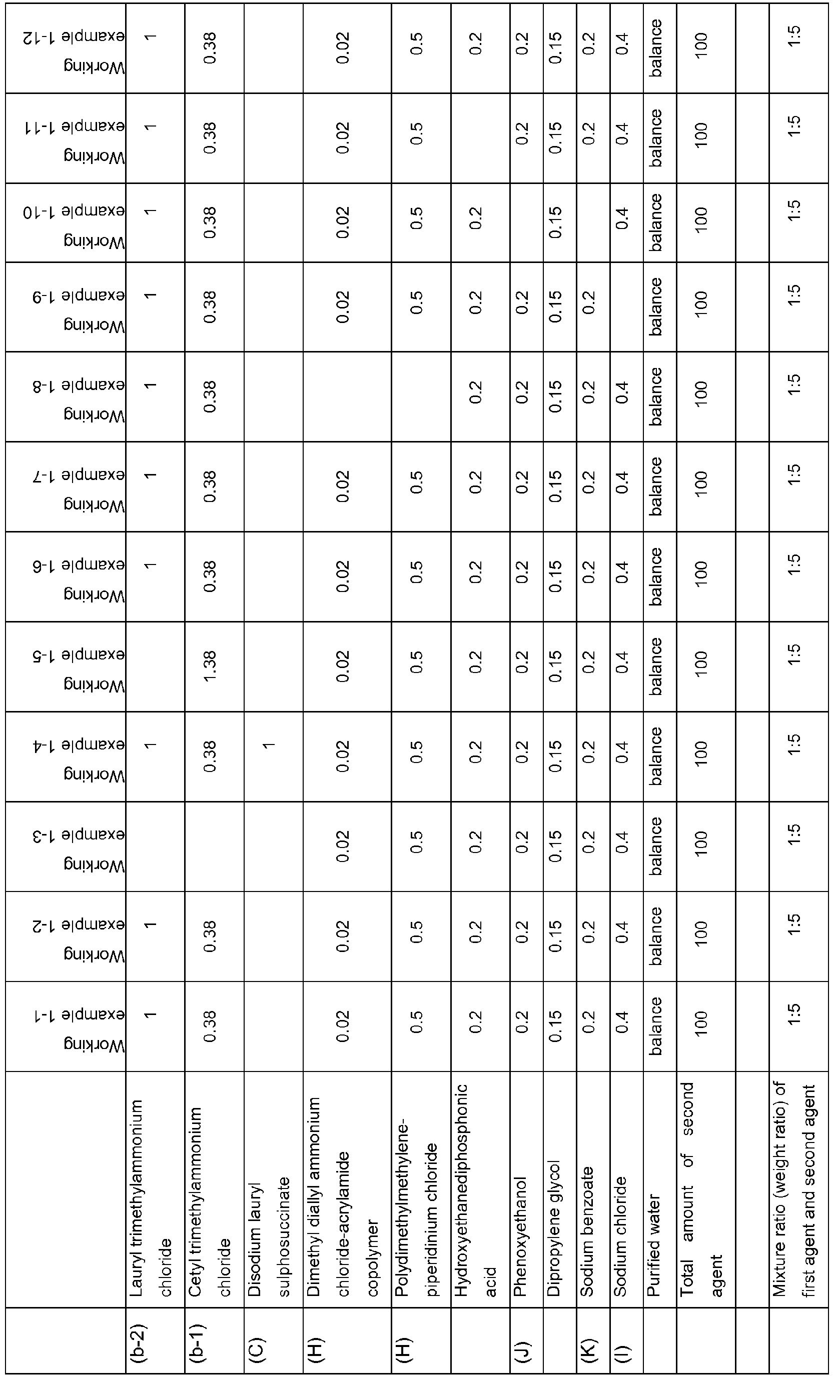

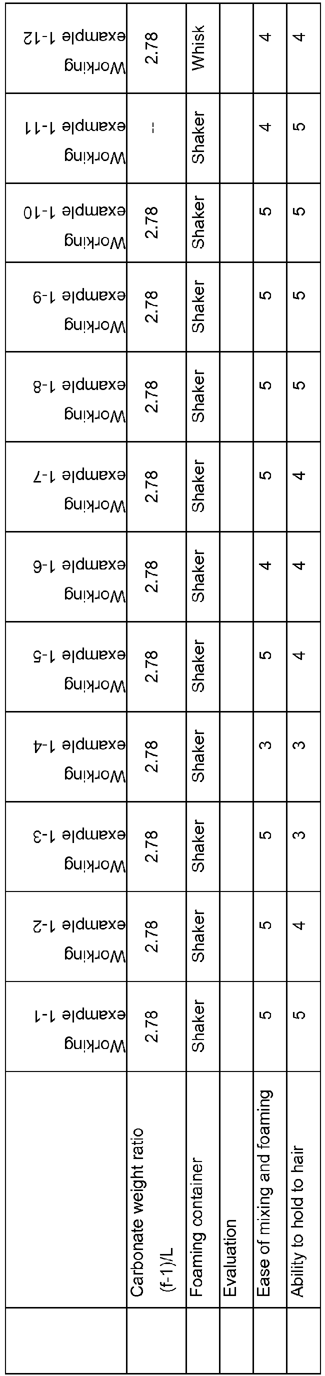

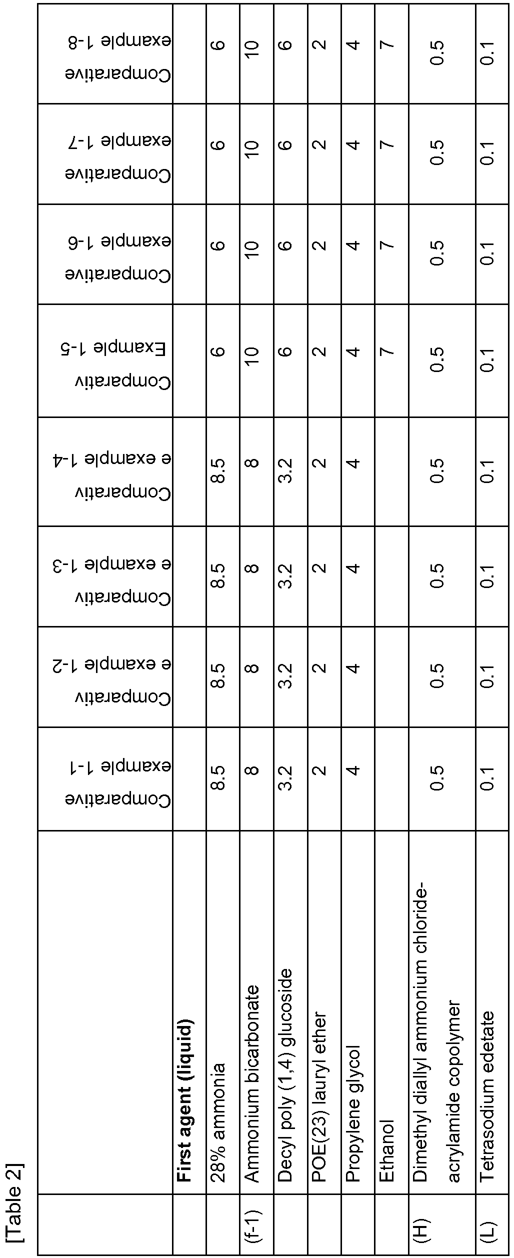

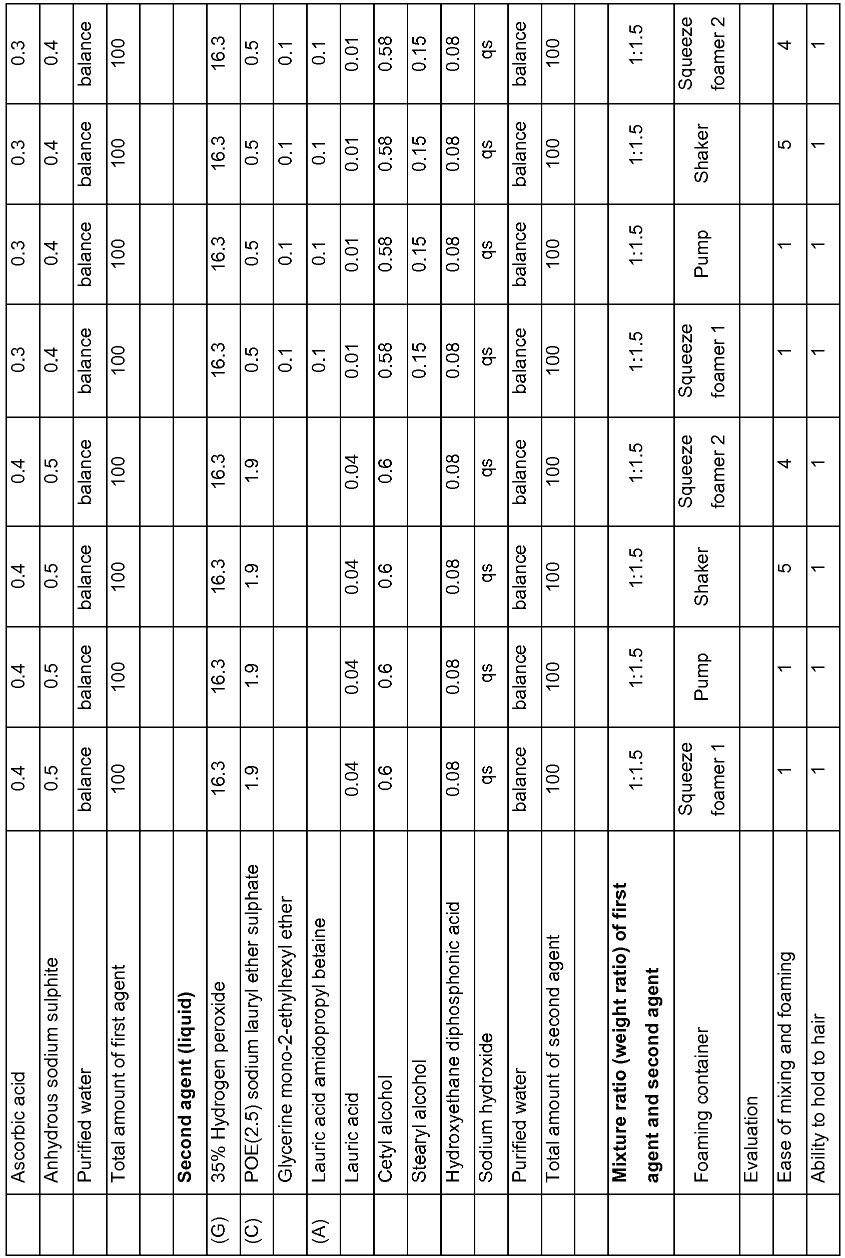

As shown in Table 1, both the evaluation of the ease of mixing and foaming and the evaluation of the ability to hold to the hair were good in the working examples in which the first agent was a powder and the second agent was a liquid and foamy agents for removing colour from hair were prepared by shaking using a shaker.

As shown in Table 2, both the evaluation of the ease of mixing and foaming and the evaluation of the ability to hold to the hair were lower than in the working examples in Comparative Examples 1-1 and 1-5 in which foamy agents for removing colour from hair were prepared using a squeeze foamer container 1 by discharging from the container after mixing the first agent and second agent without foaming.

Both the evaluation of the ease of mixing and foaming and the evaluation of the ability to hold to the hair were lower than in the working examples in Comparative Examples 1-2 and 1-6 in which foamy agents for removing colour from hair were prepared using a pumping container by discharging from the container after mixing the first agent and second agent without foaming.

The evaluation of the ability to hold to the hair was lower than in the working examples in Comparative Examples 1-3 and 1-7 in which both the first agent and second agent were liquids and agents for removing colour from hair were prepared by shaking using a shaker. The evaluation of the ability to hold to the hair was lower than in the working examples in Comparative Examples 1-4 and 1-8 in which foamy agents for removing colour from hair were prepared using a squeeze foamer container 2 by discharging from the container after mixing the first agent and second agent by shaking.

### Test 2 (Working Examples 2-1 to 2-9)

The oxidation hair dyeing agents of Working Examples 2-1 to 2-9 were prepared. These oxidation hair dyeing agents were two-component preparations comprising a powdered first agent and a liquid second agent. The compositions of the first agent and second agent were as shown in Table 3. In Table 3, the numerical values representing the amount of each component of the oxidation hair dyeing agent are expressed in weight-percentage units.

Next, the oxidation hair dyeing agent of each example was made to foam using a sealable container (A1) the same as the container 20 illustrated in FIG 1(a) to FIG 2 or a whisk (B) for oxidation hair dyeing agent preparation. The volume of the sealable container used was 770 mL, the height was 17 cm, and the inner diameter was 7 to 8 cm. One hundred fifty grams of a mixture of the first agent and second agent was placed in this container, and the container was shaken vertically twenty times. A foamy oxidation hair dyeing agent was obtained by shaking and mixing air into the mixture of the first agent and second agent in this way. Alternatively, after placing 150 g of a mixture of the first agent and second agent in a 600 mL cup, a foamy oxidation hair dyeing agent was obtained by stirring the mixture by rotating the whisk the prescribed number of times.
Reference Example 2-1 A two-component oxidation hair dyeing agent of the same composition as the oxidation hair dyeing agent of Working Example 2-1 was made to foam according to the usual method using a squeeze-type foamer container (C).
Reference Example 2-2 A two-component oxidation hair dyeing agent of the same composition as the oxidation hair dyeing agent of Working Example 2-1 was made to foam according to the usual method using a pump-type foamer container (D).
Reference Examples 2-3 and 2-4 Two-component oxidation hair dyeing agents of a composition having the amphoteric surfactant removed from the composition of the oxidation hair dyeing agent of Working Example 2-1 were foamed in the same manner as in Working Example 2-1.

### <Amount of foam>

The state of the oxidation hair dyeing agents of each of the examples after foaming was examined visually by a panel of experts. The results were assessed using criteria that assigned an evaluation of 5 to a highly exceptional amount of foam, an evaluation of 4 to an excellent amount of foam, an evaluation of 3 to good foaming, an evaluation of 2 to a fair amount of foam, and an evaluation of 1 to a poor amount of foam. The numerical values of the evaluation results are shown in the 'Amount of foam' column in Table 3.

### <Fineness of foam>

The state of the oxidation hair dyeing agents of each of the examples after foaming was examined visually by a panel of experts. The results were evaluated by criteria that assigned an evaluation of 5 to highly exceptional foam fineness, an evaluation of 4 to excellent foam fineness, an evaluation of 3 to good foam fineness, an evaluation of 2 to somewhat coarse foam, and an evaluation of 1 to very coarse foam. The numerical values of the evaluation results are shown in the 'Foam fineness' column in Table 3.

### <Ease of handling after foaming>

The way in which the agent held to the hand when the oxidation hair dyeing agents of each of the examples were taken by a hand covered by a polyethylene glove after foaming and held to the hair was examined by a panel of experts. The results were evaluated by criteria that assigned an evaluation of 5 to highly exceptional ability to hold to the hand, an evaluation of 4 to excellent holding ability, an evaluation of 3 to good holding ability, an evaluation of 2 to fair holding ability, and an evaluation of 1 to poor holding ability. The numerical values of the evaluation results are shown in the 'Ease of handling after foaming' column in Table 3.

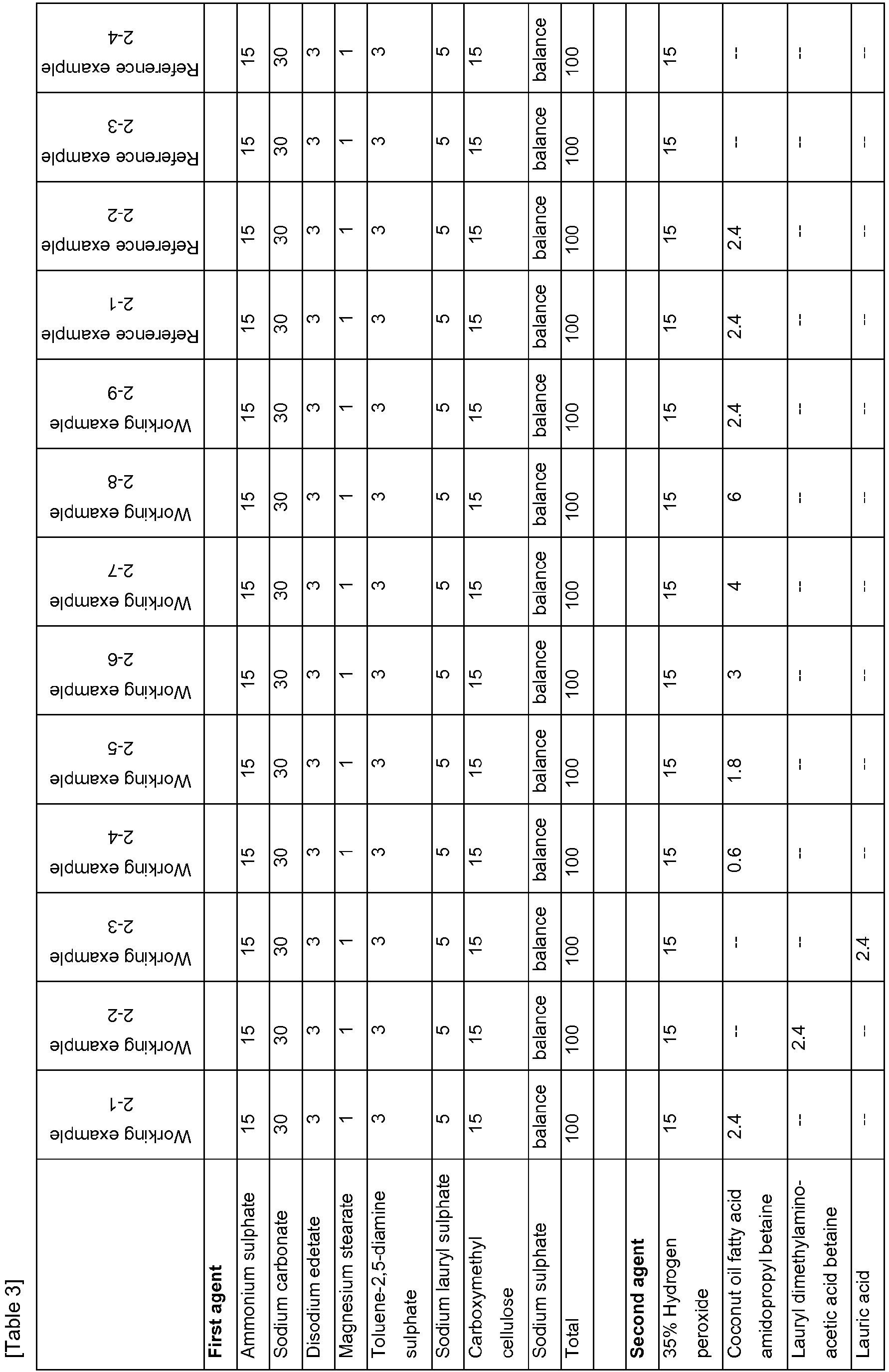

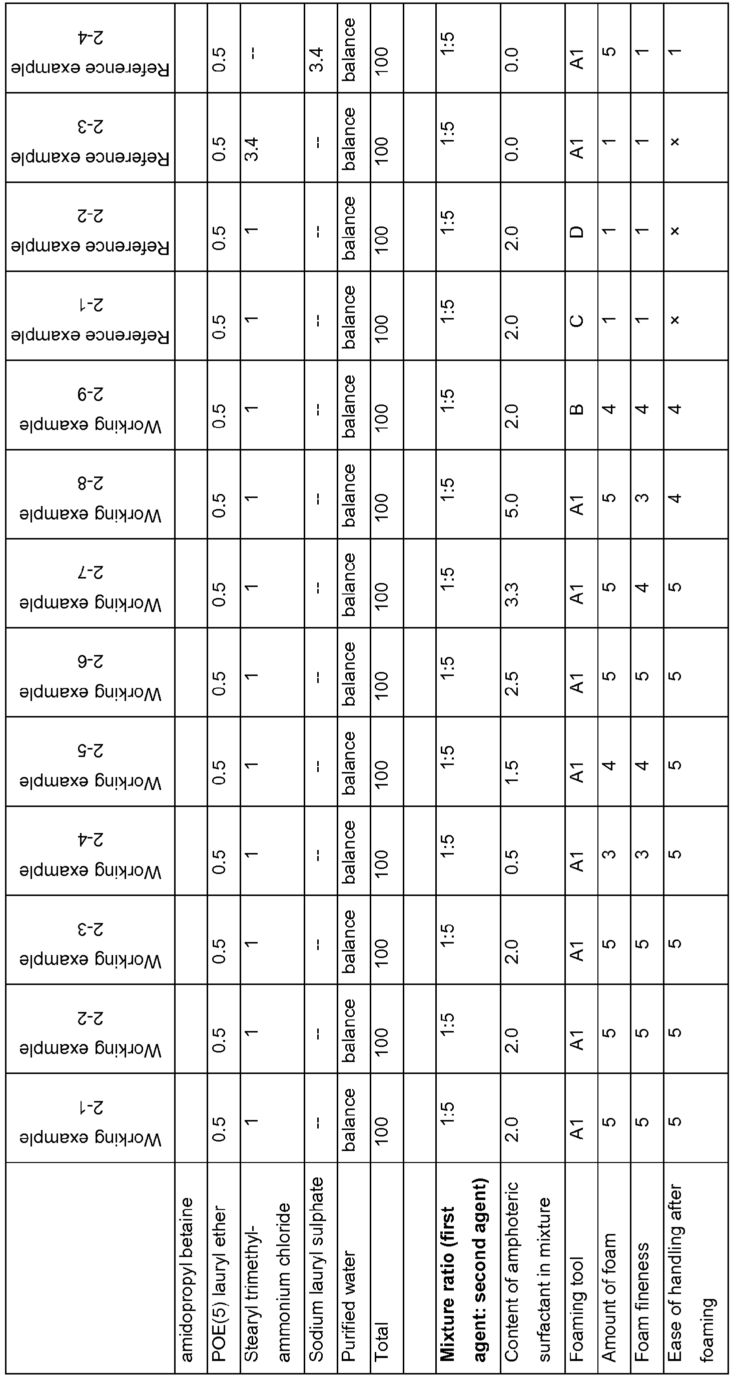

As shown in Table 3, all evaluations of the amount of foam, foam fineness, and ease of handling after the foaming procedure were 3 or higher in Working Examples 2-1 to 2-9. In Working Examples 2-1 to 2-3, 2-5 to 2-7, and 2-9 in particular, all evaluations of the amount of foam, foam fineness, and ease of handling after the foaming procedure were 4 or higher. It is understood from these results that the amphoteric surfactant content of the oxidation hair dyeing agent (in the mixture) is preferably in the range of 1.5 to 4.0 wt%.

In contrast to this, the evaluation of the amount of foam was 1 in Reference Examples 2-1 and 2-2 because a squeeze-type foamer container and a pump-type foamer container, respectively, were used as the foaming tool. Even though the same sealable container as in Working Example 2-1 was used in Reference Example 2-3, the evaluation of the amount of foam was 1 because the oxidation hair dyeing agent contained cationic surfactant instead of amphoteric surfactant. In Reference Example 2-4, the evaluation of the foam fineness was 1 because the oxidation hair dyeing agent contained anionic surfactant instead of amphoteric surfactant. The 'x' in the 'Ease of handling after foaming' column of Table 3 for Reference Examples 2-1 to 2-3 means that evaluation was not possible because foaming did not occur.

Working Example 2-1a A foamy oxidation hair dyeing agent was obtained in the same manner as in Working Example 2-1 except that the number of vertical shakes was changed to 10.
Working Examples 2-1b and 2-1c A foamy oxidation hair dyeing agent was obtained in the same manner as in Working Example 2-1 except that sealable containers of different volumes (A2 and A3) were used, and the number of vertical shakes was changed to 10.

Working Examples 2-1d to 2-1f A foamy oxidation hair dyeing agent was obtained in the same manner as in Working Example 2-1 except that the amount of oxidation hair dyeing agent introduced into the sealable container (A1) was changed and the number of vertical shakes of the sealable container (A1) was changed to 10.

### Evaluation

The above <amount of foam> and <foam fineness> were evaluated in Working Examples 2-1a to 2-1f. The numerical values of the evaluations are shown in the 'Amount of foam' and 'Foam fineness' columns of Table 4.

**[Table 4]**

| | | Working example 2-1a | Working example 2-1b | Working example 2-1c | Working example 2-1d | Working example 2-1e | Working example 2-1f |
|---|---|---|---|---|---|---|---|
| Sealable container | Type | A1 | A2 | A3 | A1 | A1 | A1 |
| | Inner capacity (mL) | 770 | 230 | 2000 | 770 | 770 | 770 |
| | Height of housing part (cm) | 17 | 7 | 30 | 17 | 17 | 17 |
| Mixture of first and second agent | Weight (g) | 150 | 150 | 150 | 30 | 72 | 450 |
| | Height before foaming (cm) | 4 | 4 | 2.5 | 1 | 2 | 10 |
| Inner capacity (mL)/weight of mixture (g) | | 5.1 | 1.5 | 13.3 | 25.7 | 10.7 | 1.7 |
| Height of housing part (cm)/height of mixture before foaming (cm) | | 4.1 | 1.8 | 12.0 | 17.0 | 8.5 | 1.7 |
| Amount of foam | | 5 | 3 | 5 | 3 | 4 | 3 |
| Foam fineness | | 5 | 3 | 4 | 3 | 4 | 3 |

As shown in Table 4, the evaluation of the amount of foam and the evaluation of the foam fineness were 4 or 5 in Working Examples 2-1a, 2-1c, and 2-1e, which was better than in Working Examples 2-1b and 2-1d. It is understood from these results that the ratio (mL/g) of the inner capacity (unit mL) of the sealable container to the weight (unit g) of the oxidation dye (mixture) is preferably in the 2 to 15 range.

### Test 3 Working Examples 3-1 to 3-8 and Reference Examples 3-1 to 3-4

The oxidation hair dyeing agents of Working Examples 3-1 to 3-8 and Reference Examples 3-1 to 3-4 were prepared. These oxidation hair dyeing agents were two-component preparations comprising a powdered first agent and a liquid second agent, and the compositions of the first agent and second agent were as shown in Tables 5 and 6. The numerical values representing the amount of each component of the oxidation hair dyeing agents are expressed in units of percentage by weight in Tables 5 and 6 and subsequently in Tables 7 and 8. The form of the first agent may not necessarily be a powder, and the form of the second agent may not necessarily be a liquid. The mixture ratios in Tables 5 to 8 are weight ratios.

Next, the oxidation hair dyeing agents of each example were foamed using a sealable container the same as container 20 illustrated in FIG 1(a) to FIG 2. Furthermore, the volume of the sealable container used was 770 mL, the height was 17 cm, and the inner diameter was 7 to 8 cm. One hundred fifty grams of the mixture of the first agent and second agent was placed in this container, and the container was shaken up and down 20 times. A foamy oxidation hair dyeing agent was obtained by shaking and mixing air into the mixture of the first agent and second agent in this way.

Working Examples 3-9 to 3-24 Foamy oxidation hair dyeing agents were obtained in the same manner as in Working Example 3-1 from two-component oxidation hair dyeing agents of a different composition from the oxidation hair dyeing agent of Working Example 3-1 in terms of the content of at least one of the anionic surfactant and cationic surfactant.

### <Foamability>

The state of the oxidation hair dyeing agent of each example after foaming was examined visually by a panel of experts. The results were evaluated according to the following criteria.
Evaluation 5: Highly exceptional amount of foaming
Evaluation 4: Excellent amount of foaming
Evaluation 3: Good amount of foaming
Evaluation 2: Fair amount of foaming
Evaluation 1: Poor amount of foaming
The numerical values of the evaluations are shown in the 'Foamability' column in Tables 5 to 8.

### <Evaluation of retention of form>

The changes over time in the state of the oxidation hair dyeing agent of each example after foaming were examined visually by a panel of experts. The results assigned an evaluation of 5 to those in which virtually no defoaming (foam breakdown) could be confirmed after 10 minutes from immediately after foaming, an evaluation of 4 to those in which defoaming (foam breakdown) of about 10% was confirmed after 10 minutes, an evaluation of 3 to those in which defoaming (foam breakdown) of about 20% was confirmed after 10 minutes, an evaluation of 2 to those in which defoaming (foam breakdown) of about 30% was confirmed after 10 minutes, and an evaluation of 1 to those in which defoaming (foam breakdown) of about 40% was confirmed after 10 minutes. The numerical values of the evaluation results are shown in the 'Retention of form' column in Tables 5 to 8.

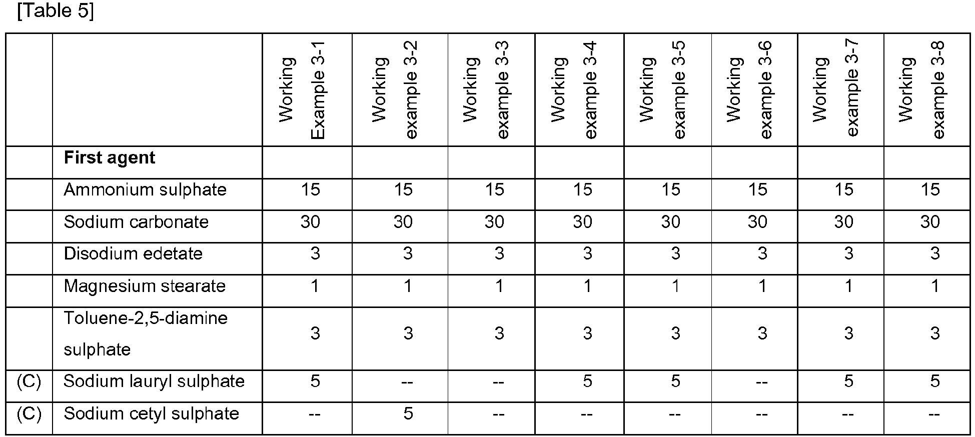

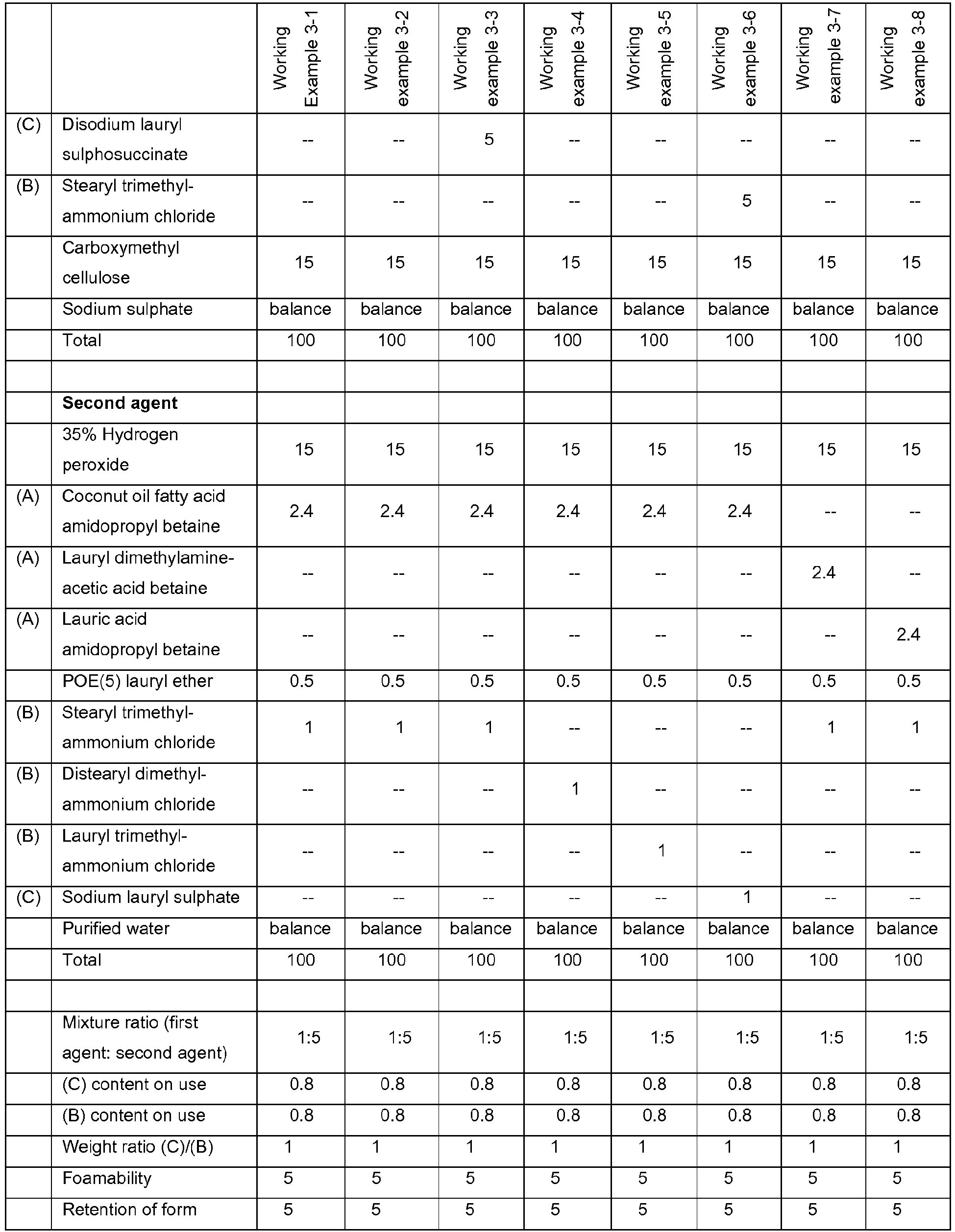

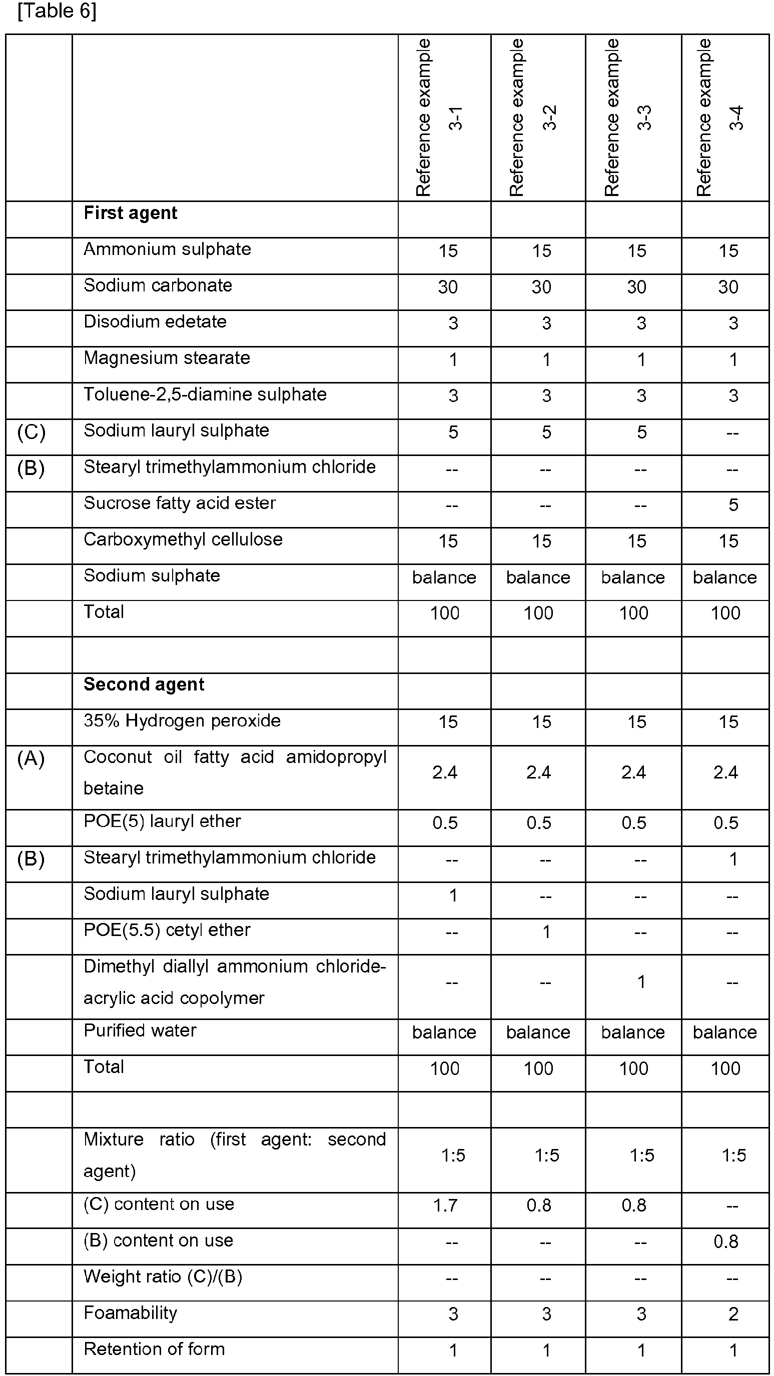

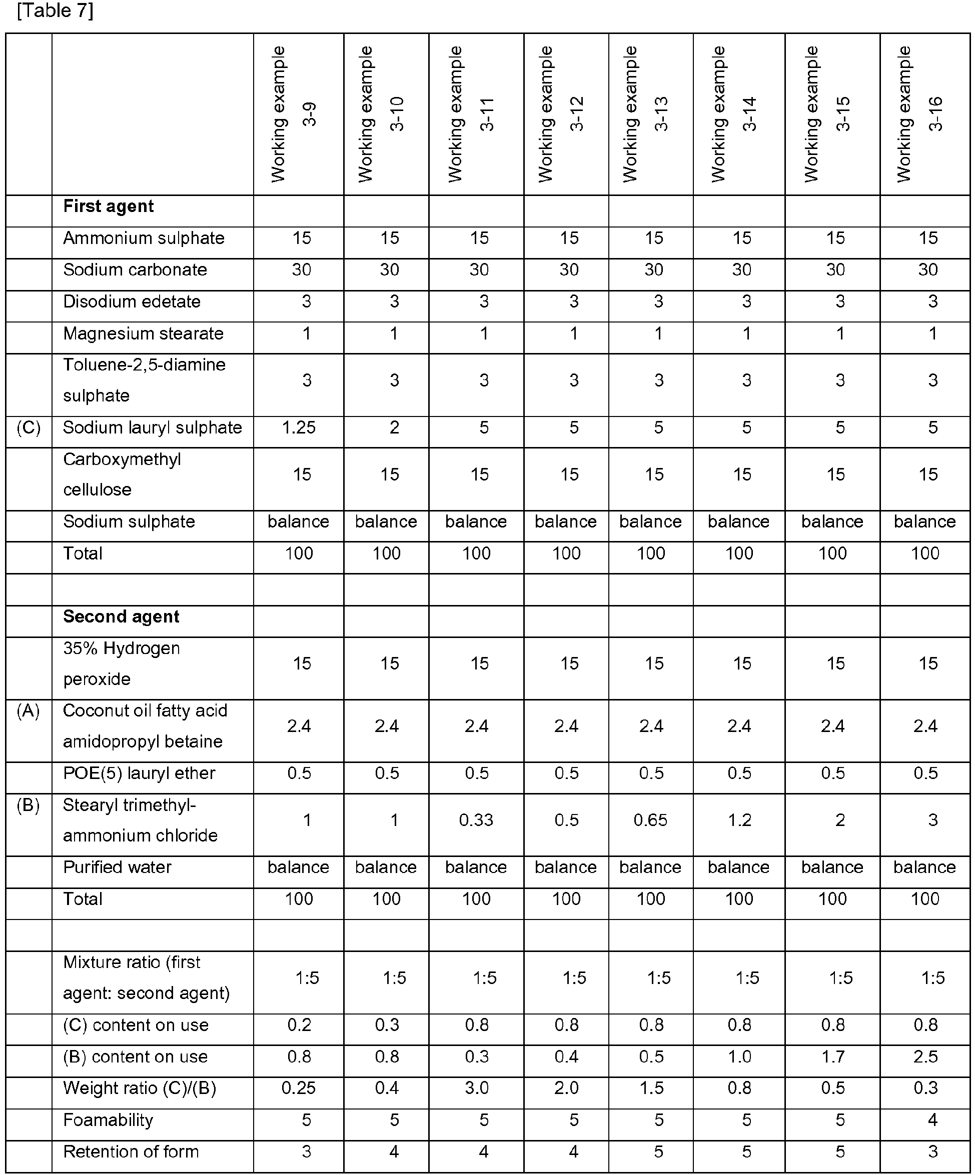

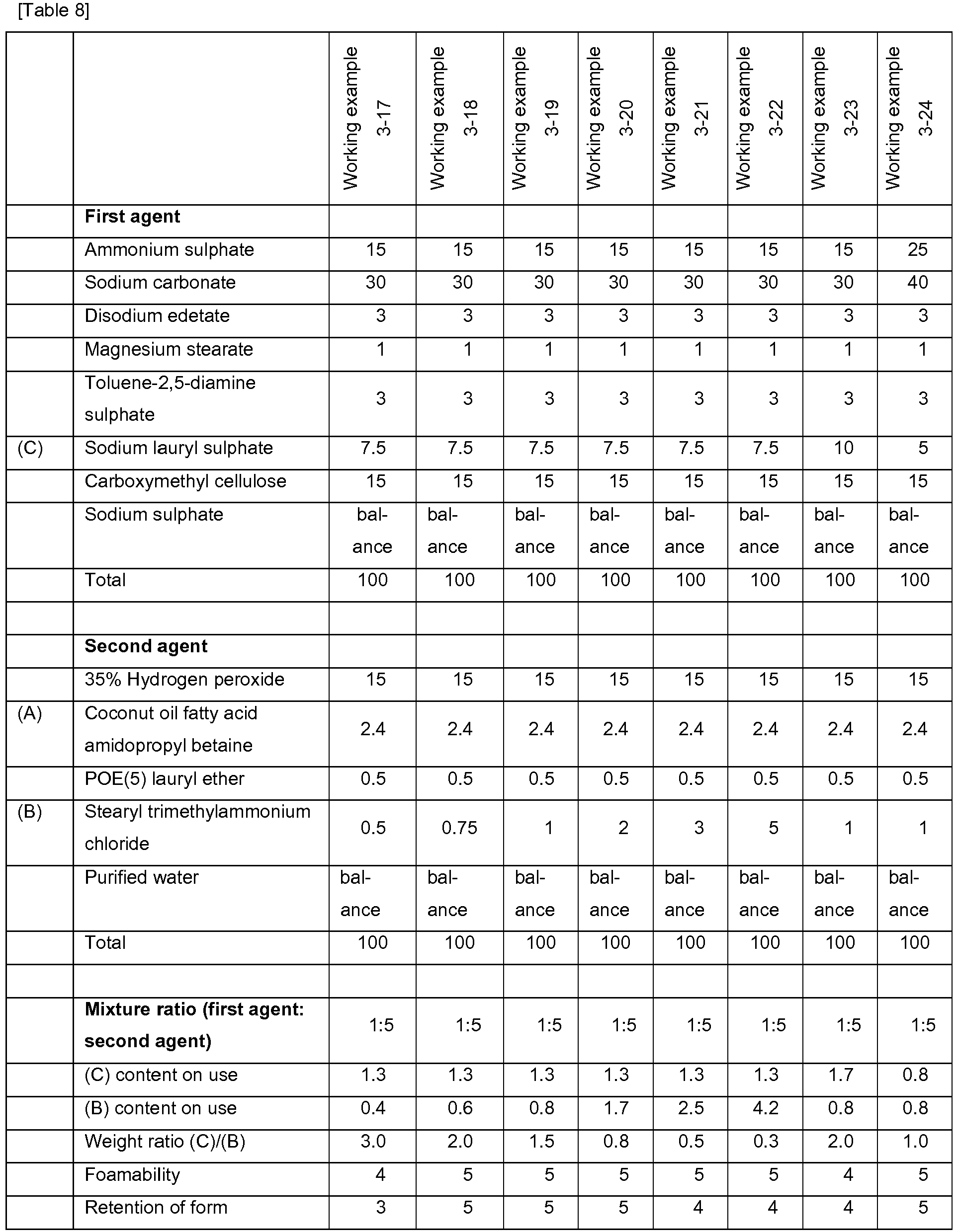

The evaluations of both the foamability and the retention of form, that is, foam hold, were 4 or higher in each of the working examples shown in Table 5. The evaluation of the retention of form was 1 in Reference Examples 3-1 to 3-4, as shown in Table 6.

In Reference Examples 3-1 to 3-3, the second agent did not contain a cationic surfactant although the first agent did contain an anionic surfactant. In Reference Example 3-1, the cationic surfactant contained in the second agent in Working Example 3-1 was changed to an anionic surfactant. In Reference Example 3-2, the cationic surfactant contained in the second agent in Working Example 3-1 was changed to POE(5.5) cetyl ether, which is a nonionic surfactant. In Reference Example 3-3, the cationic surfactant contained in the second agent in Working Example 3-1 was changed to dimethyl diallyl ammonium chloride-acrylic acid copolymer, which is a cationic polymer. In Reference Example 3-4, the anionic surfactant contained in the first agent in Working Example 3-1 was changed to sucrose fatty acid ester, which is a nonionic surfactant.

The results of these examples demonstrate the superiority of a constitution that keeps an anionic surfactant and cationic surfactant separate until they are brought into contact at the time of use. As shown in Tables 7 and 8, setting the weight ratio of anionic surfactant to cationic surfactant in the 0.25 to 3 range makes it easier to obtain better results than in the reference examples.

### Test 4

Hair dyes of Working Examples 4-1 to 4-12 and Reference Examples 4-1 and 4-2 comprising a powdered first agent and a liquid second agent containing the components shown in Tables 9 and 10 were prepared. The numerical values representing the amounts of each component of the hair dyes in Tables 9 and 10 are expressed in units of percentage by weight.

First, the first agent and second agent of the hair dye of each example were placed in a sealable container the same as container 20 illustrated in FIG 1(a) to FIG 2, and a foamy hair dye was obtained by sealing the container and shaking about 20 to 30 times to shake the mixture of the first agent and second agent in the container. The foamy hair dye obtained was applied by gloved hand to strands of black human hair and allowed to stand for 30 minutes at room temperature (25°C). The hair dye adhering to the strand of hair was then rinsed off with water, and the strand of hair was washed twice with shampoo and rinsed once. After then drying the strand of hair with hot air, a dyed strand of hair was obtained by allowing it to stand for one day.

The homogeneity of the foam, elasticity of the foam, and miscibility of the foamy hair dyes obtained by foaming the hair dyes of Working Examples 4-1 to 4-12 and Reference Examples 4-1 and 4-2 were evaluated by the following methods. The feel of the strand of hair when rinsing out the hair dye and the evenness of dyeing of the strand of hair obtained were also evaluated by the following methods. The results are shown in Tables 9 and 10.

### <Homogeneity of foam>

The foamy hair dye obtained was allowed to stand for 10 minutes inside the sealable container. The quality of the foam located in the upper part and lower part of the sealable container was then examined by a panel of experts by touching it, and thereby evaluated. In the 'Homogeneity of foam' column in Tables 9 and 10, '5' shows 'absolutely no difference in foam quality,' '4' shows 'virtually no difference in foam quality,' '3' shows 'little difference in foam quality,' '2' shows 'difference in foam quality,' and '1' shows 'difference in foam quality, with the difference being significant'.

### <Elasticity of foam>

The elasticity of the foamy hair dyes obtained was evaluated by examination by a panel of experts by touching the foam. In the 'Elasticity of foam' column in Tables 9 and 10, '5' shows 'foam did not break down at all when touched (foam pushed back when pushed),' '4' shows 'foam virtually did not break down when touched,' '3' shows 'foam broke down a little when touched,' '2' shows 'foam broke down when touched,' and '1' shows 'foam broke down significantly when touched (foam did not push back at all when pushed)'.

### <Miscibility>

The miscibility of the powdered first agent and liquid second agent was evaluated visually by a panel of experts by examining the presence of unmixed lumps in the foamy hair dyes obtained. In the 'Miscibility of foam' column in Tables 9 and 10, '5' shows 'absolutely no unmixed lumps (thoroughly mixed),' '4' shows 'virtually no unmixed lumps,' '3' shows 'few unmixed lumps,' '2' shows 'evident unmixed lumps,' and '1' shows 'very evident unmixed lumps (unmixed)'.

### <Feel (stickiness)>

The feel of the strand of hair (hair) when rinsing out the hair dye was evaluated by a panel of experts by touching it. In the 'Feel (stickiness)' column in Tables 9 and 10, '5' shows 'absolutely no stickiness,' '4' shows 'virtually no stickiness,' '3' shows 'little stickiness,' '2' shows 'feels sticky,' and '1' shows 'feels very sticky'.

### <Evenness of dyeing>

The evenness of dyeing of the dyed strand of hair was examined visually by a panel of experts under standard lighting, and thereby evaluated. In the 'Evenness of dyeing' column in Tables 9 and 10, '5' shows 'absolutely no dye streaks,' '4' shows 'virtually no dye streaks,' '3' shows 'few dye streaks,' '2' shows 'evident dye streaks,' and '1' shows 'very evident dye streaks'.

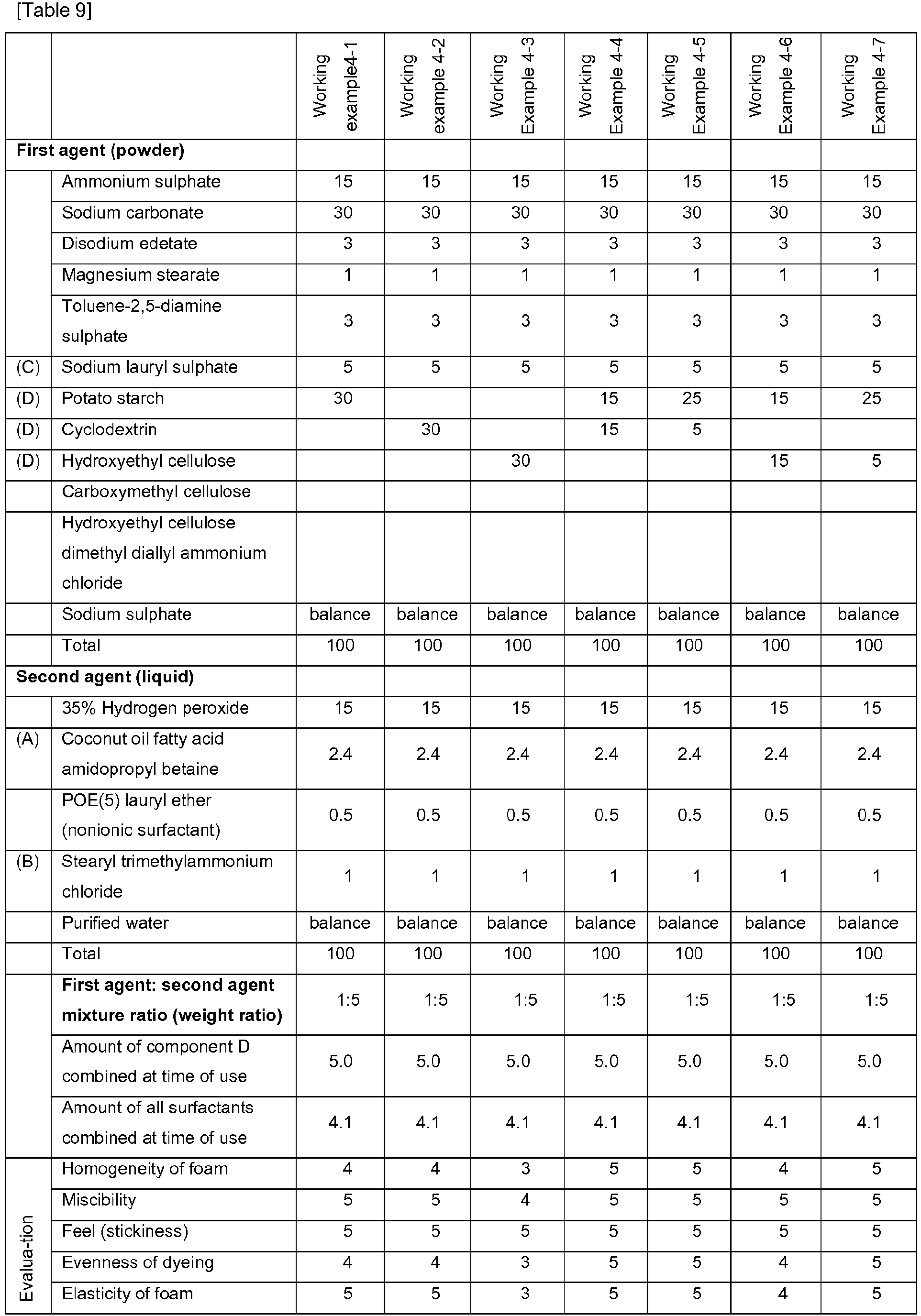

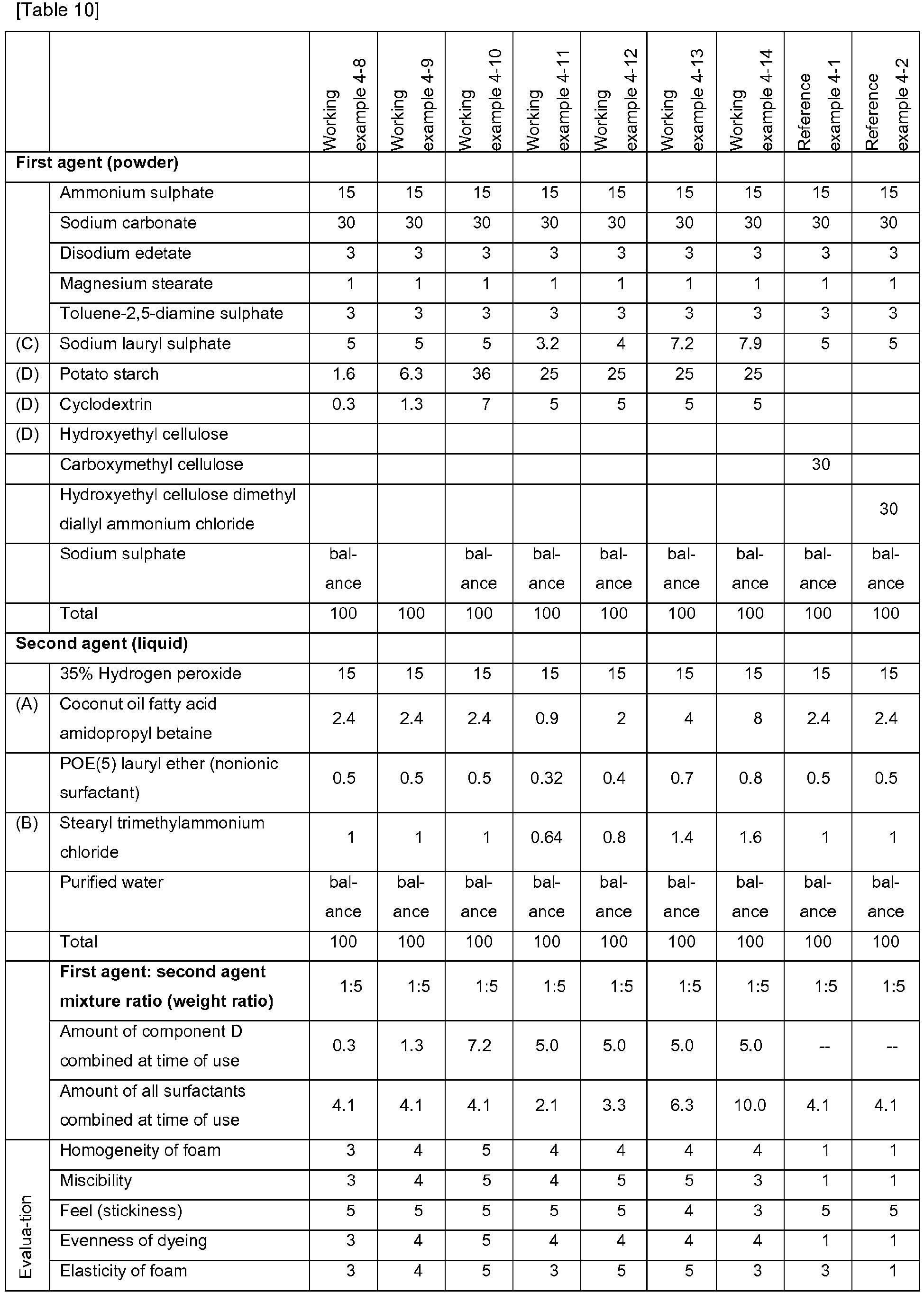

As shown in Tables 9 and 10, the working examples that contained nonionic polymers obtained good evaluations in all categories. In particular, the evaluations of 'homogeneity of foam,' 'elasticity of foam,' 'miscibility,' and 'evenness of dyeing' were understood to be higher in Working Examples 4-1 and 4-2 that contained starch and cyclodextrin, which are a polysaccharide and an oligosaccharide having an α-glucose compound as a structural unit, as nonionic polymers, in comparison to Working Example 4-3, which contained another nonionic polymer. The evaluations of 'homogeneity of foam' and 'evenness of dyeing' were also understood to be higher in Working Examples 4-4 and 4-5 that contained two types of nonionic polymer in comparison to Working Examples 4-1 and 4-2 that contained the same amount of one type of nonionic polymer.

On the other hand, the evaluations of 'homogeneity of foam,' 'miscibility,' and 'evenness of dyeing' were understood to be low in comparison to the working examples in Reference Example 4-1 that contained an anionic polymer instead of a nonionic polymer. The evaluations of 'homogeneity of foam,' 'elasticity of foam,' 'miscibility,' and 'evenness of dyeing' were understood to be low in comparison to the working examples in Reference Example 4-2 that contained a cationic polymer instead of a nonionic polymer.

### Test 5

Working Examples 5-1 to 5-5 and Reference Examples 5-1 to 5-3 The oxidation hair dyeing agents of Working Examples 5-1 to 5-5 and Reference Examples 5-1 to 5-3 were prepared. These oxidation hair dyeing agents were two-component dyes comprising a powdered first agent and a liquid second agent. The compositions of the first agent and second agent were as shown in Table 11. The numerical values representing the content of each component of the oxidation hair dyeing agents in Table 11 and later in Tables 12 and 13 are expressed in units of percentage by weight.

Next, the oxidation hair dyeing agent of each example was foamed using a sealable container the same as container 20 illustrated in FIG 1(a) to FIG 2. Furthermore, the volume of the sealable container used was 770 mL, the height was 17 cm, and the inner diameter was 7 to 8 cm. One hundred fifty grams of a mixture of the first agent and second agent was placed in this container, and the container was shaken up and down 20 times. A foamy oxidation hair dyeing agent was obtained by shaking and mixing air into the mixture of the first agent and second agent in this way.

Working Examples 5-6 to 5-17 Foamy oxidation hair dyeing agents were obtained in the same manner as in Working Example 5-1 from oxidation hair dyeing agents of compositions different from the oxidation hair dyeing agent of Working Example 5-1 in terms of the amount of at least one of the ammonium-type cationic surfactant comprising an alkyl group having from 16 to 22 carbon atoms of component b-1 and the ammonium-type cationic surfactant comprising an alkyl group having from 10 to fewer than 16 carbon atoms of component b-2.

### <Evaluation of usability>

The oxidation hair dyeing agent of each example was placed on the hands of a panel of experts. The oxidation hair dyeing agent was then partially lifted, and the state of stringiness and state of foam breakaway were examined visually. The results were evaluated by the following criteria.
Evaluation 5: No stringiness, foam breakaway highly exceptional
Evaluation 4: Virtually no stringiness, foam breakaway excellent
Evaluation 3: Foam breakaway good despite a little stringiness
Evaluation 2: Stringy, foam breakaway fairly poor
Evaluation 1: Very stringy, foam breakaway poor
The numerical values of the evaluation results are shown in the 'Usability' column in Tables 11 to 13.

### <Evaluation of ease of application>

The oxidation hair dyeing agent of each example was applied to hair by a panel of experts and the condition at that time was evaluated by the following criteria.
Evaluation 5: Spread on and adherence to hair highly exceptional
Evaluation 4: Spread on and adherence to hair excellent
Evaluation 3: Spread on and adherence to hair good
Evaluation 2: Spread on hair fairly poor or adherence to hair fairly poor
Evaluation 1: Spread on hair poor or adherence to hair poor
The numerical values of the evaluation results are shown in the 'Ease of application' column in Tables 11 to 13.

### <Evaluation of feel of hair>

The oxidation hair dyeing agent of each example was applied to a strand of hair and allowed to stand for the prescribed time. A hair-dyeing step was carried out by rinsing out the strand of hair with warm water. The feel of the strand of hair when rinsed out with warm water (at the time of rinsing) and the feel of the strand of hair after the strand of hair had been dried by hair dryer (after finishing) were evaluated organoleptically by a panel of experts according to the following criteria.
Evaluation 5: No stickiness at the time of rinsing or after finishing
Evaluation 4: Virtually no stickiness at the time of rinsing or after finishing
Evaluation 3: Slight stickiness at the time of rinsing or after finishing
Evaluation 2: Sticky at the time of rinsing or after finishing
Evaluation 1: Sticky at the time of rinsing and after finishing
The numerical values of the evaluation results are shown in the 'Feel of hair' column in Tables 11 to 13.

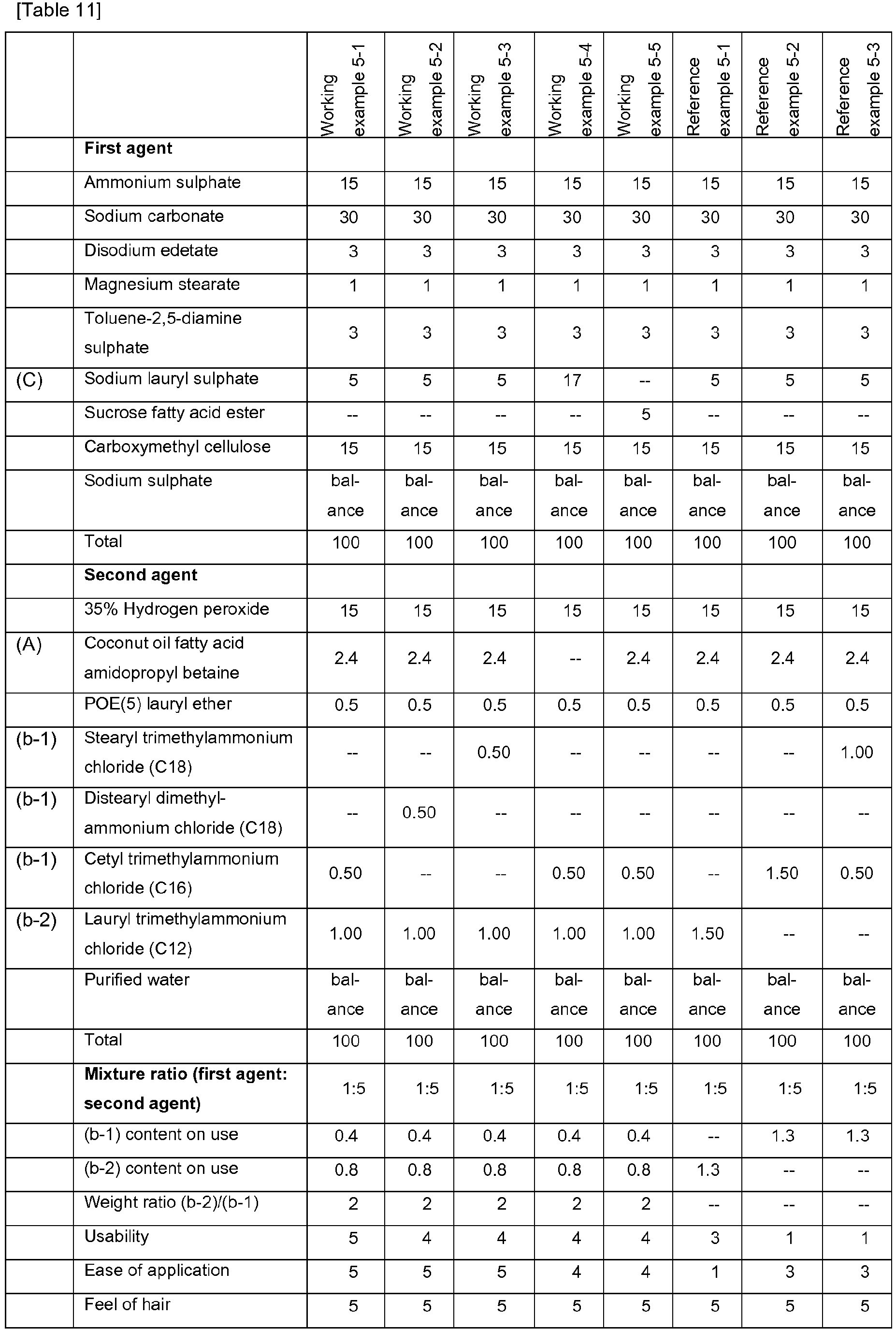

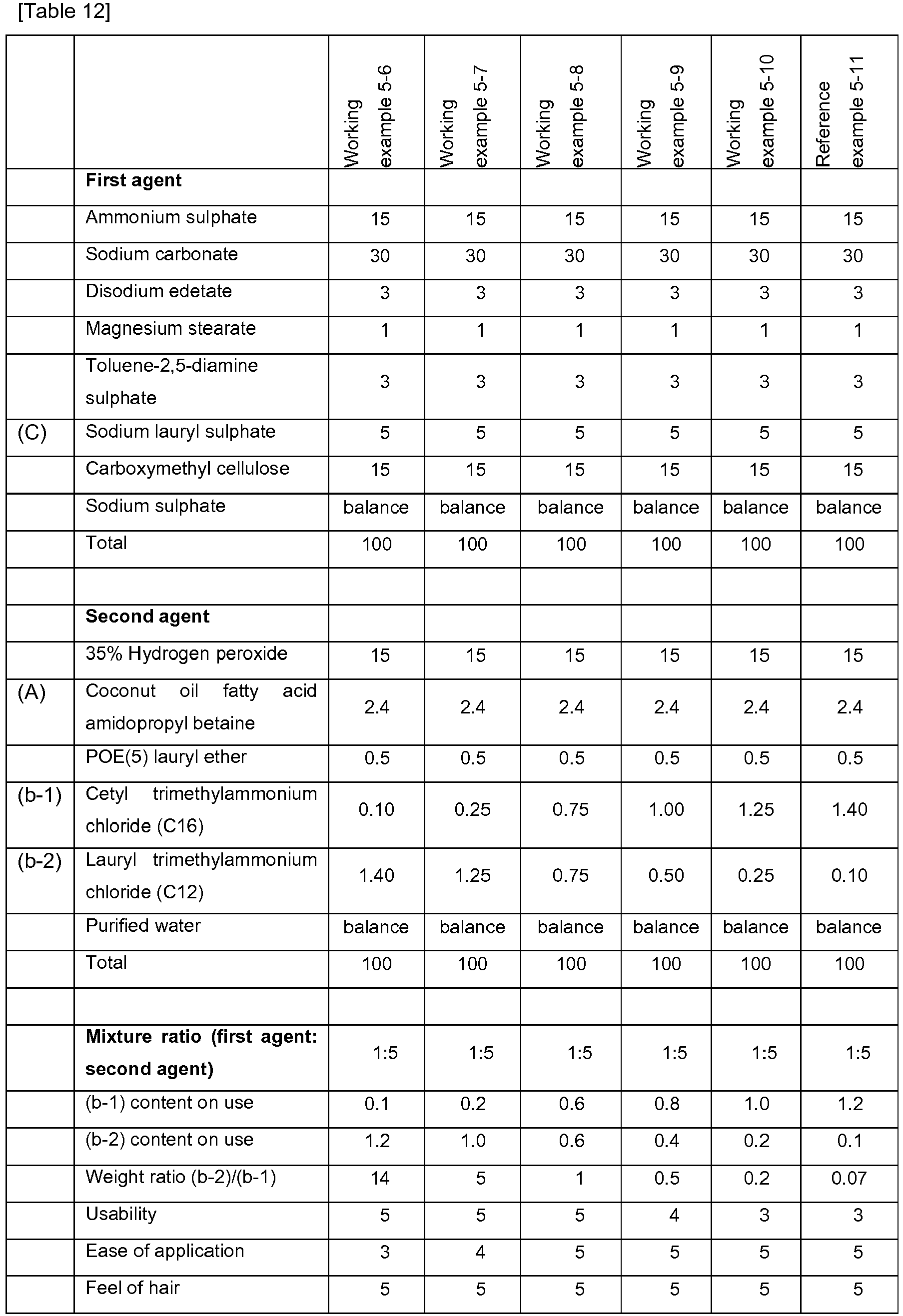

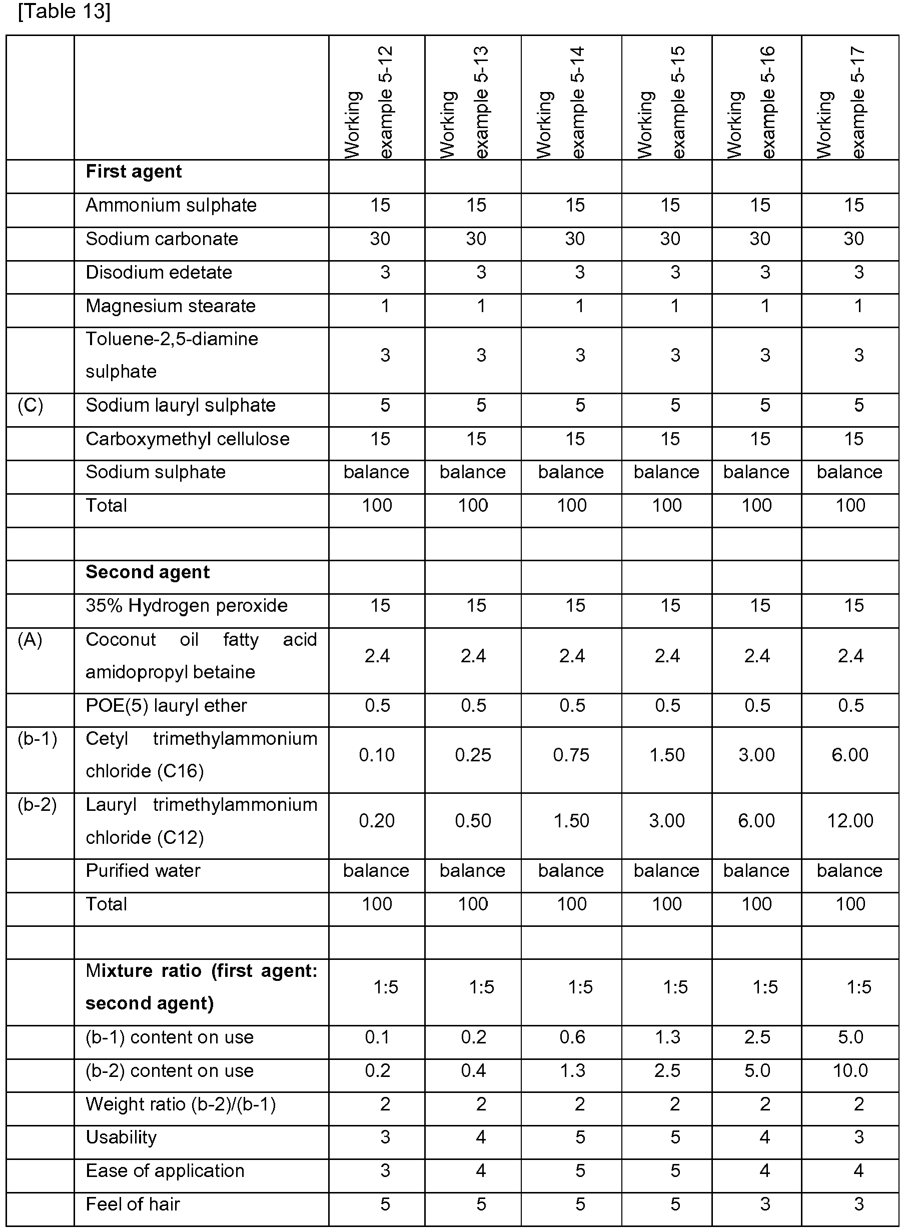

As shown in Table 11, the usability, ease of application, and feel of the hair received an evaluation of 4 or higher in Working Examples 5-1 to 5-5. Either the usability or ease of application received an evaluation of 1 in Reference Examples 5-1 to 5-3.

In Reference Example 5-1, no (b-1) ammonium-type cationic surfactant comprising an alkyl group having from 16 to 22 carbon atoms was contained. In Reference Example 5-2, no (b-2) ammonium-type cationic surfactant comprising an alkyl group having from 10 to fewer than 16 carbon atoms was contained. In Reference Example 5-3, two types having different numbers of carbon atoms in the alkyl chain were contained among (b-1) ammonium-type cationic surfactants comprising an alkyl group having from 16 to 22 carbon atoms, but the results on ease of application were inferior to those of the working examples.

As shown in Table 12, setting the weight ratio of (b-2) ammonium-type cationic surfactant comprising an alkyl group having from 10 to fewer than 16 carbon atoms to (b-1) ammonium-type cationic surfactant comprising an alkyl group having from 16 to 22 carbon atoms within the prescribed range is understood to make it even easier to improve both the usability and ease of application. As shown in Table 13, adjusting the amount of (b-1) ammonium-type cationic surfactant comprising an alkyl group having from 16 to 22 carbon atoms and (b-2) ammonium-type cationic surfactant comprising an alkyl group having from 10 to fewer than 16 carbon atoms is understood to make it even easier to improve the usability, ease of application, and feel of the hair.

Furthermore, the foamability was good in the foaming step of the first agents and second agents of Working Examples 5-1 to 5-4 and 5-6 to 5-17. Defoaming (foam breakdown) also did not begin immediately after foaming, and the form of a foam was retained for the prescribed length of time. The form of a foam was therefore adequately maintained in the hair dyeing step.

### Test 6-1

Hair bleaching agents of Working Examples 6-1 to 6-24 and Reference Examples 6-1 to 6-4 comprising a powdered first agent and emulsion second agent containing the components shown in Tables 14 and 15 were prepared. The numerical values representing the content of each component of the agents for removing colour from hair in Tables 14 and 15 are expressed in terms of percentage by weight. The agents for removing colour from hair were prepared by mixing the first agent and second agent in a weight ratio of 1:4 and stirring using a stirring rod. The rate of onset of a thickening effect and the viscosity stability of the agents for removing colour from hair obtained were evaluated. The results are shown in Tables 14 and 15.

The 'Starch weight ratio' in Tables 14 and 15 shows the weight ratio of the starch content to the thickener content at the time of use. The 'Anionic surfactant weight ratio' in Tables 14 and 15 shows the weight ratio of the anionic surfactant (component C) content to the cationic surfactant content (component B) at the time of use.

### <Measurement and evaluation of the speed of appearance of a thickening effect>

The viscosity of the agents for removing colour from hair obtained by mixing was measured every minute from the time of mixing the first agent and second agent, by a model B viscometer. The measurement conditions were: rotor No. 4, 12 rpm, 25°C, one minute.

In the 'Speed of appearance of a thickening effect' in Tables 14 and 15, '5' shows a viscosity elevation rate up to three minutes after mixing the first agent and second agent of 1000 mPa·s/min or higher, '4' shows from 850 mPa·s/min to less than 1000 mPa·s/min, '3' shows from 650 mPa·s/min to less than 850 mPa·s/min, '2' shows from 500 mPa·s/min to less than 650 mPa·s/min, and '1' shows less than 500 mPa·s/min.

### <Viscosity stability>

The viscosity ratio (= viscosity after 5 minutes/viscosity after 25 minutes) was calculated from the viscosity 5 minutes after mixing the first agent and second agent and the viscosity 25 minutes after mixing the first agent and second agent.

This viscosity ratio was evaluated on a scale of 5 comprising from 0.80 to less than 1.20 (highly exceptional: 5), from 0.60 to less than 0.80 or from 1.2 to less than 2.0 (excellent: 4), from 0.40 to less than 0.60 or from 2.0 to less than 4.0 (good: 3), from 0.20 to less than 0.40 or from 4.0 to less than 6.0 (somewhat poor: 2), and less than 0.20 or 6.0 or higher (poor: 1).

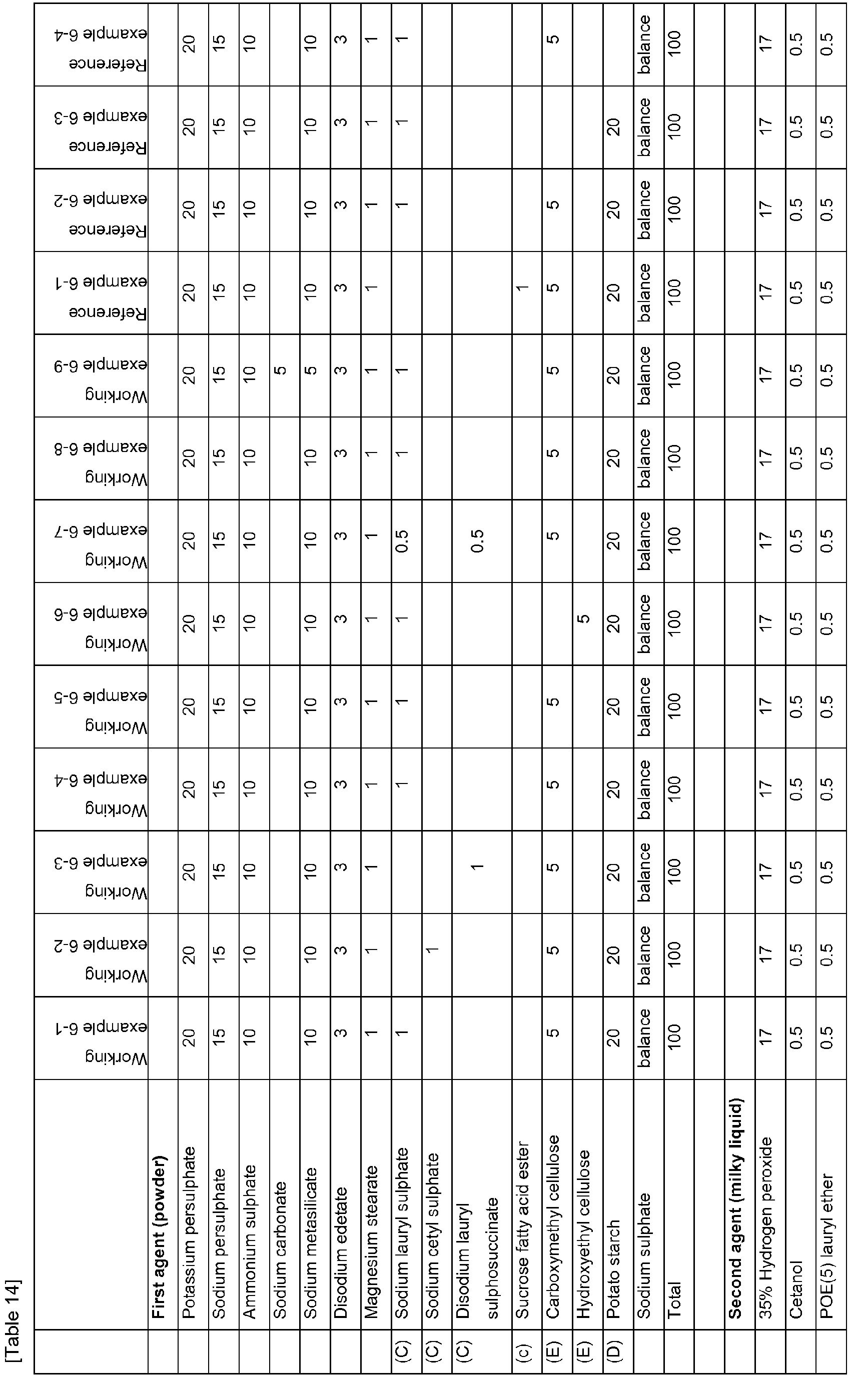

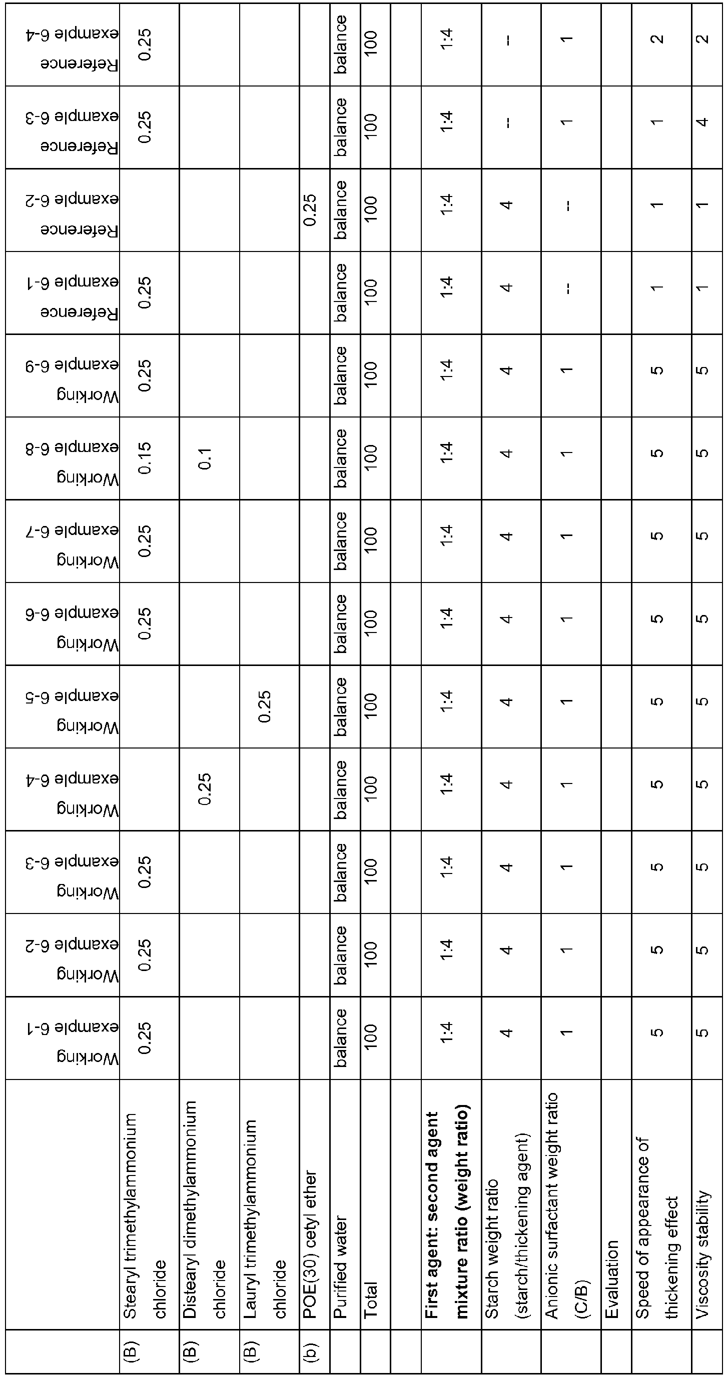

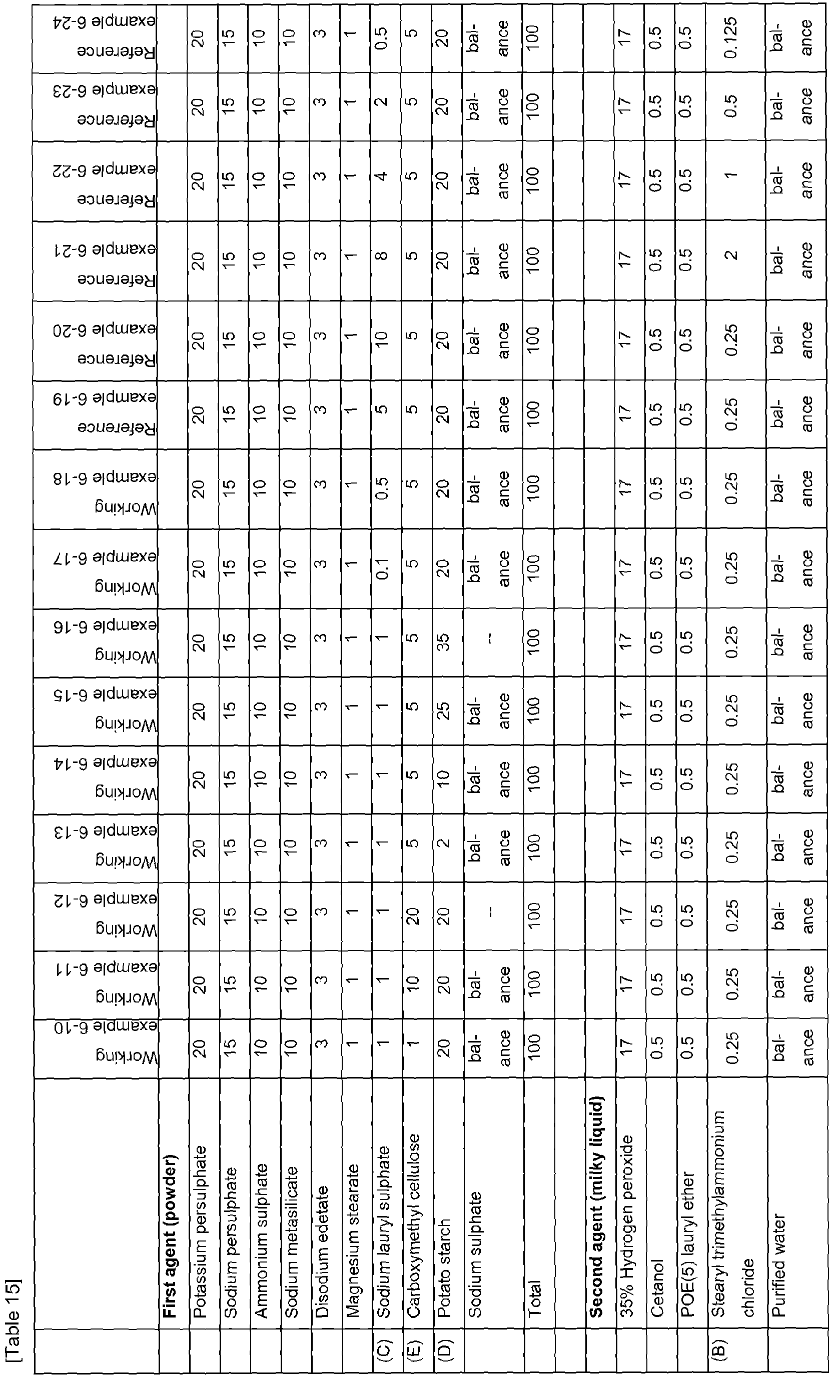

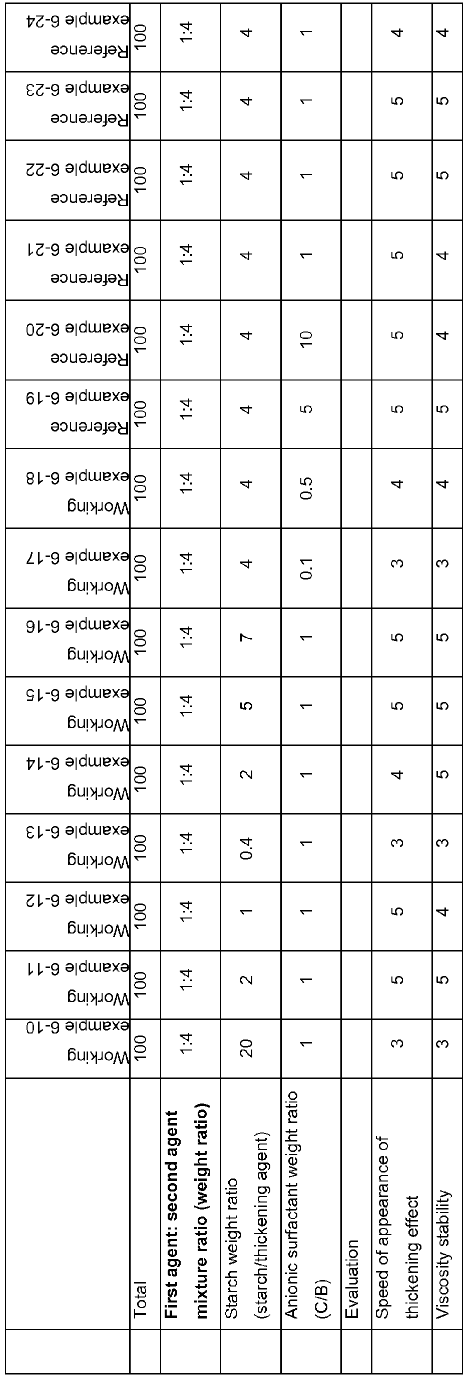

As shown in Tables 14 and 15, the agents for removing colour from hair of each of the working examples constituted by combining an anionic surfactant, thickening agent, and starch in the first agent and a cationic surfactant in the second agent obtained good results in both categories 'speed of appearance of thickening effect' and 'viscosity stability'.

As shown in Table 14, Reference Example 6-1 that contained a nonionic surfactant instead of an anionic surfactant in the first agent and Reference Example 6-2 that contained a nonionic surfactant instead of a cationic surfactant in the second agent were understood to receive low evaluations in comparison to the working examples in the category 'speed of appearance of thickening effect'. The category 'viscosity stability' received lower evaluations in comparison to the working examples in Reference Examples 6-1 and 6-2 because the viscosity continued to rise gradually over time.

Reference Example 6-3 that did not contain a thickening agent in the first agent received a low evaluation in comparison to the working examples in the category 'speed of appearance of thickening effect' because the final viscosity of the mixture was low. Reference Example 6-4 that did not contain starch in the first agent received a low evaluation in comparison to the working examples in the category 'speed of appearance of thickening effect' because the final viscosity of the mixture was low. Reference Example 6-4 also received a low evaluation in comparison to the working examples in the category 'viscosity stability' because the viscosity continued to decline gradually over time.

Test 6-2 Hair bleaching agents of Working Example 6-25 and Reference Examples 6-5 to 6-7 comprising a powdered first agent and a milky-liquid-form second agent containing the components shown in Table 16 were prepared. The numerical values representing the content of each component of the agents for removing colour from hair in Table 16 are expressed in units of percentage by weight. The first agent and second agent were placed in a weight ratio of 1:4 in a sealable container the same as container 20 illustrated in FIG 1(a) to FIG 2, and a foamy hair bleaching agent was obtained by sealing the container and shaking it about 20 to 30 times. The rate of onset of the thickening effect and the viscosity stability of the agents for removing colour from hair obtained were evaluated by the following methods.

### <Measurement and evaluation of speed of appearance of thickening effect>

The degree of dripping when lifted by hand three minutes after mixing the first agent and second agent was evaluated by a panel of five experts as regards the viscosity of the foamy agents for removing colour from hair obtained. In the 'Speed of appearance of thickening effect' column in Table 16, '5' shows highly exceptional appearance of thickening with absolutely no dripping three minutes after mixing the first agent and second agent, '4' shows an excellent effect with virtually no dripping, '3' shows a good effect with little dripping, '2' shows a somewhat poor effect with some dripping, and '1' shows a poor effect with extensive dripping.

### <Viscosity stability>

The difference in the extent of dripping when the foam was lifted by hand five minutes after mixing the first agent and second agent and the extent of dripping when the foam was lifted by hand 25 minutes after mixing the first agent and second agent was evaluated by a panel of five experts. In the 'Viscosity stability' column in Table 16, '5' shows a highly exceptional effect with no difference in dripping five and 25 minutes after mixing the first agent and second agent, '4' shows an excellent effect with virtually no difference in dripping, '3' shows a good effect with little difference in dripping, '2' shows a somewhat poor effect with a difference in dripping, and '1' shows a poor effect with a clear difference in dripping.

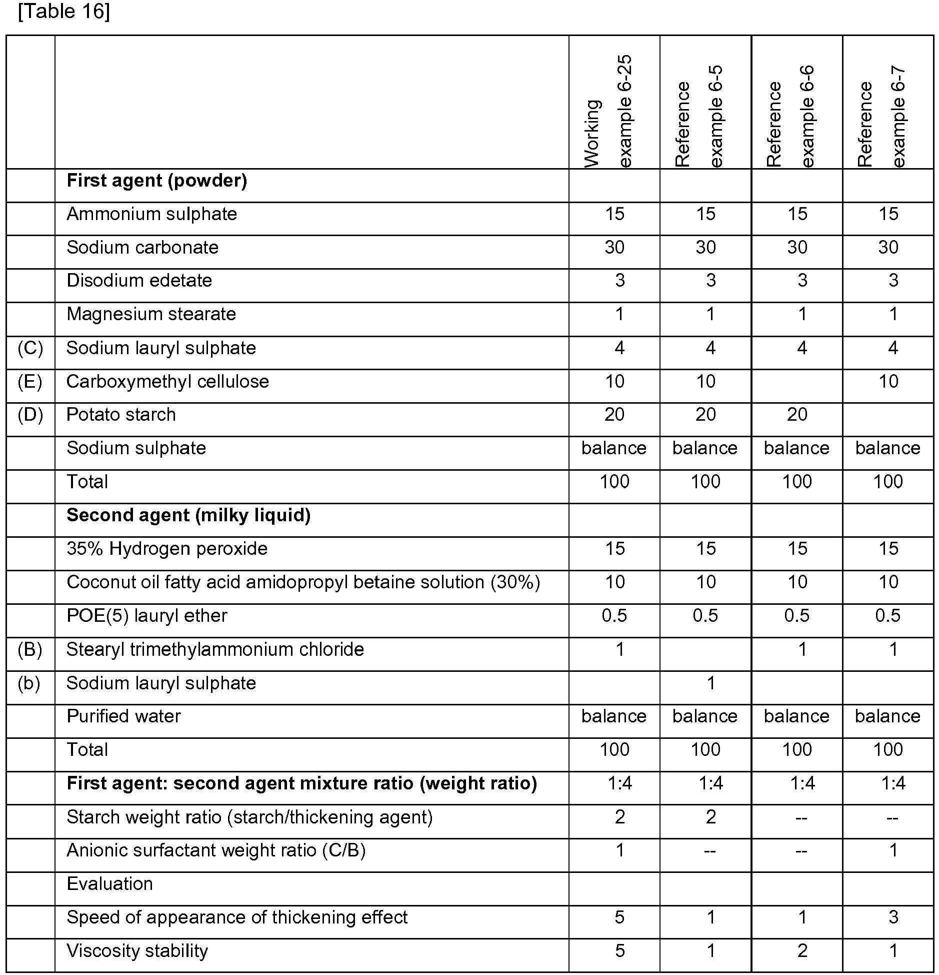

As shown in Table 16, the hair bleaching agent of Working Example 6-25 constituted by combining an anionic surfactant, thickening agent, and starch in the first agent and a cationic surfactant in the second agent obtained good results in the categories 'speed of appearance of thickening effect' and 'viscosity stability'. In sum, the 'speed of appearance of thickening effect' and 'viscosity stability' were also understood to be excellent when the form at the time of use was a foam as well.

As shown in Table 16, Reference Example 6-5 that contained an anionic surfactant instead of a cationic surfactant in the second agent was understood to receive a low evaluation in comparison to the working examples in the category 'speed of appearance of thickening effect'. Reference Example 6-5 received a low evaluation in comparison to the working examples in the category 'viscosity stability' because the foam became hard over time.

Reference Example 6-6 that did not contain a thickening agent in the first agent received a low evaluation in comparison to the working examples in the category 'speed of appearance of thickening effect' because the final viscosity of the mixture was low.

Reference Example 6-7 that did not contain starch in the first agent received a low evaluation in comparison to the working examples in the category 'viscosity stability' because the viscosity continued to decline gradually over time and dripping of the solution increased.

### Test 7

Hair dyes of Working Examples 7-1 to 7-13 and Reference Examples 7-1 to 7-5 comprising a powdered first agent containing the components shown in Table 17 and a liquid second agent containing the components shown in Tables 18 to 20 were prepared. The numerical values representing the content of each component of the hair dyes in Tables 17 to 20 are expressed in units of percentage by weight. In this test, Merquat 550 (made by Ondeo Nalco Co) was used as a dimethyl diallyl ammonium chloride-acrylamide copolymer, Merquat 295 (made by Ondeo Nalco Co) was used as a dimethyl diallyl ammonium chloride-acrylic acid copolymer, and Merquat 100 (made by Ondeo Nalco Co) was used as polydimethylmethylenepiperidinium chloride. The numerical values representing the contents of each component in Tables 17 to 20 represent the pure fraction of that component.

**[Table 17]**

| **First agent (powder)** | |
|---|---|
| Ammonium sulphate | 15 |
| Sodium carbonate | 30 |
| Disodium edetate | 3 |
| Magnesium stearate | 1 |
| Toluene-2,5-diamine sulphate | 3 |
| Sodium lauryl sulphate | 5 |
| Carboxymethyl cellulose | 15 |
| Sodium sulphate | balance |
| Total | 100 |

The first agent and second agent of the hair dyes of each example were stored for 30 days at 45°C then used in this test. The first agent and second agent were placed in a sealable container the same as container 20 illustrated in FIG 1(a) to FIG 2, and a foamy hair dye was obtained by sealing the container and shaking about 20 to 30 times to shake the mixture of the first agent and second agent inside the container. The foamy hair dye obtained was applied to a strand of black human hair by a gloved hand and allowed to stand for 30 minutes at room temperature (25°C). The hair dye adhering to the strand of hair was then rinsed off with water, and the strand of hair was shampooed twice and rinsed once. After then drying the strand of hair with hot air, the strand of hair was allowed to stand for one day, yielding a dyed strand of hair. The foamability of the hair dyes of the working examples and reference examples and the feel of the strand of hair when rinsing out the hair dye were evaluated by the following methods. The results are shown in Tables 18 to 20.

### <Foamability>

The state of the hair dye after foaming was examined visually by a panel of experts. In the 'Foamability' column in Tables 18 to 20, '5' shows 'highly exceptional foaming,' '4' shows 'excellent foaming,' '3' shows 'good foaming,' '2' shows 'somewhat inadequate foaming,' and '1' shows 'inadequate foaming or excessive foaming creating low-viscosity, light, large foam'.

### <Feel (to the touch)>

The feel of the strand of hair (hair) when rinsing out the hair dye was examined by touching by a panel of experts, and thereby evaluated. In the 'Feel (to the touch)' column in Tables 18 to 20, '5' shows 'highly exceptional to the touch,' '4' shows 'excellent to the touch,' '3' shows 'good to the touch,' '2' shows 'poor to the touch, feels squeaky,' and '1' shows 'very poor to the touch, fingers get caught easily'.

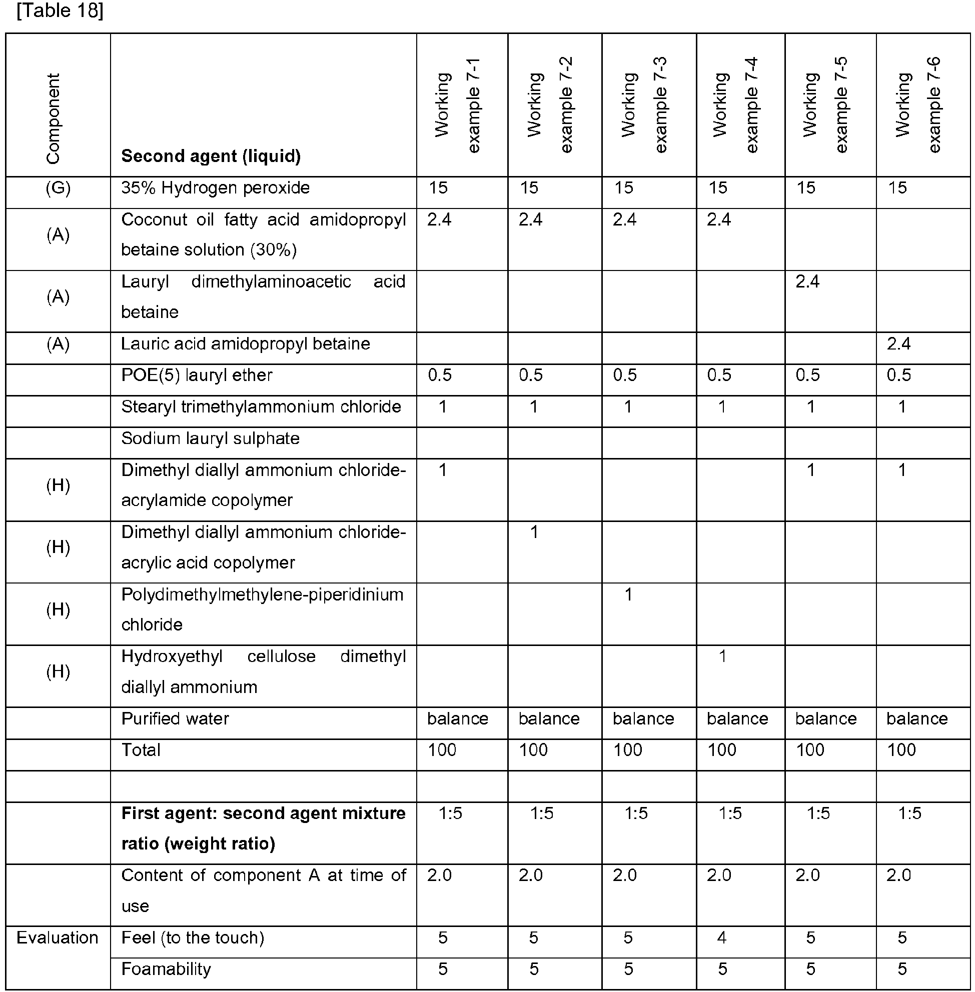

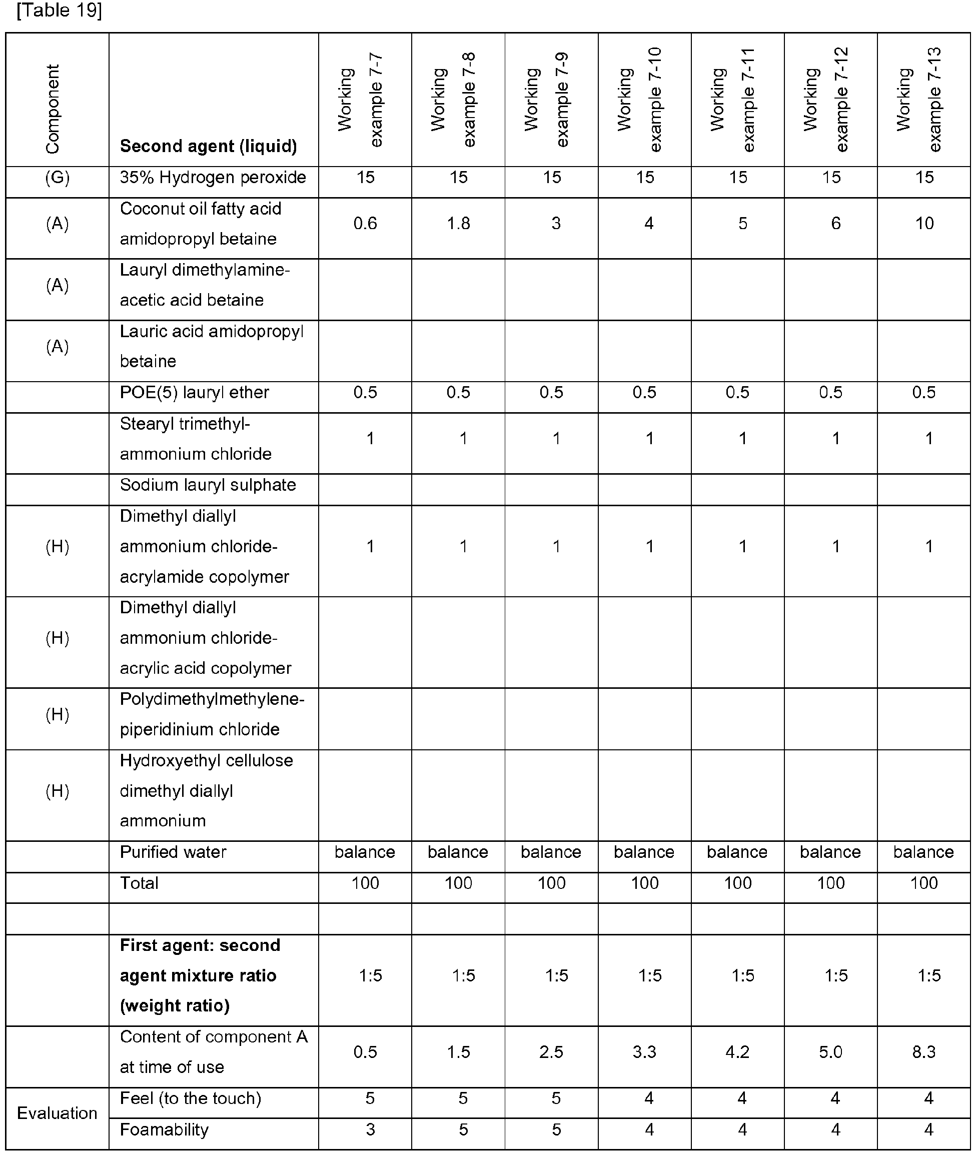

As shown in Tables 18 and 19, the working examples that contained hydrogen peroxide, an amphoteric surfactant, and a cationic polymer in the second agent received good evaluations in the categories 'feel (to the touch)' and 'foamability'. In particular, Working Examples 7-1 to 7-3 that contained products prepared in liquid form at 25°C as cationic polymers were understood to have a higher evaluation of 'feel (to the touch)' in comparison to Working Example 7-4 that contained a cationic polymer that is solid at 25°C. Working Examples 7-8 and 7-9 in which the amphoteric surfactant content of the second agent was in the 1.5 to 3.5 wt% range also received high evaluations of both 'feel (to the touch)' and 'foamability'.

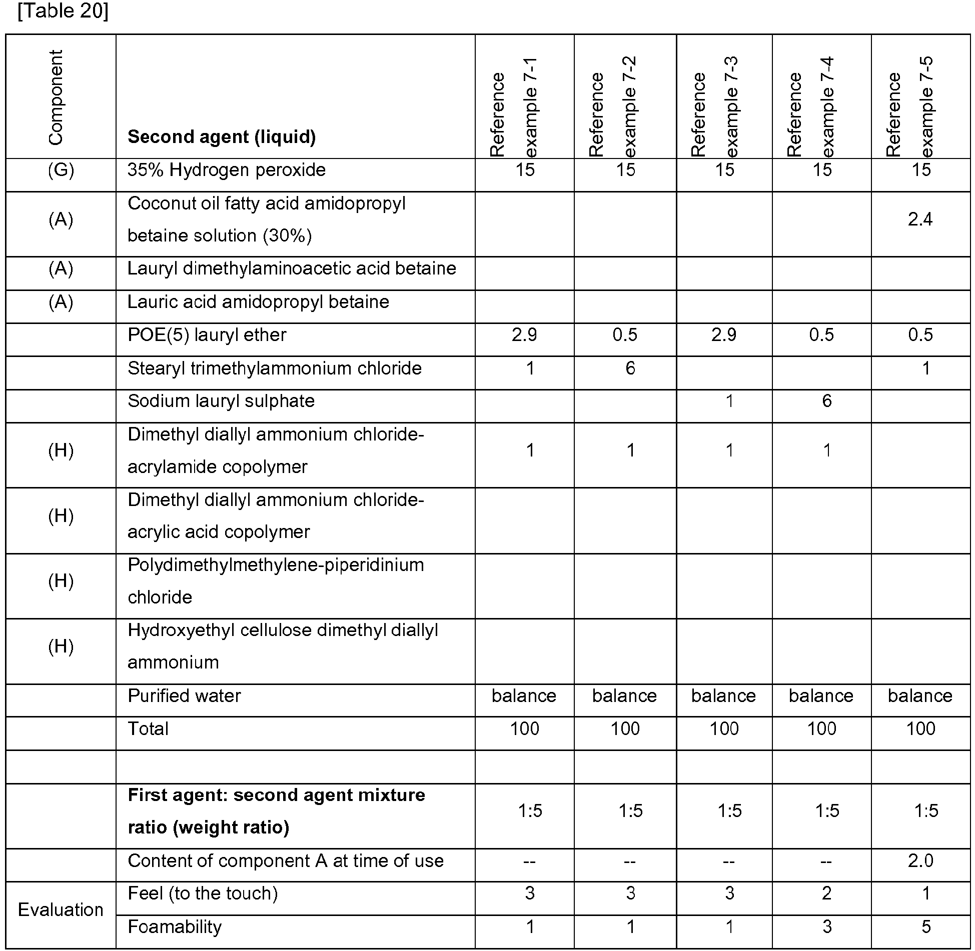

As shown in Table 20, Reference Examples 7-1 to 7-4 that did not contain amphoteric surfactant in the second agent and had increased contents of nonionic surfactant, cationic surfactant, or anionic surfactant in the second agent were understood to receive low evaluations of both 'feel (to the touch)' and 'foamability' in comparison to the working examples. Reference Example 7-5 that did not contain a cationic polymer in the second agent was also understood to receive a low evaluation of 'feel (to the touch)' even though the evaluation of 'foamability' was high.

### Test 8 (Preparation of hydrogen peroxide-containing composition)

Hydrogen peroxide-containing compositions of Reference Examples 8-1-1 to 8-1-19 and 8-2-1 to 8-2-5 were prepared by combining the components listed in Tables 21 and 22. The stability of the hydrogen peroxide and odour of the hydrogen peroxide-containing compositions prepared were evaluated by the following methods. The numerical values representing the content of each component of the hydrogen peroxide-containing compositions in Tables 21 and 22 are expressed in units of percentage by weight.

### <Stability of hydrogen peroxide>

The amount of residual hydrogen peroxide was assayed by redox titration after storing the prepared hydrogen peroxide-containing composition for one month in a 45°C thermostatic chamber, and the percentage of residual hydrogen peroxide was calculated. The evaluation criteria were as follows. The results of evaluation are shown in Tables 21 and 22.
Evaluation 5: Percentage of residual hydrogen peroxide 99% or higher
Evaluation 4: Percentage of residual hydrogen peroxide from 98% to less than 99%
Evaluation 3: Percentage of residual hydrogen peroxide from 97% to less than 98%
Evaluation 2: Percentage of residual hydrogen peroxide from 96% to less than 97%
Evaluation 1: Percentage of residual hydrogen peroxide less than 96%

### <Odou r>

The odour of the prepared hydrogen peroxide-containing compositions was evaluated. The evaluation criteria were as follows. The results of evaluation are shown in Tables 21 and 22.
Evaluation 3: Absolutely no odour of the phenoxyethanol raw material
Evaluation 2: Virtually no odour of the phenoxyethanol raw material
Evaluation 1: Evident odour of the phenoxyethanol raw material

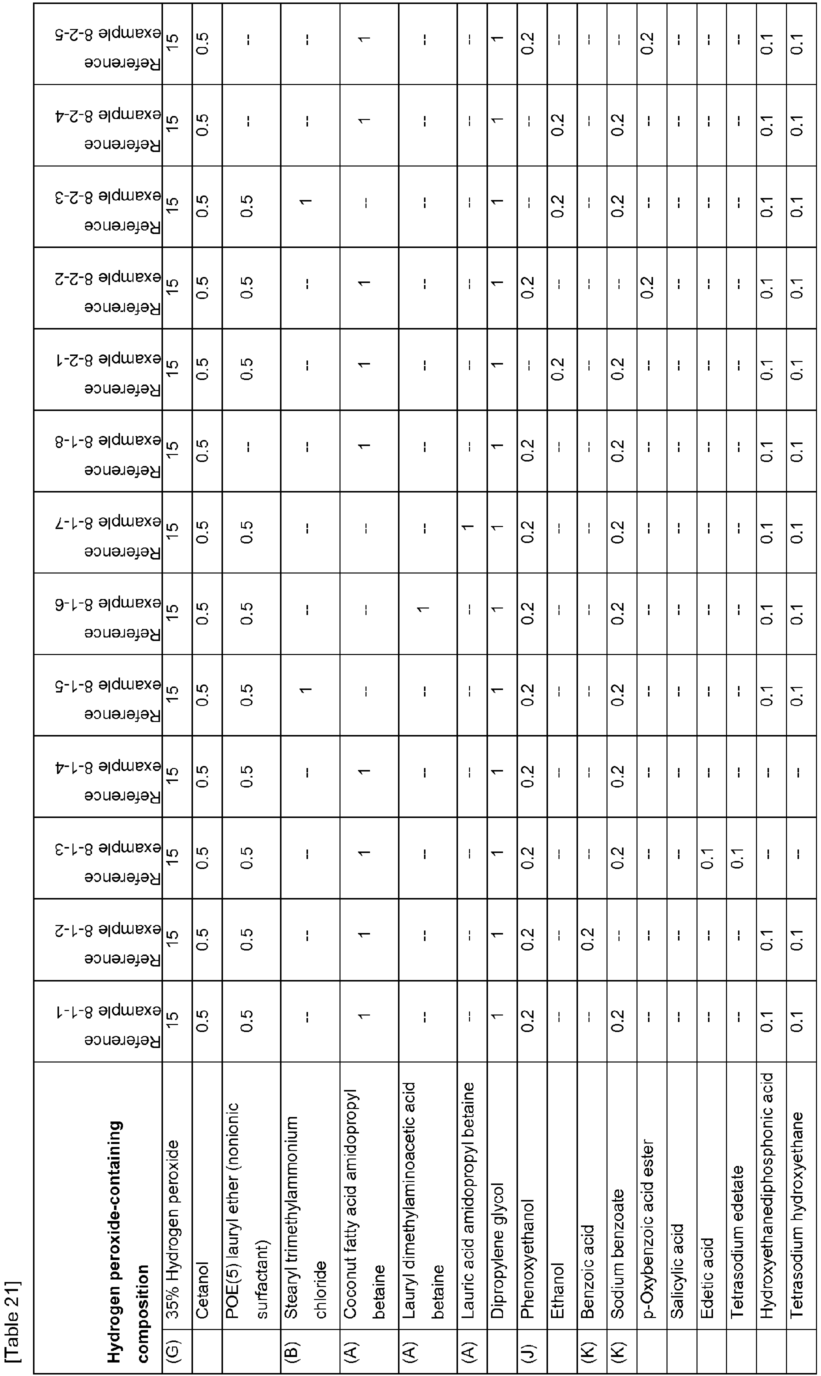

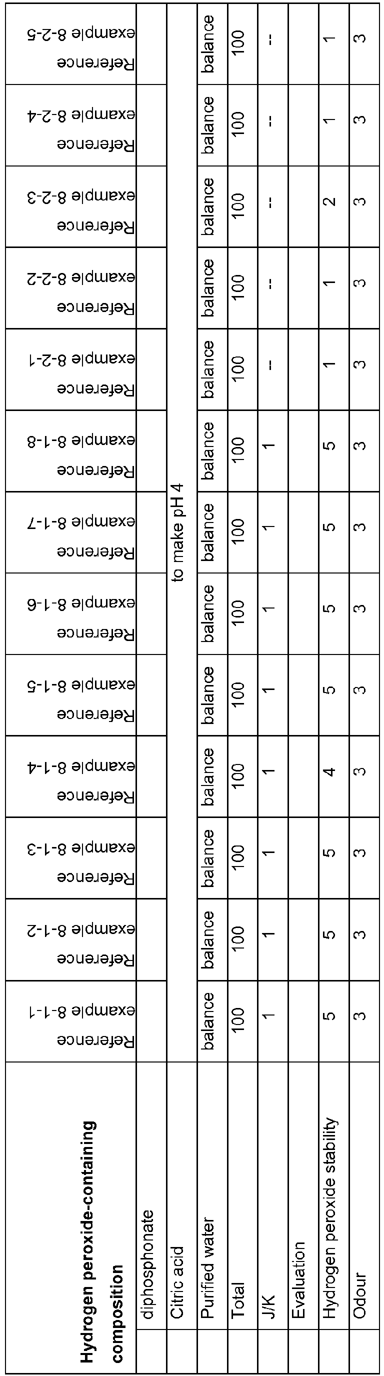

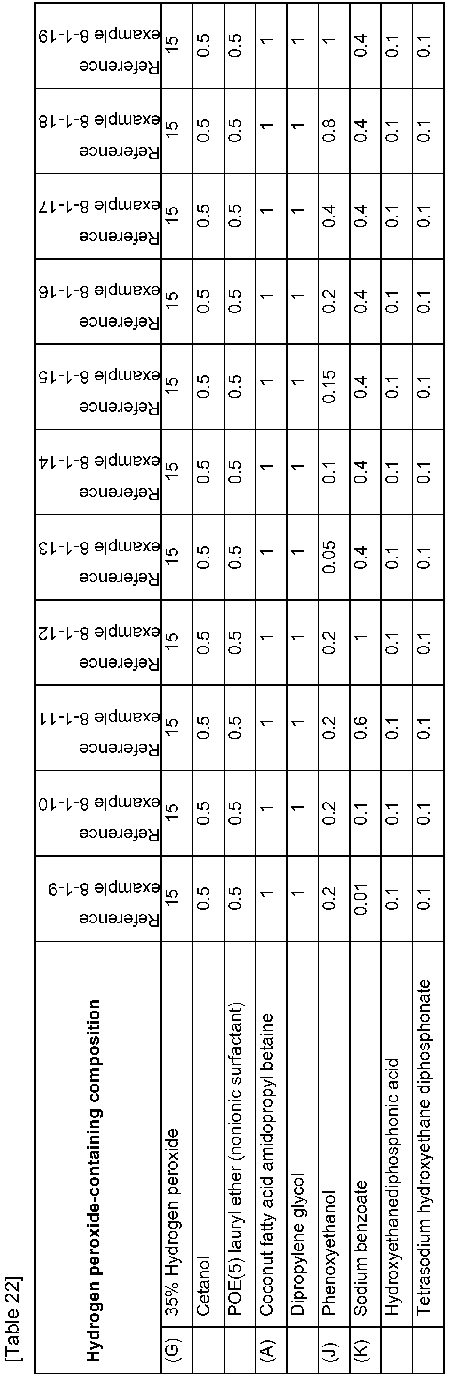

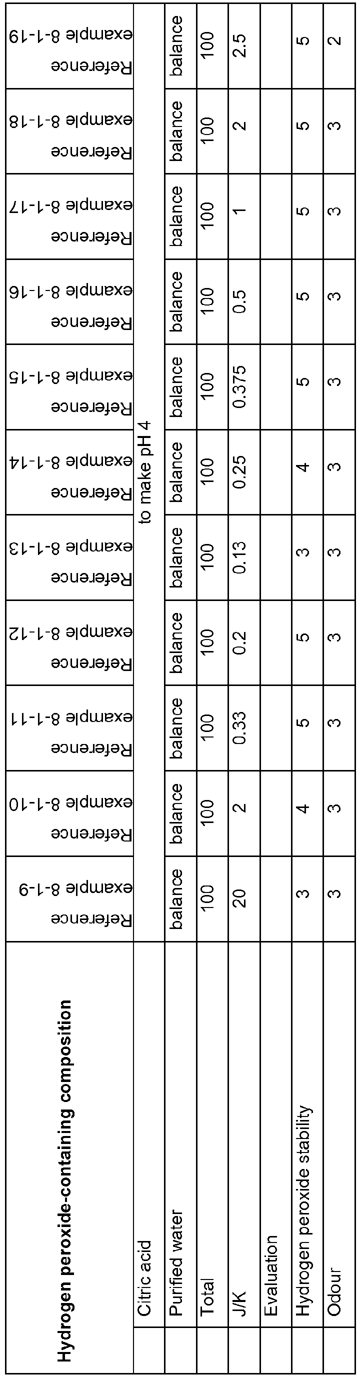

As shown in Table 21, the hydrogen peroxide-containing compositions of Reference Examples 8-2-1 to 8-2-5 did not contain either phenoxyethanol or at least one benzoic acid (benzoate) in the system containing the surfactant. It was understood by examining the stability of the hydrogen peroxide during storage in Reference Examples 8-2-1 to 8-2-5 that the stability was inferior. The hydrogen peroxide-containing compositions of Reference Examples 8-2-1, 8-2-2, 8-2-4, and 8-2-5 among them contained an amphoteric surfactant as a surfactant. These had especially inferior hydrogen peroxide stability during storage.

In contrast, the hydrogen peroxide-containing compositions of Reference Examples 8-1-1 to 8-1-19 contained both phenoxyethanol and benzoic acid (benzoate) in the system containing the surfactant, and it was possible to confirm excellent stability of the hydrogen peroxide during storage. It is understood by comparing Reference Examples 8-1-1 and 8-1-2 with Reference Examples 8-2-1 and 8-2-2 that Reference Examples 8-1-1 and 8-1-2 have improved hydrogen peroxide stability. It is also understood by comparing Reference Example 8-1-5 with Reference Example 8-2-4 that Reference Example 8-1-5 has improved hydrogen peroxide stability. Moreover, it is understood by comparing Reference Example 8-1-8 with Reference Examples 8-2-4 and 8-2-5 that Reference Example 8-1-8 has improved hydrogen peroxide stability. It is thus understood by comparison with cases in which an amphoteric surfactant was contained as the surfactant (Reference Examples 8-1-1, 8-2-1, 8-2-2, and so forth) that improvement of the hydrogen peroxide stability is especially great when an amphoteric surfactant is contained.

The following is thus understood by comparison between Reference Examples 8-1-1 to 8-1-19. Specifically, it is understood by comparing Reference Examples 8-1-9 and 8-1-13 with the others that the hydrogen peroxide stability is even better if the weight ratio (J/K ratio) of component J to component K is in the range of 0.2 to 2.5. It is also understood by comparing Reference Examples 8-1-9 and 8-1-10 with the others that the hydrogen peroxide stability is even better if the amount of component K is 0.1 wt% or higher and that the hydrogen peroxide stability is especially excellent if the amount of component K is 0.15 wt% or higher. It is also understood by comparing Reference Examples 8-1-13 and 8-1-14 with the others that the hydrogen peroxide stability is even better if the amount of component J is 0.1 wt% or higher and that the hydrogen peroxide stability is especially excellent if the amount of component J is 0.15 wt% or higher. Furthermore, it is understood by comparing Reference Example 8-1-19 with the others that hydrogen peroxide stability can be achieved while suppressing the odour of phenoxyethanol if the amount of component J combined is in the range of 0.15 to 0.8 wt%.

### Test 9 (Preparation of composition for hair dyeing)

First and second agents of the compositions for hair dyeing of Working Examples 9-1 to 9-5 and 9-7 to 9-14 and Reference Examples 9-1 to 9-4 and 9-6 were prepared by combining the components listed in Tables 23 and 24. Next, the compositions for hair dyeing were prepared by mixing the resulting first agents and second agents in the mixture ratios (weight ratios) listed in Tables 23 and 24. For those listed as having a final form of 'foam' in Tables 23 and 24, the first agent and second agent were placed in a sealed container, and a foamy composition for hair dyeing was obtained by shaking the container. On the other hand, for those listed as having a final form of 'cream,' the first agent and second agent were placed in an open container and mixed gently so that foaming did not occur inside the container. The numerical values representing the content of each component of the compositions for hair dyeing in Tables 23 and 24 are expressed in units of percentage by weight.

### <Dyeing>

The prepared compositions for hair dyeing were applied to strands of black human hair by a gloved hand using a thin glove made of resin, and the hair strands were allowed to stand for 30 minutes in a thermostatic chamber (30°C). The strands of hair were then washed with tap water, then shampooed and rinsed (conditioner) one time each. The hair strands were then dried and allowed to stand for one day thereafter at normal temperature. The strands of hair were dyed in this way.

### <Finished feel (sleekness)>

The feel of the hair strand when touched by a finger after dyeing and the feel of an undyed hair strand when touched by a finger were compared, and the feel of the dyed hair strand was evaluated in view of an absence of a dry feel to the hair strand and whether the feeling of sleekness was good when passing a hand through the hair. The evaluation criteria were as follows. The results of evaluation are shown in Tables 23 and 24.
Evaluation 5: Sleekness very good
Evaluation 4: Sleekness good
Evaluation 3: Sleekness relatively good
Evaluation 2: Sleekness somewhat poor
Evaluation 1: Sleekness very poor

### <Finished feel (squeakiness, tangling, stiffness)>

The feel of the hair strand when touched by a finger after dyeing and the feel of an undyed hair strand when touched by a finger were compared, and the feel of the dyed hair strand was evaluated in view of squeakiness, tangling, and stiffness of the hair and whether or not a finger passed through it easily. The evaluation criteria were as follows. The results of evaluation are shown in Tables 23 and 24.
Evaluation 5: Very good feel
Evaluation 4: Good feel
Evaluation 3: Relatively good feel
Evaluation 2: Somewhat poor feel
Evaluation 1: Very poor feel

### <Finished brightness>

The finished brightness was evaluated by visual examination of the hair strands after dyeing. The evaluation criteria were as follows. The results of evaluation are shown in Tables 23 and 24.
Evaluation 5: Brightness very good
Evaluation 4: Brightness good
Evaluation 3: Brightness relatively good
Evaluation 2: Brightness somewhat poor
Evaluation 1: Brightness very poor

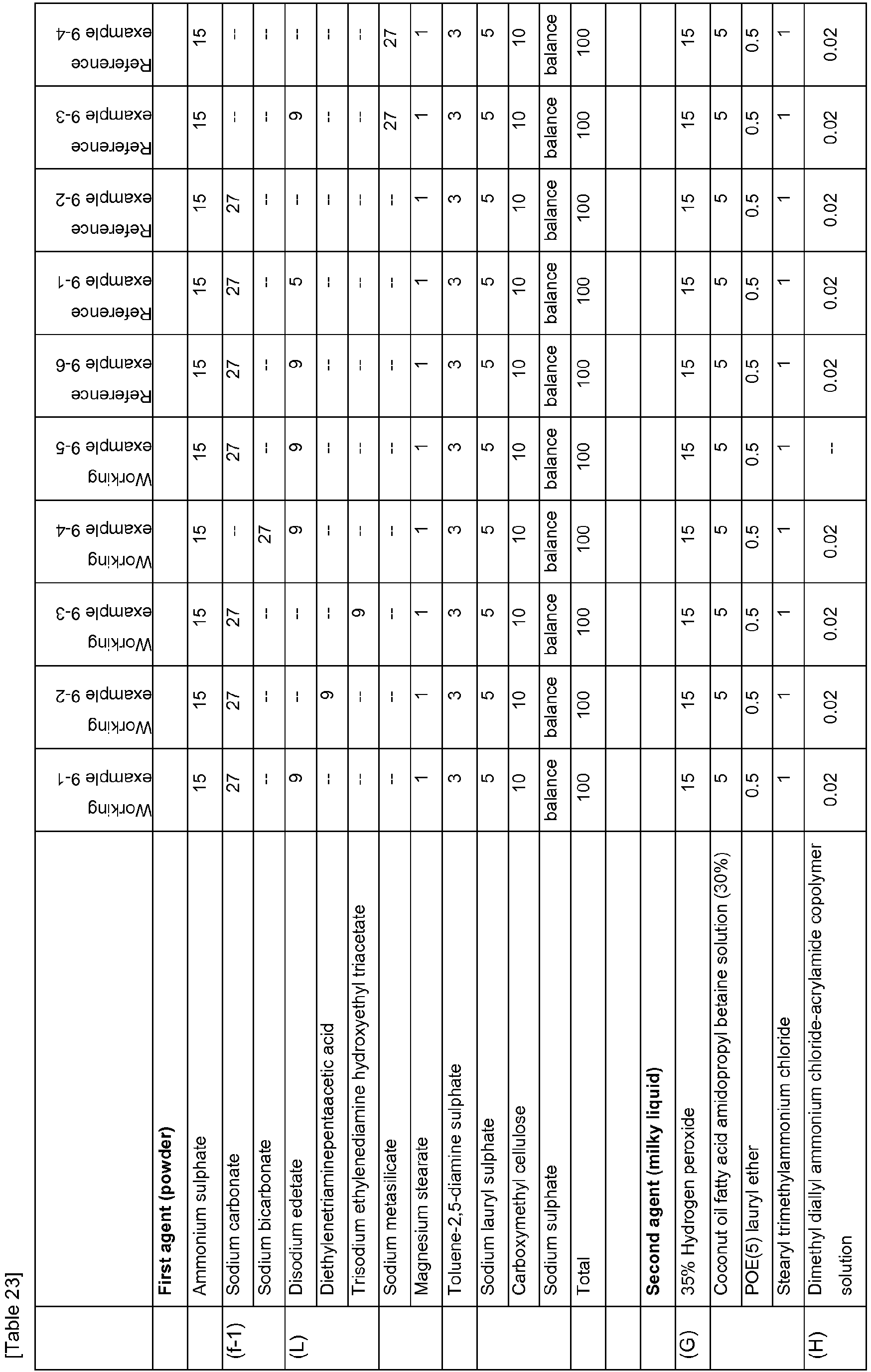

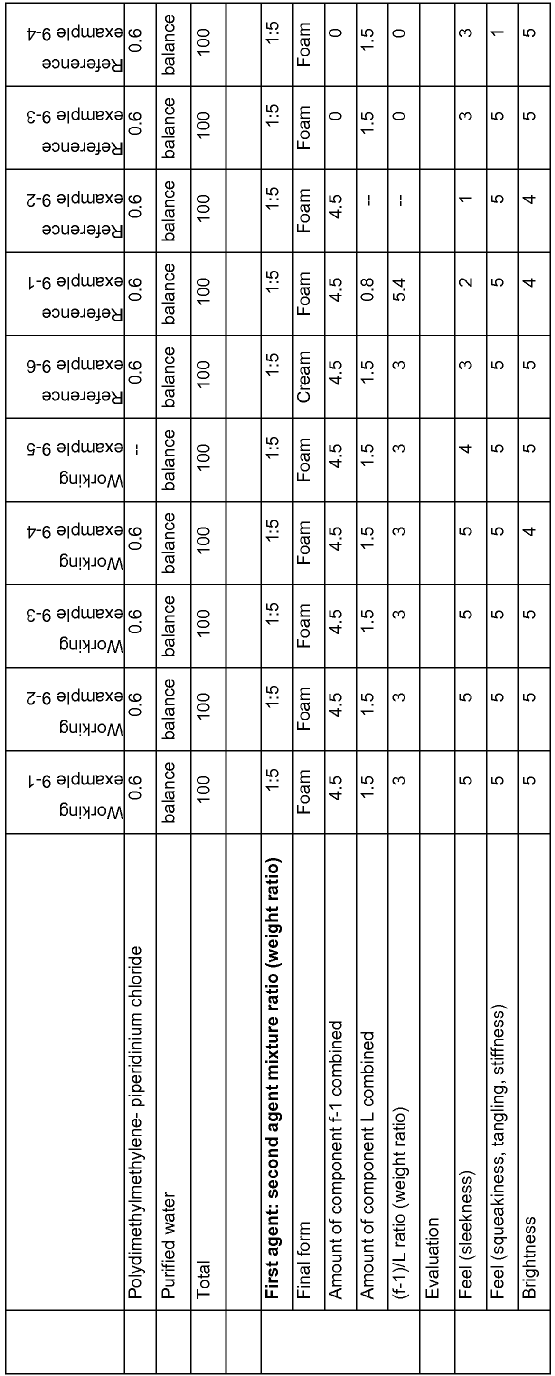

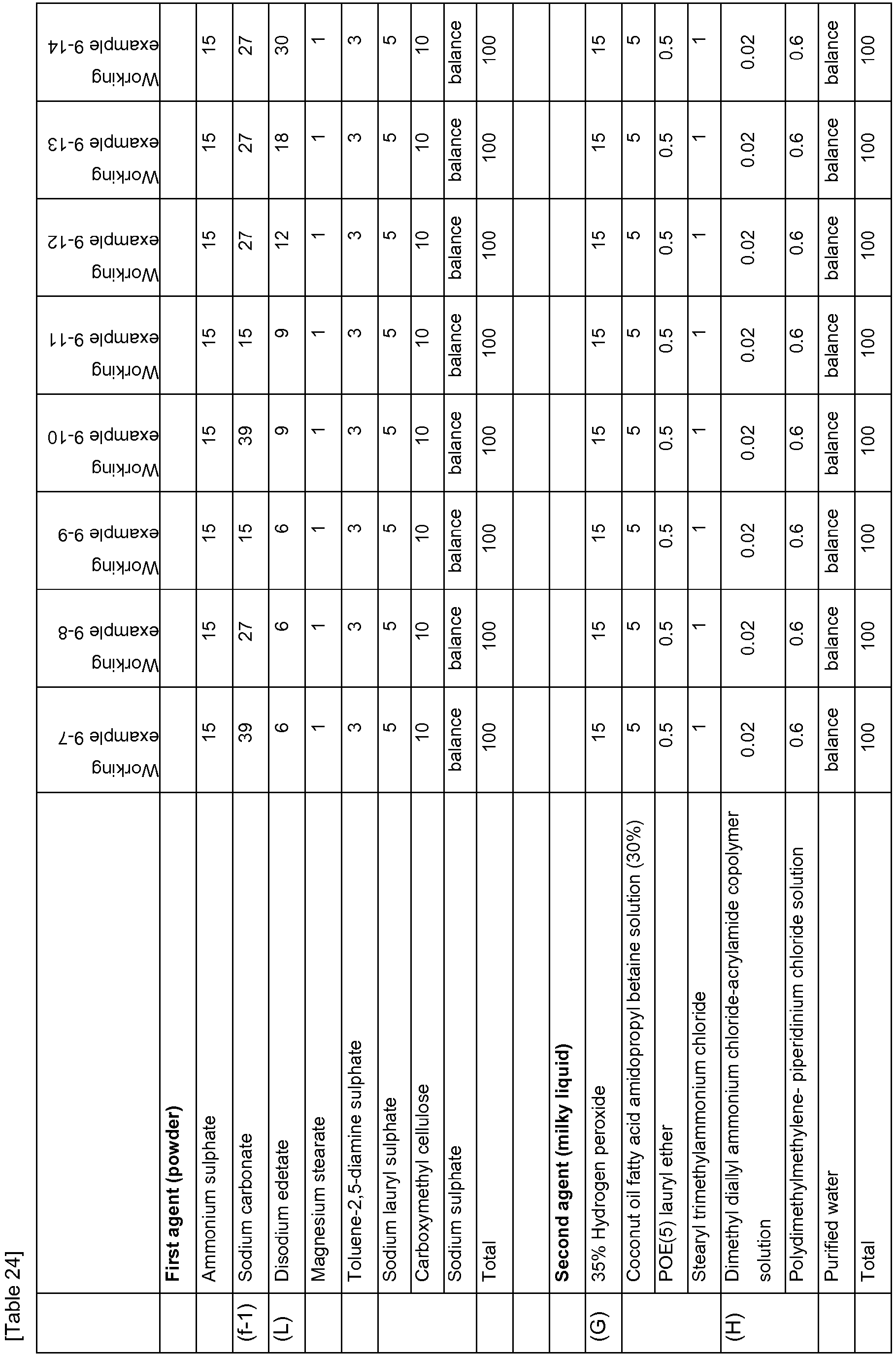

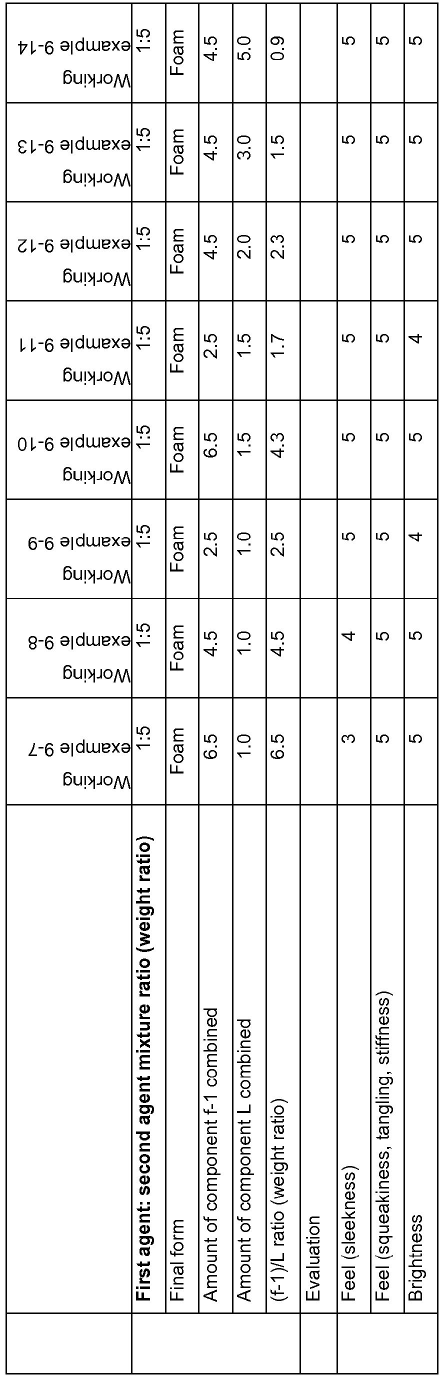

Based on Tables 23 and 24, the finished feel (sleekness) was somewhat poor in the composition for hair dyeing of Reference Example 9-1 because the amount of component L combined was less than 1 wt%. The finished feel (sleekness) was very poor in the composition for hair dyeing of Reference Example 9-2 because no component L was combined.

In contrast to this, the compositions for hair dyeing of the working examples were confirmed to have good finished feel (sleekness) and good finished brightness as well. A comparison of the working examples finds that Working Example 9-5 did not combine a specific cationic polymer. The finished feel (sleekness) was therefore somewhat inferior to that of the other working examples. The final form was a cream in Reference Example 9-6. The final form was a foam in the working examples, and it was possible to confirm that the finished feel was even better than in Reference Example 9-6.

The weight ratio of component f-1 to component L exceeded 4.5 in Working Example 9-7. In contrast to this, the above weight ratio fell within the 0.02 to 4.5 range in the working examples other than Working Example 9-7. It was possible to confirm that the finished feel (sleekness) was even better in the working examples other than Working Example 9-7 than in Working Example 9-7.

Furthermore, Reference Examples 9-3 and 9-4 used sodium metasilicate instead of component f-1. Improvement of the finished feel was seen in Reference Example 9-3 in contrast to Reference Example 9-4 due to suppression of hardening, squeakiness, tangling, stiffness, and the like of the finished hair strand, but no improvement was seen of the finished feeling of sleekness due to suppression of a feeling of dryness after finishing.

Embodiments of the present invention have been explained in detail above, but the present invention is not in any way limited to the above embodiments; various modifications being possible as long as no departure is made from the spirit of the invention.

### Test 10-1

Hair dyes comprising a powdered first agent containing the components shown in Table 25 and a milky-liquid-form second agent shown in Tables 26 and 27 were prepared. The second agent was prepared by first preparing an amphoteric surfactant-containing aqueous solution and then mixing this amphoteric surfactant-containing aqueous solution with the other components, for example, hydrogen peroxide and the like. The numerical values representing the content of each component of the hair dyes in Tables 25 to 27 are expressed in units of percentage by weight. Foamy hair dyes were prepared by mixing the first agent and second agent in a 1:5 weight ratio and stirring using a stirring rod. The foamability of the hair dyes obtained was evaluated. The changes in pH and the pH stability of the second agent obtained were also evaluated. The results are shown in Tables 26 and 27.

The numerical values in the 'Weight ratio of amphoteric surfactant' column in Tables 26 and 27 show the weight ratio (A/I) of the amphoteric surfactant content to the alkali metal inorganic salt content of the amphoteric surfactant-containing aqueous solution.

Amogen CB-H (30 wt% coconut oil fatty acid amidopropyl betaine (desalted), made by Daiichi Kogyo Co, Ltd) was used as the coconut oil fatty acid amidopropyl betaine in Tables 26 and 27. The coconut oil fatty acid amidopropyl betaine of Working Examples 10-8 and 10-9 was used after heating the above product having a concentration of 30 wt% to 80°C and evaporating off the water. Obazolin LB-SF (35 wt% lauryl dimethylaminoacetic acid betaine (desalted), made by Toho Chemical Industry Co, Ltd) was used as the lauryl dimethylaminoacetic acid betaine in Tables 26 and 27. Softazolin LPB-R (30 wt% lauric acid amidopropyl betaine (desalted), made by Kawaken Fine Chemicals Co, Ltd) was used as the lauric acid amidopropyl betaine in Tables 26 and 27. The numerical values representing the content of each component in Tables 26 and 27 show the pure fraction of that component.

### <Change in pH and pH stability of second agent>

The pH of the second agent was measured after storing the second agent of the hair dye of each example for one month in a 45°C thermostatic chamber. The change in pH from pH 4 at the time of preparation of the second agent was determined from that value.

In the 'pH stability of second agent' column in Tables 26 and 27, '5' shows a change in pH of less than 0.10, '4' shows a change in pH of from 0.10 to less than 0.15, '3' shows a change in pH of from 0.15 to less than 0.20, '2' shows a change in pH of from 0.20 to less than 0.25, and '1' shows a change in pH of 0.25 or greater.

### <Foamability>

A panel of experts examined the state of the hair dye visually after foaming. In the 'Foamability' column in Tables 26 and 27, '5' shows 'highly exceptional foaming,' '4' shows 'excellent foaming,' '3' shows 'good foaming,' '2' shows 'somewhat inadequate foaming,' and '1' shows 'inadequate foaming or excessive foaming creating low-viscosity, light, large foam'.

**[Table 25]**

| **First agent (powder)** | |
|---|---|
| Ammonium sulphate | 15 |
| Sodium carbonate | 30 |
| Disodium edetate | 3 |
| Magnesium stearate | 1 |
| Toluene-2,5-diamine sulphate | 3 |
| Sodium lauryl sulphate | 5 |
| Carboxymethyl cellulose | 10 |
| Sodium sulphate | balance |
| Total | 100 |

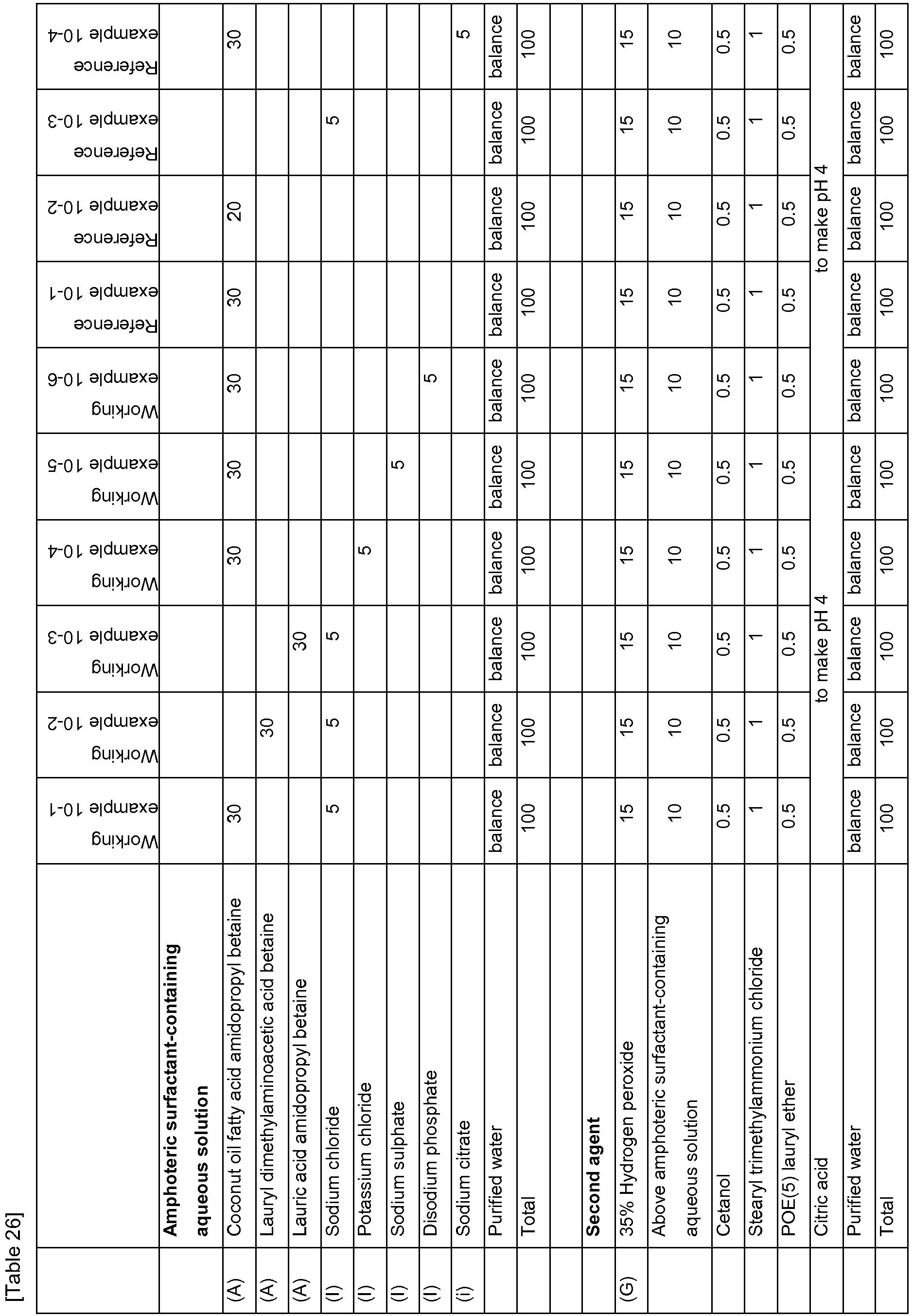

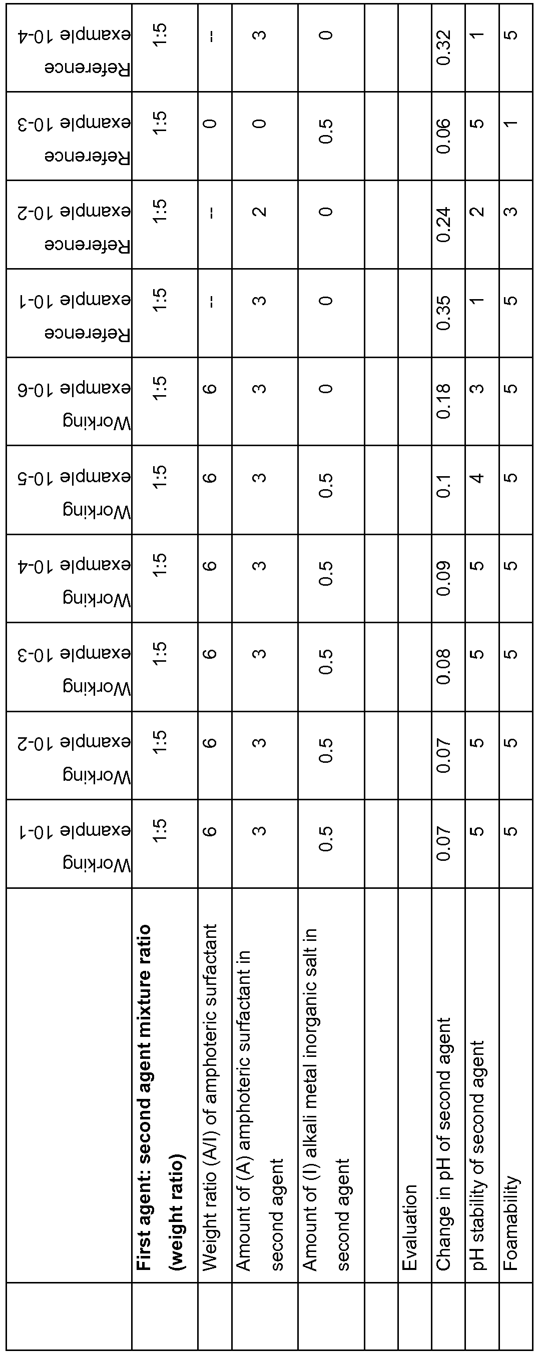

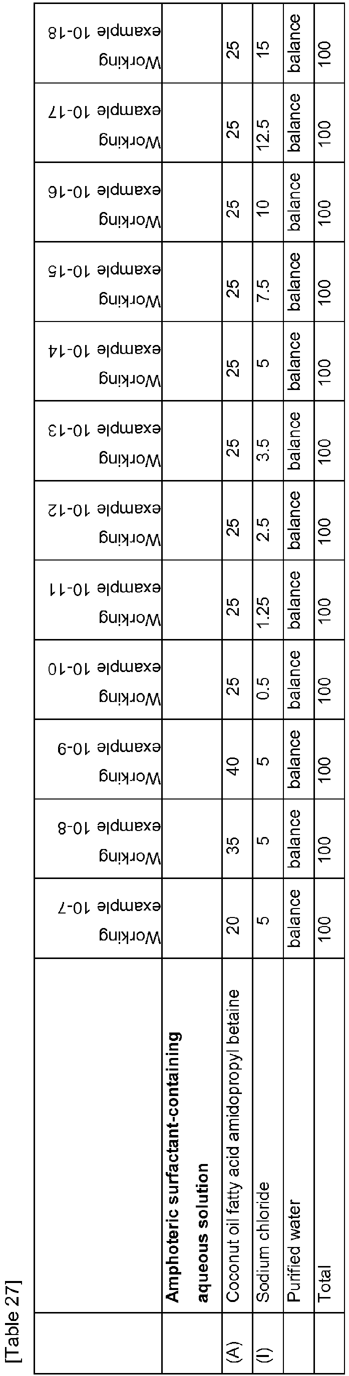

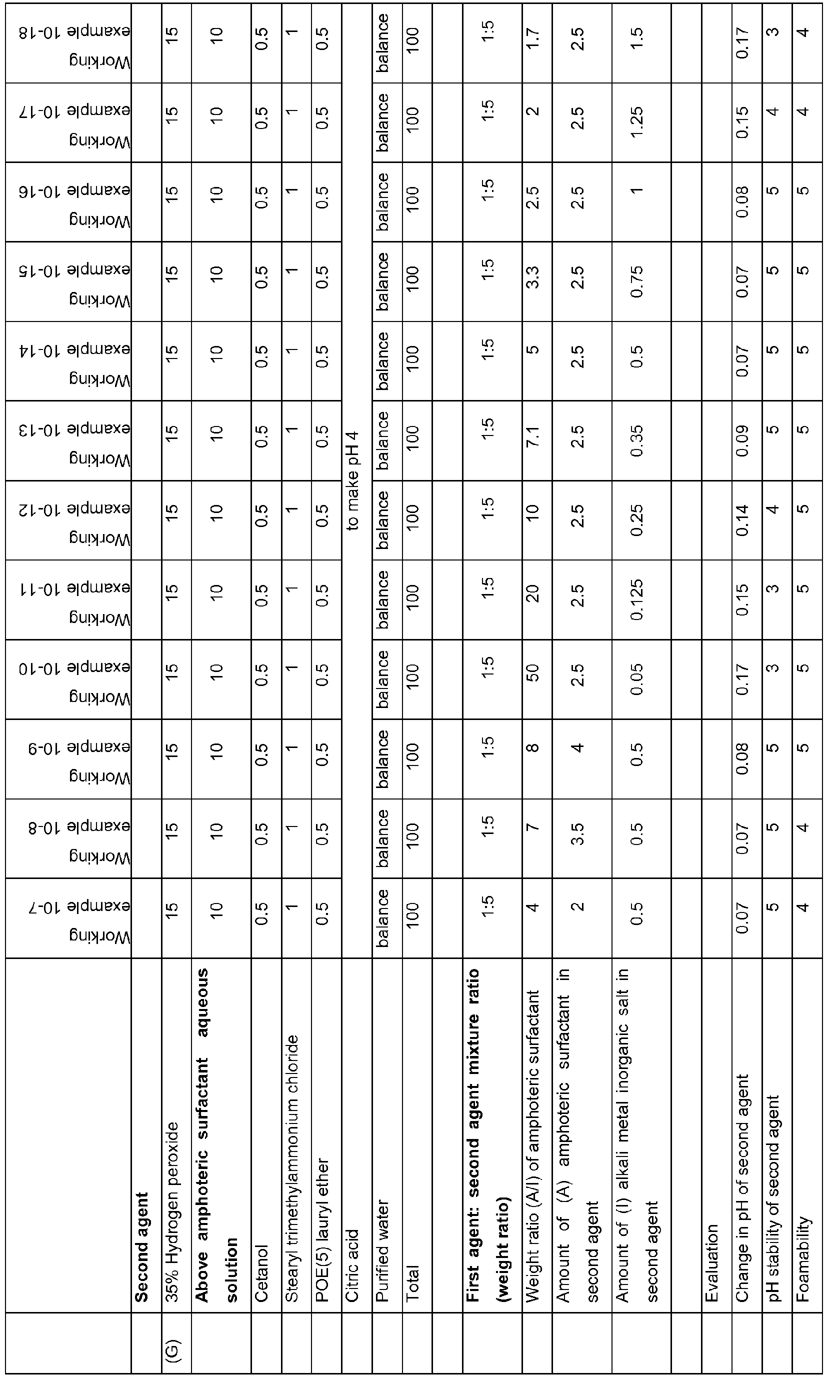

As shown in Tables 26 and 27, hair dyes of the working examples constituted by combining (B) amphoteric surfactant and (C) [sic] alkali metal inorganic salt in the second agent obtained good results in the category 'pH stability'.

Reference Examples 10-1 and 10-2 that did not contain an alkali metal inorganic salt in the second agent were understood to receive a low evaluation in comparison to the working examples in the category 'pH stability'.
Reference Example 10-3 that did not contain an amphoteric surfactant in the second agent received a low evaluation in comparison to the working examples in the category 'foamability'. It is difficult to obtain a good foamy hair dye when no amphoteric surfactant is used.

Reference Example 10-4 that contained sodium citrate instead of an alkali metal inorganic salt in the second agent received a low evaluation in comparison to the working examples in the category 'pH stability'.

### (Test 10-2)

Milky-liquid-like second agents containing the components shown in Tables 28 to 34 were prepared. For the second agents, amphoteric surfactant-containing aqueous solution was first prepared by mixing the components listed in the table in order from the top of the table. Next, the second agent was prepared by mixing the other components in order from the top of the table with this amphoteric surfactant-containing aqueous solution. The numerical values in the columns showing the components in Tables 28 to 34 show the amounts of each component listed in that column unless indicated otherwise in the table. The values are expressed in units of percentage by weight. The numerical values of each component shown in the amphoteric surfactant-containing aqueous solution column show the content of that component in the second agent. Foamy hair dyes were prepared by mixing the first agent shown in Table 25 and the second agents shown in Tables 28 to 34 in a 1: 5 weight ratio and stirring using a stirring rod. The foamability of the hair dyes obtained was evaluated. The changes in pH and the pH stability of the second agents obtained were also evaluated. The results are shown in Tables 28 to 34.

**[Table 28]**

| | **Second agent (liquid)** | Working Example 10-19 |
|---|---|---|
| | Amphoteric surfactant-containing aqueous solution | |
| | (A) coconut oil fatty acid amidopropyl betaine | 2.4 |
| | Purified water | 5.6 |
| | (I) Sodium chloride | 0.4 |
| | Cetanol | 0.5 |
| | Stearyl trimethylammonium chloride | 1 |
| | POE(5) lauryl ether | 0.5 |
| | Citric acid | to make pH 4 |
| | Purified water | to make a total of 85 wt% |
| (G) | 35% Hydrogen peroxide | 15 |
| | Total | 100 |
| | | |

| | **Mixture ratio of first agent: second agent (weight ratio)** | 1: 5 |
|---|---|---|
| | (A) amphoteric surfactant content of amphoteric surfactant-containing aqueous solution | 28.6 |
| | (I) alkali metal inorganic salt content of amphoteric surfactant-containing aqueous solution | 4.8 |
| | Weight ratio (A/I) of amphoteric surfactant | 6 |
| | | |

| | Evaluation | |
|---|---|---|
| | Change in pH of second agent | 0.07 |
| | pH stability of second agent | 5 |
| | Foamability | 5 |

| | | |
|---|---|---|
| Components mixed in order from top of table | | |

**[Table 29]**

| | **Second agent (liquid)** | Working Example 10-20 |
|---|---|---|
| | Amphoteric surfactant-containing aqueous solution | |
| | (A) coconut oil fatty acid amidopropyl betaine | 2.4 |
| | Purified water | 9.2 |
| | (I) Sodium chloride | 0.4 |
| | Cetanol | 0.5 |
| | Stearyl trimethylammonium chloride | 1 |
| | POE(5) lauryl ether | 0.5 |
| | Citric acid | to make pH 4 |
| | Purified water | to make a total of 85 wt% |
| (G) | 35% Hydrogen peroxide | 15 |
| | Total | 100 |
| | | |

| | **Mixture ratio of first agent: second agent (weight ratio)** | 1: 5 |
|---|---|---|
| | (A) amphoteric surfactant content of amphoteric surfactant-containing aqueous solution | 20.0 |
| | (I) alkali metal inorganic salt content of amphoteric surfactant-containing aqueous solution | 3.3 |
| | Weight ratio (A/I) of amphoteric surfactant | 6 |
| | | |

| | Evaluation | |
|---|---|---|
| | Change in pH of second agent | 0.16 |
| | pH stability of second agent | 3 |
| | Foamability | 5 |

| | | |
|---|---|---|
| Components mixed in order from top of table | | |

**[Table 30]**

| | **Second agent (liquid)** | Working Example 10-21 |
|---|---|---|
| | Amphoteric surfactant-containing aqueous solution | |
| | (A) coconut oil fatty acid amidopropyl betaine | 2.4 |
| | Purified water | 5.6 |
| | Cetanol | 0.5 |
| | (I) Sodium chloride | 0.4 |
| | Stearyl trimethylammonium chloride | 1 |
| | POE(5) lauryl ether | 0.5 |
| | Citric acid | to make pH 4 |
| | Purified water | to make a total of 85 wt% |
| (G) | 35% Hydrogen peroxide | 15 |
| | Total | 100 |
| | | |

| | **Mixture ratio of first agent: second agent (weight ratio)** | 1:5 |
|---|---|---|
| | (A) amphoteric surfactant content of amphoteric surfactant-containing aqueous solution | 27.0 |
| | (I) alkali metal inorganic salt content of amphoteric surfactant-containing aqueous solution | 4.5 |
| | Weight ratio (A/I) of amphoteric surfactant | 6 |
| | | |

| | Evaluation | |
|---|---|---|
| | Change in pH of second agent | 0.07 |
| | pH stability of second agent | 5 |
| | Foamability | 5 |

| | | |
|---|---|---|
| Components mixed in order from top of table | | |

**[Table 31]**

| | **Second agent (liquid)** | Working Example 10-22 |
|---|---|---|
| | Amphoteric surfactant-containing aqueous solution | |
| | (A) coconut oil fatty acid amidopropyl betaine | 2.4 |
| | Purified water | 5.6 |
| | Cetanol | 0.5 |
| | Stearyl trimethylammonium chloride | 1 |
| | POE(5) lauryl ether | 0.5 |
| | (I) Sodium chloride | 0.4 |
| | Citric acid | to make pH 4 |
| | Purified water | to make a total of 85 wt% |
| (G) | 35% Hydrogen peroxide | 15 |
| | Total | 100 |

| | **Mixture ratio of first agent: second agent (weight ratio)** | 1: 5 |
|---|---|---|
| | (A) amphoteric surfactant content of amphoteric surfactant-containing aqueous solution | 23.1 |
| | (I) alkali metal inorganic salt content of amphoteric surfactant-containing aqueous solution | 3.8 |
| | Weight ratio (A/I) of amphoteric surfactant | 6 |
| | | |

| | Evaluation | |
|---|---|---|
| | Change in pH of second agent | 0.07 |
| | pH stability of second agent | 5 |
| | Foamability | 5 |

| | | |
|---|---|---|
| Components mixed in order from top of table | | |

**[Table 32]**

| | **Second agent (liquid)** | Working Example 10-23 |
|---|---|---|
| | Amphoteric surfactant-containing aqueous solution | |
| | Purified water | 5.6 |
| | (I) Sodium chloride | 0.4 |
| | Cetanol | 0.5 |
| | (A) coconut oil fatty acid amidopropyl betaine | 2.4 |
| | Stearyl trimethylammonium chloride | 1 |
| | POE(5) lauryl ether | 0.5 |
| | Citric acid | to make pH 4 |
| | Purified water | to make a total of 85 wt% |
| (G) | 35% Hydrogen peroxide | 15 |
| | Total | 100 |
| | | |

| | **Mixture ratio of first agent: second agent (weight ratio)** | 1: 5 |
|---|---|---|
| | (A) amphoteric surfactant content of amphoteric surfactant-containing aqueous solution | 27.0 |
| | (I) alkali metal inorganic salt content of amphoteric surfactant-containing aqueous solution | 4.5 |
| | Weight ratio (A/I) of amphoteric surfactant | 6 |
| | | |

| | Evaluation | |
|---|---|---|
| | Change in pH of second agent | 0.08 |
| | pH stability of second agent | 5 |
| | Foamability | 5 |

| | | |
|---|---|---|
| Components mixed in order from top of table | | |

**[Table 33]**

| | **Second agent (liquid)** | Working Example 10-24 |
|---|---|---|
| | Amphoteric surfactant-containing aqueous solution | |
| | Purified water | 5.6 |
| | (I) Sodium chloride | 0.2 |
| | Cetanol | 0.5 |
| | (A) coconut oil fatty acid amidopropyl betaine | 2.4 |
| | Stearyl trimethylammonium chloride | 1 |
| | POE(5) lauryl ether | 0.5 |
| | Citric acid | to make pH 4 |
| | Purified water | to make a total of 85 wt% |
| (G) | 35% Hydrogen peroxide | 15 |
| | Total | 100 |
| | | |

| | **Mixture ratio of first agent: second agent (weight ratio)** | 1: 5 |
|---|---|---|
| | (A) amphoteric surfactant content of amphoteric surfactant-containing aqueous solution | 27.6 |
| | (I) alkali metal inorganic salt content of amphoteric surfactant-containing aqueous solution | 2.3 |
| | Weight ratio (A/I) of amphoteric surfactant | 12 |
| | | |

| | Evaluation | |
|---|---|---|
| | Change in pH of second agent | 0.15 |
| | pH stability of second agent | 3 |
| | Foamability | 5 |

| | | |
|---|---|---|
| Components mixed in order from top of table | | |

**[Table 34]**

| | **Second agent (liquid)** | Working Example 10-25 |
|---|---|---|
| | Amphoteric surfactant-containing aqueous solution | |
| | Purified water | 8.7 |
| | (I) Sodium chloride | 0.4 |
| | Cetanol | 0.5 |
| | (A) coconut oil fatty acid amidopropyl betaine | 2.4 |
| | Stearyl trimethylammonium chloride | 1 |
| | POE(5) lauryl ether | 0.5 |
| | Citric acid | to make pH 4 |
| | Purified water | to make a total of 85 wt% |
| (G) | 35% Hydrogen peroxide | 15 |
| | Total | 100 |
| | | |

| | **Mixture ratio of first agent: second agent (weight ratio)** | 1: 5 |
|---|---|---|
| | (A) amphoteric surfactant content of amphoteric surfactant-containing aqueous solution | 20.0 |
| | (I) alkali metal inorganic salt content of amphoteric surfactant-containing aqueous solution | 3.3 |
| | Weight ratio (A/I) of amphoteric surfactant | 6 |
| | | |

| | Evaluation | |
|---|---|---|
| | Change in pH of second agent | 0.16 |
| | pH stability of second agent | 3 |
| | Foamability | 5 |

| | | |
|---|---|---|
| Components mixed in order from top of table | | |

As shown in Tables 28 to 31, Working Examples 10-19, 10-21, and 10-22 in which sodium chloride was added after combining approximately 2.3 parts by weight of water per part by weight of amphoteric surfactant during preparation of the amphoteric surfactant-containing aqueous solution were understood to have superior pH stability relative to Working Example 10-20 in which sodium chloride was added after combining approximately 3.8 parts by weight of water per part by weight of amphoteric surfactant.

As shown in Tables 32 to 34, Working Example 10-23 in which amphoteric surfactant was added after mixing approximately 14 parts by weight of water per part by weight of sodium chloride during preparation of the amphoteric surfactant-containing aqueous solution was understood to have superior pH stability to Working Examples 10-24 and 10-25 in which amphoteric surfactant was added after mixing approximately 20 parts by weight or more of water per part by weight of sodium chloride.

Next, the following is appended as technical concepts that can be appreciated from the above embodiments and other examples.

A hair cosmetic that provides
a cosmetic preparation for hair to be applied to the hair in the form of a foam obtained by mixing a powdered agent and a liquid agent and creating a foam through shaking and
a foaming tool to induce foaming by mixing and shaking a powdered agent and a liquid agent.
A foamy cosmetic preparation for hair can be prepared simply in this case.

### Key to figures FIG 1 and FIG 2

10...Agent to remove colour or dye from hair, 11...First agent, 12...Second agent, 13...Mixture, 14...Foamy agent for bleaching or removing dye from hair, 20...Container, 21...Container body, 22...Lid

## Claims

1. A method of dyeing hair or bleaching hair or removing dye from hair,
the method being **characterised in** comprising:
a step of forming a cosmetic preparation for hair in the form of a foam by introducing a powdered first agent (11) including an alkali agent and liquid second agent (12) of a cosmetic preparation for hair including hydrogen peroxide as an oxidising agent into the container body (21), bringing the first agent (11) and second agent (12) into contact in this way and obtaining a mixture (13);
attaching a lid (22) to the container body (21), wherein a flange-shaped fitting part is formed on the rim of the lid (22), and the fitting part of the lid (22) is fitted to the opening of the container body (21) so that the lid (22) is attached in a fluid-tight manner to the container body (21) by rotating the lid (22),
thereby obtaining a fluid-tight, sealable container (20) and
creating a cosmetic preparation for hair in the form of a foam (14) through shaking the container (20);
removing the lid (22) from the container body (21); and
a step of manually removing the foam (14) directly from the container body (21) by hand and applying it to the hair.

2. The method according to Claim 1 **characterised in that** the cosmetic preparation for hair comprises an amphoteric surfactant.

3. The method according to Claim 1 or Claim 2 **characterised in that** the cosmetic preparation for hair comprises a cationic surfactant.

4. The method according to Claim 3 **characterised in that** the cosmetic preparation for hair further comprises an anionic surfactant; the anionic surfactant being brought into contact with the cationic surfactant in the presence of a solvent at the time of use.

5. The method according to any of Claim 1 through Claim 4 **characterised in that** the cosmetic preparation for hair comprises a nonionic polymer; the nonionic polymer being combined with the powdered agent.

6. The method according to Claim 5 **characterised in that** the cosmetic preparation for hair comprises a thickener; the thickener being combined with the powdered agent.

7. The method according to any of Claim 1 through Claim 6 **characterised in that** the second agent also includes an amphoteric surfactant and a cationic polymer.

8. The method according to Claim 2 or Claim 7 **characterised in that** the cosmetic preparation for hair also includes an alkali metal inorganic salt.

9. The method according to any of Claim 1 through Claim 8 **characterised in that**:
the alkali agent is a carbonate;
the cosmetic preparation for hair also includes 1 to 5 wt% of a chelating agent; and
the weight ratio of the carbonate content of the cosmetic preparation for hair to the chelating agent content of the cosmetic preparation for hair is 0.02 to 6.5.

10. The method according to any of Claim 1 through Claim 9 **characterised in that** the container (20) is provided with a bottomed cylindrical container body (21) and a hemispherical lid (22) that seals the opening of the container body (21).

11. The method according to any of Claim 1 through Claim 10 **characterised in that** the container body (21) is shaped so that the opening is wider than the bottom.

12. The method according to any of Claim 1 through Claim 11 **characterised in that** the height h1 of the housing part inside the container (20) is 10 to 25 cm.

13. The method according to Claim 12 **characterised in that** the ratio (h1/h2) of the height h1 (cm) of the housing part inside the container (20) to the height h2 (cm) of the mixture (13) inside the container (20) before foaming is in the range of 2 to 15.

14. The method according to any of Claim 1 through Claim 13 **characterised in that** the first agent (11) contains at least one component selected from direct dyes and oxidation dyes.

15. The method according to any of Claim 1 through Claim 14 **characterised in that** the alkali agent is comprised in such a quantity that the pH of the mixture of the first agent and a second agent falls in the range of 7 to 12 at the time of use.

## Patentansprüche

1. Verfahren zum Färben von Haaren oder zum Bleichen von Haaren oder zum Entfernen eines Farbstoffes aus den Haaren, wobei das Verfahren **dadurch gekennzeichnet ist, dass** es umfasst:
einen Schritt des Ausbildens einer kosmetischen Zubereitung für Haare in der Form eines Schaums durch Einführen eines pulverförmigen ersten Mittels (11), das ein Alkalimittel beinhaltet, und eines flüssigen zweiten Mittels (12) einer kosmetischen Zubereitung für Haare, die Wasserstoffperoxid als ein Oxidationsmittel beinhaltet, in den Behälterkörper (21), wobei das erste Mittel (11) und das zweite Mittel (12) auf diese Weise in Kontakt gebracht werden und eine Mischung (13) erhalten wird;
Anbringen eines Deckels (22) an den Behälterkörper (21), wobei ein flanschförmiges Passteil auf dem Rand des Deckels (22) ausgebildet wird und das Passteil des Deckels (22) an der Öffnung des Behälterkörpers (21) derart angebracht ist, dass der Deckel (22) durch Drehen des Deckels (22) in einer fluiddichten Weise an dem Behälterkörper (21) befestigt wird, wobei dadurch ein fluiddichter, verschließbarer Behälter (20) erhalten wird und durch Schütteln des Behälters (20) eine kosmetische Zubereitung für Haare in der Form eines Schaums (14) gebildet wird;
Entfernen des Deckels (22) von dem Behälterkörper (21); und
einen Schritt des manuellen Entfernens des Schaums (14) direkt von dem Behälterkörper (21) von Hand und des Aufbringens auf das Haar.

2. Verfahren nach Anspruch 1, **dadurch gekennzeichnet, dass** die kosmetische Zubereitung für Haare ein amphoteres Tensid umfasst.

3. Verfahren nach Anspruch 1 oder 2, **dadurch gekennzeichnet, dass** die kosmetische Zubereitung für Haare ein kationisches Tensid umfasst.

4. Verfahren nach Anspruch 3, **dadurch gekennzeichnet, dass** die kosmetische Zubereitung für Haare ferner ein anionisches Tensid umfasst;
wobei das anionische Tensid zu dem Zeitpunkt der Verwendung in der Gegenwart eines Lösungsmittels mit dem kationischen Tensid in Kontakt gebracht wird.

5. Verfahren nach einem der Ansprüche 1 bis 4, **dadurch gekennzeichnet, dass** die kosmetische Zubereitung für Haare ein nichtionisierendes Polymer umfasst;
wobei das nichtionisierende Polymer mit dem pulverförmigen Mittel kombiniert wird.

6. Verfahren nach Anspruch 5, **dadurch gekennzeichnet, dass** die kosmetische Zubereitung für Haare ein Verdickungsmittel umfasst;
wobei das Verdickungsmittel mit dem pulverförmigen Mittel kombiniert wird.

7. Verfahren nach einem der Ansprüche 1 bis 6, **dadurch gekennzeichnet, dass** das zweite Mittel ebenso ein amphoteres Tensid und ein kationisches Polymer beinhaltet.

8. Verfahren nach Anspruch 2 oder 7, **dadurch gekennzeichnet, dass** die kosmetische Zubereitung für Haare ebenso ein anorganisches Alkalimetallsalz beinhaltet.

9. Verfahren nach einem der Ansprüche 1 bis 8, **dadurch gekennzeichnet, dass**:
das Alkalimittel ein Carbonat ist;
die kosmetische Zubereitung für Haare ebenso 1 bis 5 Gew.-% eines Chelatbildners beinhaltet; und
das Gewichtsverhältnis des Carbonatgehalts der kosmetischen Zubereitung für Haare zu dem Chelatbildnergehalt der kosmetischen Zubereitung für Haare 0,02 bis 6,5 beträgt.

10. Verfahren nach einem der Ansprüche 1 bis 9, **dadurch gekennzeichnet, dass** der Behälter (20) mit einem zylindrischen Behälterkörperboden (21) und einem hemisphärischen Deckel (22), der die Öffnung des Behälterkörpers (21) verschließt, versehen ist.

11. Verfahren nach einem der Ansprüche 1 bis 10, **dadurch gekennzeichnet, dass** der Behälterkörper (21) derart geformt ist, dass die Öffnung breiter ist als der Boden.

12. Verfahren nach einem der Ansprüche 1 bis 11, **dadurch gekennzeichnet, dass** die Höhe h1 des Gehäuseteils in dem Inneren des Behälters (20) 10 bis 25 cm ist.

13. Verfahren nach Anspruch 12, **dadurch gekennzeichnet, dass** das Verhältnis (h1/h2) der Höhe h1 (cm) des Gehäuseteils in dem Inneren des Behälters (20) zu der Höhe h2 (cm) der Mischung (13) in dem Inneren des Behälters (20) vor dem Schäumen in dem Bereich von 2 bis 15 ist.

14. Verfahren nach einem der Ansprüche 1 bis 13, **dadurch gekennzeichnet, dass** das erste Mittel (11) wenigstens eine Komponente, ausgewählt aus Direktfarbstoffen und Oxidationsfarbstoffe, enthält.

15. Verfahren nach einem der Ansprüche 1 bis 14, **dadurch gekennzeichnet, dass** das Alkalimittel in einer derartigen Menge enthalten ist, dass der pH-Wert der Mischung aus dem ersten Mittel und einem zweiten Mittel zu dem Zeitpunkt der Verwendung in den Bereich von 7 bis 12 fällt.

## Revendications

1. Procédé de coloration des cheveux ou de décoloration des cheveux ou d'élimination de coloration des cheveux, **caractérisé en ce qu'**il comprend :
une étape de formation d'une préparation cosmétique pour les cheveux sous la forme d'une mousse
par l'introduction d'un premier agent en poudre (11) comprenant un agent alcalin et un second agent liquide (12) d'une préparation cosmétique pour cheveux comprenant du peroxyde d'hydrogène en tant qu'agent oxydant dans le corps de récipient (21), mettant de cette façon en contact le premier agent (11) et le second agent (12) et obtenant un mélange (13) ;
la fixation d'un couvercle (22) au corps de récipient (21), une partie d'ajustement en forme de bride étant formée sur le bord du couvercle (22), et la partie d'ajustement du couvercle (22) étant ajustée à l'ouverture du corps de récipient (21) de telle sorte que le couvercle (22) est fixé de manière étanche au corps de conteneur (21) par rotation du couvercle (22), obtenant ainsi un récipient refermable et étanche aux fluides (20) et créant une préparation cosmétique pour cheveux sous la forme d'une mousse (14) par agitation du récipient (20) ; l'enlèvement du couvercle (22) du corps de récipient (21) ; et
une étape consistant à retirer manuellement la mousse (14) directement du corps du récipient (21) à la main et à l'appliquer sur les cheveux.

2. Procédé selon la revendication 1, **caractérisé en ce que** la préparation cosmétique pour cheveux comprend un agent tensioactif amphotère.

3. Procédé selon la revendication 1 ou la revendication 2, **caractérisé en ce que** la préparation cosmétique pour cheveux comprend un agent tensioactif cationique.

4. Procédé selon la revendication 3, **caractérisé en ce que** la préparation cosmétique pour cheveux comprend en outre un agent tensioactif anionique ; l'agent tensioactif anionique étant mis en contact avec l'agent tensioactif cationique en présence d'un solvant au moment de l'utilisation.

5. Procédé selon l'une quelconque des revendication 1 à la revendication 4, **caractérisé en ce que** la préparation cosmétique pour cheveux comprend un polymère non ionique ; le polymère non ionique étant combiné à l'agent en poudre.

6. Procédé selon la revendication 5, **caractérisé en ce que** la préparation cosmétique pour cheveux comprend un agent épaississant ; l'agent épaississant étant combiné à l'agent en poudre.

7. Procédé selon l'une quelconque des revendication 1 à la revendication 6, **caractérisé en ce que** le second agent comprend également un agent tensioactif amphotère et un polymère cationique.

8. Procédé selon la revendication 2 ou la revendication 7, **caractérisé en ce que** la préparation cosmétique pour cheveux comprend également un sel inorganique de métal alcalin.

9. Procédé selon l'une quelconque des revendications 1 à la revendication 8, **caractérisé en ce que** :
l'agent alcalin est un carbonate ;
la préparation cosmétique pour cheveux comprend également 1 à 5 % en poids d'un agent chélatant ; et
le rapport en poids de la teneur en carbonate de la préparation cosmétique pour cheveux à la teneur en agent chélatant de la préparation cosmétique pour cheveux est de 0,02 à 6,5.

10. Procédé selon l'une quelconque des revendication 1 à la revendication 9, **caractérisé en ce que** le récipient (20) est pourvu d'un corps de récipient cylindrique avec un fond (21) et un couvercle hémisphérique (22) qui ferme l'ouverture du corps de récipient (21).

11. Procédé selon l'une quelconque des revendication 1 à la revendication 10, **caractérisé en ce que** le corps de récipient (21) est formé de telle sorte que l'ouverture est plus large que le fond.

12. Procédé selon l'une quelconque des revendication 1 à la revendication 11, **caractérisé en ce que** la hauteur h1 de la partie de boîtier à l'intérieur du récipient (20) est de 10 à 25 cm.

13. Procédé selon la revendication 12, **caractérisé en ce que** le rapport (h1/h2) de la hauteur h1 (cm) de la partie de boîtier à l'intérieur du récipient (20) à la hauteur h2 (cm) du mélange (13) à l'intérieur du récipient (20) avant moussage est compris dans la plage de 2 à 15.

14. Procédé selon l'une quelconque des revendication 1 à la revendication 13, **caractérisé en ce que** le premier agent (11) contient au moins un composant choisi parmi des colorants directs et des colorants d'oxydation.

15. Procédé selon l'une quelconque des revendication 1 à la revendication 14, **caractérisé en ce que** l'agent alcalin est compris dans une quantité telle que le pH du mélange du premier agent et d'un second agent se situe dans la plage de 7 à 12 au moment de l'utilisation.
